# EUROPEAN PATENT SPECIFICATION

(11) **EP 0 643 720 B1**
(45) Date of publication and mention of the grant of the patent: **16.12.1998**
(21) Application number: 93915177.5
(22) Date of filing: 01.06.1993
(51) Int. Cl.: C07H 21/00, A61K 31/70

(54) **BINDING COMPETENT OLIGOMERS CONTAINING 2', 5' LINKAGES**
BINDUNGSKOMPETENTE OLIGOMERE, DIE 2',5'-BINDUNGEN ENTHALTEN
OLIGOMERES APTES A LA FIXATION CONTENANT DES LIAISONS 2', 5'

(30) Priority: 01.06.1992 US 892902
(43) Date of publication of application: 22.03.1995
(73) Proprietor: GILEAD SCIENCES, INC., Foster City, CA 94404 (US)
(72) Inventor: SWAMINATHAN, Sundaramoorthi, Burlingame, CA 94010 (US); MATTEUCCI, Mark, Burlingame, CA 94010 (US); PUDLO, Jeff, Burlingame, CA 94010 (US); JONES, Robert J., Millbrae, CA 94030 (US)
(74) Representative: Ablewhite, Alan James
(86) International application number: PCT/US93/05202
(87) International publication number: WO 93/24508

(56) References cited:
- CHEMICAL ABSTRACTS, vol. 120, no. 15, 11 April 1994, Columbus, Ohio, US; abstract no. 192189j, L.WANG ET AL. 'Studies on Synthesis of Diadenylyl Sulfites' page 1100 ;column 1 ; & CHINESE CHEMICAL LETTERS, vol.4, no.2, 1993 pages 101 - 104
- Chemical Reviews, Volume 90, No. 4, issued June 1990, UHLMANN et al., "Antisense Oligonucleotides: A New Therapeutic Principle", pages 543-584, especially pages 554-555.
- Proceedings of the National Academy of Sciences, Volume 85, issued October 1988, AGRAWAL et al., "Oligodeoxynucleoside Phosphoramidates and Phosphorothioates as Inhibitors of Human Immunodeficiency Virus", pages 7079-7083, see page 7080.
- Science, Volume 251, issued 15 March 1991, BEAL et al., "Second Structural Motif for Recognition of DNA by Oligonucleotide-Directed Triple-Helix Formation", pages 1360-1363, see entire document.
- Proceedings of the National Academy of Sciences, Volume 88, issued November 1991, YOUNG et al., "Triple Helix Formation Inhibits Transcription Elongation in Vitro", pages 10023-10026, see entire document.

## Description

### Field of the Invention

The invention relates to novel oligomers, the construction thereof, and their use in oligomer-based therapies. More specifically, the invention is to novel oligomers having substitute oligonucleotide linkages which are resistant to nucleases, having enhanced ability to penetrate cells, and which are capable of binding target oligonucleotide sequences in vitro and in vivo. The modified oligomers of the invention are particularly useful in oligomer-based therapies utilizing the modified oligomers to interrupt protein synthesis or otherwise inactivate messenger RNA or double stranded DNA.

### Background of the Invention

The application of oligonucleotide analogs for therapeutic uses represents a relatively new development in drug design and discovery. Several fundamental therapeutic approaches that utilize oligomers have been proposed.

One approach is based largely on interfering with gene expression through oligomer binding to a complementary RNA sequence. This application is known as "antisense" therapy because the oligomer base sequence is identical to the antisense strand of the gene that gave rise to the RNA (Uhlmann, E., et al., Chem Reviews (1990) 90:543-584; and Stein, C.A., et al., Cancer Res (1988) 48:2659-2668). Another approach, referred to herein as "triple helix" therapy utilizes oligomers that bind to duplex DNA as detailed below. Binding to a target DNA is sequence specific but involves different base pairing binding (i.e. Hoogsteen binding). Both antisense and triple helix therapies exert therapeutic effects via binding to nucleic acid sequences that are responsible for disease conditions. Such sequences are found in the genome of pathogenic organisms including bacteria, protozoa, yeasts, parasites, fungi or viruses or may be endogenous sequences (oncogenes). By modulating the expression of a gene important for establishment, maintenance or elimination of a disease condition, the corresponding condition may be cured, prevented or ameliorated.

Another therapeutic approach that is based on the use of oligomers includes generation of "aptamers" and is disclosed in commonly owned international patent application nos. WO 92/14842 and WO 92/14843. This approach utilizes oligomers that specifically bind to proteins thereby interfering with their function. The use of oligomers that mimic the structure of certain RNA molecules that are bound by intracellular proteins has also been adduced as a therapeutic approach as described in international application no. PCT/US91/01822.

Antisense oligonucleotides are synthetic oligonucleotides which bind complementary RNAs (i.e. sense strand sequences) via hydrogen bonding, thereby inhibiting translation of these sequences. Therapeutic intervention at the nucleic acid level using antisense oligonucleotides offers a number of advantages. For example, gene expression can be inhibited using antisense or triple helix oligomers. Inhibition of gene expression is more efficient than inhibition of the protein encoded by the gene since transcription of a single DNA sequence gives rise to multiple copies of mRNA which, in turn, are translated into many protein molecules.

Antisense and triple helix therapies for diseases whose etiology is characterized by, or associated with, specific DNA or RNA sequences, are particularly useful. The oligomer employed as the therapeutic agent can be directly administered or generated in situ and is one that is complementary to a DNA or RNA needed for the progress of the disease. The oligomer specifically binds to this target nucleic acid sequence, thus modulating or disturbing its ordinary function.

An oligomer having a base sequence complementary to that of an mRNA which encodes a protein necessary for the progress of the disease, is particularly useful. By hybridizing specifically to this mRNA, the synthesis of the protein will be interrupted. However, it is also possible to bind double-stranded DNA using an appropriate oligomer capable of effecting the formation of a specific triple helix by inserting the administered oligomer into the major groove of the double-helical DNA. The resulting triple helix structure can then interfere with transcription of the target gene (Young, S.L. et al., Proc Natl Acad Sci (1991) 88:10023-10026).

An important feature of therapeutic oligomers is the design of the backbone or linkage of the administered oligomer. Specifically, the backbone should contain internucleoside linkages that are stable in vivo and should be structured such that the oligomer is resistant to endogenous nucleases, such as nucleases that attack the phosphodiester linkage. At the same time, the oligomer must also retain its ability to hybridize to the target DNA or RNA. (Agarwal, K.L. et al., Nucleic Acids Res (1979) 6:3009; Agarwal, S. et al., Proc Natl Acad Sci USA (1988) 85:7079.) In order to ensure these properties, a number of modified oligonucleotides have been constructed which contain alternate internucleoside linkages. Several of these oligonucleotides are described in Uhlmann, E. and Peyman, A., Chemical Reviews (1990) 90:543-584. Among these are methylphosphonates (wherein one of the phosphorous-linked oxygens has been replaced by methyl); phosphorothioates (wherein sulphur replaces one of these oxygens) and various amidates (wherein NH₂ or an organic amine derivative, such as morpholidates or piperazidates, replace an oxygen). These substitutions confer enhanced stability, for the most part, but suffer from the drawback that they result in a chiral phosphorous in the linkage, thus leading to the formation of 2ⁿ diastereomers where n is the number of modified diester linkages in the oligomer. The presence of these multiple diastereomers considerably weakens the capability of the modified oligonucleotide to hybridize to target sequences. Some of these substitutions also retain the ability to support a negative charge and the presence of charged groups decreases the ability of the compounds to penetrate cell membranes. There are numerous other disadvantages associated with these modified linkages, depending on the precise nature of the linkage.

It has also been suggested to use carbonate diesters. However, these are highly unstable, and the carbonate diester link does not maintain a tetrahedral configuration exhibited by the phosphorous in the phosphodiester. Similarly, carbamate linkages, while achiral, confer trigonal symmetry and it has been shown that poly dT having this linkage does not hybridize strongly with poly dA (Coull, J.M., et al., Tet Lett (1987) 28:745; Stirchak, E.P., et al., J Org Chem (1987) 52:4202).

WO 91/15500, published October 17, 1991, teaches various oligonucleotide analogs in which one or more of the internucleotide linkages are replaced by a sulfur based linkage typically sulfamate diesters which are isosteric and isoelectric with the phosphodiester.

WO 89/12060, published December 14, 1989, discloses linkages containing sulfides, sulfoxides, and sulfones.

WO 86/05518, published September 25, 1986, suggests a variant of stereoregular polymeric 3',5' linkages.

U.S. Patent No. 5,079,151 to Lampson et al., discloses a msDNA molecule of branched RNA linked to a single strand DNA via a 2',5' phosphodiester linkage.

Commonly owned, U.S. Patent No. 5.264.562 filed April 24, 1991, the entirety of which is incorporated by notice, describes modified linkages of the formula -Y'CX'₂Y'- wherein Y' is independently O or S and wherein each X' is a stabilizing substituent and independently chosen. Amide-containing linkages disclosed in patent application no. WO 93/24507 the entirety of which application is incorporated herein by reference, are also suitable for incorporation into oligomers containing one or more of the linkages disclosed herein.

Modifications of oligomers that enhance their affinity for target molecules will generally improve the therapeutic potential for those compounds. Previous approaches to improve binding affinity for complementary nucleic acids have centered primarily on (i) covalent linkage of intercalating agents to oligomers (Asseline, U., et al., Proc Natl Acad Sci (1984) 81:3297-3401), (ii) introduction of modified bases to form more stable base pairs (Inoue, H. et al., Nucl Acids Res (1985) 13:7119) and (iii) altering the charge characteristics of oligomer internucleotide linkages (Letsinger, R.L. et al., J. Am Chem Soc (1988) 110:4470). Morpholino-type internucleotide linkages are described in U.S. Patent 5,034,506 and in some cases give rise to an increased affinity of the oligomer for complementary target sequences.

Patent application no. WO 92/05186, and US patent no. 5.264.562 disclose modified oligonucleotides having modified nucleoside linkages which are capable of strong hybridization to target RNA and DNA.

That disclosure suggests that 3',5' internucleoside linkages are especially appropriate since the resulting oligonucleotides are stable in vivo, resistant to endogenous nucleases, and are able to hybridize to target nucleotide sequences. The structure of the oligonucleotide backbone suggests that 3',5' linkage is optimal for a phosphodiester linkage in view of the steric size of the phosphodiester group and the related bond lengths, angles, and torsions involved. Although a phosphodiester should be suitable for the 2',5' linkage since the O2'-P, P-O5' bond lengths, angles, and torsions are all appropriate, the steric bulk of the phosphodiester is too large for the space available between the 5' sugar (2' and 3' atoms) and the 3' sugar (O4' and C5'). Our discovery of the 2',5' linkage and its characteristics is based on modeling studies that both (i) predicted such linkages in a binding-competent oligonucleotide and (ii) defined the range of molecular characteristics such linkages could assume without loss of binding competence. Binding competence, as used herein, refers to either Watson-Crick pairing with single-stranded DNA or single-stranded RNA or to Hoogsteen pairing (Beal, P.A. et al., Science (1991) 251:1360-1363) with duplex nucleic acids including duplex DNA or duplex RNA.

Although space available for the 2',5' linkage has more stringent steric restraints upon it than does the space for the 3',5' linkage, there are fewer restrictions with regard to bond lengths, angles and the like. Because of this, the 2',5' linkage is fundamentally different from the 3',5' linkage. This restraint has been experimentally verified in that 2',5' phosphodiesters have been found not to be suitable for efficient hybridization (Kierzek, R. et al., Nucl Acids Res (1992) 20:1685-1690). However, the 2',5' linkages of this invention do not have the steric bulk of a phosphodiester but do fall within the range of requirements for bond length, angles, and torsion placed upon a linkage in that position. Consequently, the substitute linkages (invention 2',5' linkages) are quite suitable and, in some cases, superior to 3',5' phosphodiester linkages.

The therapeutic potential of oligomers is generally enhanced by modifications that increase oligomer uptake by cells or reduce the rate of metabolism by cells or serum. Such modifications include (i) reduced oligomer charge, (ii) increased stability toward nuclease activity, and (iii) increased lipophilicity of the oligomer. Oligomers having modified internucleotide linkages as described in the invention exhibit sequence-specific binding to complementary single stranded and duplex target sequences.

The present invention provides substitute linkages which are resistant to nuclease digestion, and which are stable under physiological conditions, and which can be neutral or positively charged so as to enhance cell permeation. Both nuclease stability and enhanced cellular permeation are important considerations for the development of oligomers that are intended to be used as therapeutic agents that function by binding to specific DNA or RNA (mRNA, hnRNA, etc.) sequences. Such specific target sequence binding underlies their therapeutic efficacy by interfering with the normal biological function of nucleic acid sequences associated with pathological conditions. Furthermore, the substitute linkages can be achiral and thus do not lead to the problem of multiple diastereomers in the resulting compounds.

### Summary of the Invention

The present invention is based on the construction of oligomers containing novel substitute linkages which substitute linkages are also referred to as 2',5' linkages. Such linkages comprise substitution, for one or more linkages between adjacent nucleomonomers a linkage between the 2' and 5' position of adjacent nucleomonomers. The 2',5' linkages comprise a two to four atom long substitute linkage wherein at least one of the atoms making up the substitute linkage is selected from nitrogen (N), oxygen (O) and sulfur (S), with the remainder being carbon. Adjacent carbons may be connected via single, double or triple bonds. These oligonucleotides are stable in vivo, resistant to endogenous nucleases and are able to hybridize to target nucleotide sequences.

In another embodiment, the subject invention is directed to an oligomer of the formula:

(W,Y)-Q-(Z-Q)ₙ-(W,Y),

where each 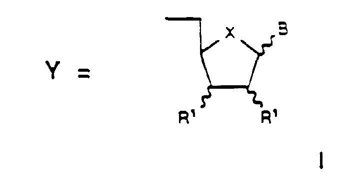 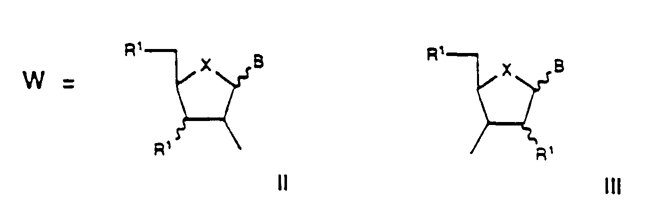 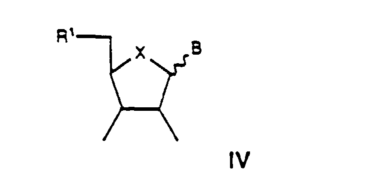 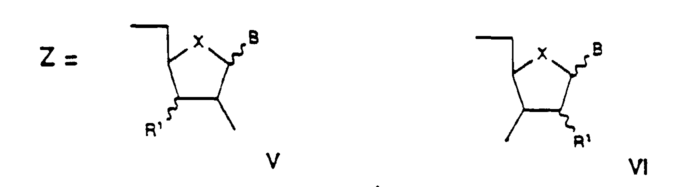 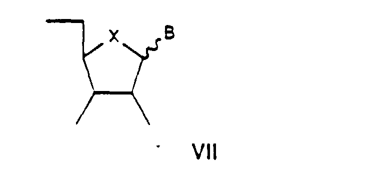 where X is S, O, CH₂, CHF or CF₂;
R¹ independently is -O-alkyl (C₁-C₁₂), -S-alkyl (C₁-C₁₂), H, OH, OCH₃, SCH₃, OC₃H₅ (O-allyl), OC₃H₇(O-propyl), SC₃H₅, or F and
where R¹ is on a terminal group of the oligomer, R¹ may additionally be a blocking group including PO₃⁻²,
dimethoxytrityl (DMT), monomethoxytrityl (MMT), H-phosphonate (OPO₂H), methylphosphonate (OPO₂CH₃) methylphosphonamidite, or a phosphoramidite such as β-cyanoethylphosphoramidite;
- B: is independently a base, and
- Q: is independently
a linkage or
a two to four atom long substitute linkage
   wherein at least one of the atoms making up the substitute linkage is selected from nitrogen, oxygen or sulfur, with the remainder being carbon; but no two adjacent atoms are oxygen and, for three atom linkages, no 3 adjacent atoms are all nitrogen or all oxygen or all sulfur and wherein at least one substitute linkage is a 2',5' linkage and;
- n: is 1-100, preferably 2-28,
but where each Q and each Z in each unit (n) is independently selected.

The designation (W, Y) means that either W or Y is linked to Q at the indicated positions. In preferred embodiments, a maximum of 20% of the linkages give rise to inversion of oligomer polarity. Linkages that invert polarity occur when there is a 5' to 5', 3' to 2' or 3', or 2' to 3' or 2' linkage between adjacent nucleomonomer residues. The preferred linkage type for the majority of linkages and substitute linkages in most oligomers is thus 3' or 2' to 5'. When X is CH₂ or CHF, the material may be produced according to published procedures (Otvos, L. et al. Tet Letters (1987) 28:6381-6384; Divakar, K.L. et al J. Chem Soc Perkin Trans I (1982) 1625; J. Chem Soc., Perk. Trans. I (1991) 2373-2377).

In preferred embodiments structure VII is included in oligomers as a nucleomonomer linked to an adjacent nucleomonomer through a riboacetal substitute linkage. In other embodiments where VII is not linked via a riboacetal substitute linkage, circular or branched oligomers may be obtained which are useful in oligonucleotide based therapies or diagnosis applications (Kool, E. T., J Am Chem Soc (1991) 113:6265-6266).

Oligomers are conveniently produced from dimers, trimers or tetramers as synthons for solid phase or solution phase synthesis using standard methods known in the art.

In yet other embodiments, the invention is directed to methods for treating diseases mediated by the presence of a nucleotide sequence which method comprises administering to a subject in need of such treatment an amount of an invention oligomers capable of specifically binding the nucleotide sequence effective so to inactivate the nucleotide sequence or to modulate its biological activity.

A feature of substitute linkages wherein an electron withdrawing heteroatom such as oxygen is located at the 2' position is enhanced stability of the glycosidic bond with the heterocycle. Such linkages are included in the group of preferred linkages described in detail below.

It has been found that oligomers containing the invention substitute linkages efficiently bind complementary single-stranded and double-stranded nucleic acid sequences. Triple helix structures were formed under physiological salt conditions. Unmodified control oligomers were often less efficient at forming triple helices under the same conditions. Thus, oligomers of the present invention are generally characterized as containing one or more 2',5' substitute linkages. The analogs may be utilized in oligomers that contain additional modifications of other nucleomonomers that comprise the oligomer. An exemplary list of such modifications include oligomers where (i) one or more nucleomonomer residue is modified at the 2' position, (ii) one or more crosslinking moieties have been incorporated, (iii) switchback linkers have been incorporated as described in U.S. patent no. 5,399,676 incorporated herein by reference, (iv) other substitute linkages have been included and (v) bases that facilitate duplex or triplex formation, such as 8-oxo-N⁶-methyladenine, 5-propynyluracil, 5-propynylcytosine or 7-deazaxanthine have been included. One or more of such modifications may advantageously be incorporated into a given oligomer depending upon target nucleic acid sequences.

These and other embodiments of the present invention will readily occur to those of ordinary skill in the art in view of the disclosure herein.

### Brief Description of the Figures

Figures 1 through 25 are depictions of chemical reaction sequences usable for synthesizing internucleoside linkages of the present invention. More specifically:
Figure 1 shows the synthesis of a three atom long linkage with a nitrogen at the 5' end.
Figure 2 shows the synthesis of a three atom long linkage with a nitrogen at the 2' end.
Figure 3 depicts the synthesis of a three atom long linkage with a nitrogen in the middle.
Figure 4 depicts the formation of a four atom long linkage with oxygen at the 2' end and nitrogen at the 5' end.
Figure 5 shows the formation of a four atom long linkage with nitrogen at the 2' end and oxygen at the 5' end.
Figure 6 depicts the formation of a two atom long linkage with nitrogen at the 5' end.
Figure 7 shows the formation of a two atom long linkage with nitrogen at the 2' end.
Figure 8 shows the formation of three different three atom long linkages with sulfur at the 2' end.
Figure 9 depicts the formation of three different two atom long linkages with sulfur at the 2' end.
Figure 10 shows the synthesis of three different two atom long linkages with sulfur at the 5' end.
Figure 11 depicts the synthesis of a two atom long linkage with oxygen at the 2' end.
Figure 12 depicts the formation of a three atom long linkage with oxygen at the 5' end.
Figure 13 shows the formation of several three atom long linkages with derivatized nitrogen at the 2' end.
Figure 14 shows the synthesis of a three atom long linkage containing nitrogen at the 2' end and oxygen at the 5' end.
Figure 15 shows the formation of a three atom long linkage with sulfur at the 2' end.
Figure 16 shows the formation of a three atom linkage having a nitrogen atom at the 2' end and an oxygen-bearing carbon atom at mid-linkage and oxygen at the 5' end.
Figure 17 shows the formation of a three atom linkage having an oxygen atom at the 2' end and an oxygen-bearing carbon atom at mid linkage and nitrogen at the 5' end and also a linkage having an oxygen at the 2' end and a sulfur at the 5' end and a carbon midlinkage.
Figures 18 and 19 show the formation of certain feedstocks such as compound 1 in Figure 1 and compound 10 in Figure 2 suitable for synthesis of the desired oligomers of this invention.
Figure 20 shows the formation of a three atom formacetal linkage.
Figure 21 shows the synthesis of a xylosofluoro monomer synthon.
Figure 22 shows the synthesis of a ribofluoro monomer synthon.
Figure 23 shows the formation of a three atom 2', 5'-thioformacetal linkage using xylosofluoro and ribofluoro monomers.
Figure 24 shows the formation of a three atom 2',5' formacetal linkage using xylosofluoro and ribofluoro monomers.
Figure 25 shows the formation of a three atom 2',5' thioformacetal linkage.
Figure 26-1 shows structures of coupling groups used for linkage of nucleomonomers linked via phosphorous containing linkages.
Figure 26-2 shows structures of coupling groups used for linkage of nucleomonomers linked via phosphorous containing linkages.
Figure 27 shows synthesis of a nucleomonomer used to synthesize substitute linkages containing a double bond.
Figure 28-1 shows synthesis of an intermediate in the synthesis of a dimer linked by a three atom substitute linkage having sulfur at the 2' position and a double bond.
Figure 28-2 shows synthesis of a dimer linked by a three atom substitute linkage having sulfur at the 2' position and a double bond.

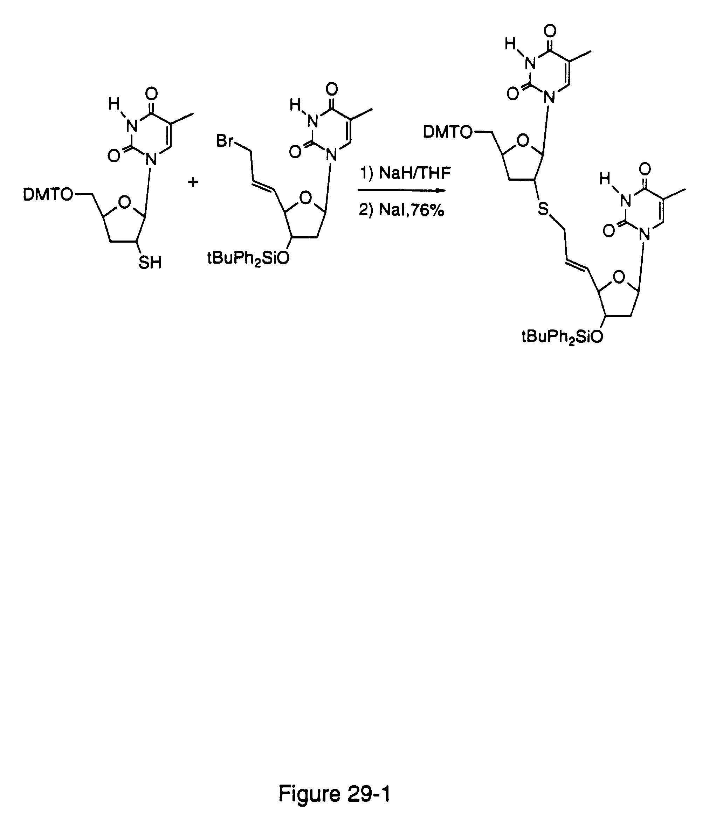

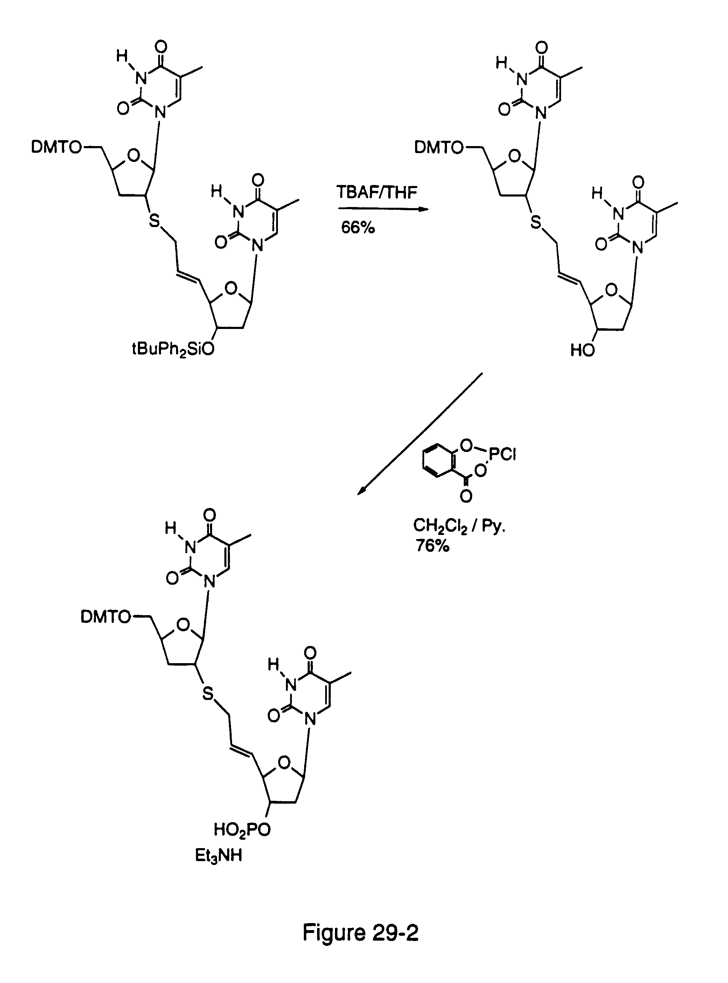

Figure 29-1 shows synthesis of an intermediate in the synthesis of a dimer linked by a three atom substitute linkage having oxygen at the 2' position and a double bond.
Figure 29-2 shows synthesis of a dimer linked by a three atom substitute linkage having oxygen at the 2' position and a double bond.

### Description of the Invention

The practice of the present invention will employ, unless otherwise indicated, conventional techniques of chemistry, molecular biology, biochemistry, protein chemistry, and recombinant DNA technology, which are within the skill of the art. Such techniques are explained fully in the literature. See, e.g., Oligonucleotide Synthesis (M.J. Gait ed. 1984); Nucleic Acid Hybridization (B.D. Hames & S.J. Higgins eds. 1984); Sambrook, Fritsch & Maniatis, Molecular Cloning: A Laboratory Manual, Second Edition (1989); and the series Methods in Enzymology (S. Colowick and N. Kaplan eds., Academic Press, Inc.).

### Structural Formulas

Structural formulas described herein are designated as roman numerals (I, II, etc) and chemical compounds are designated a numeral (**1**, **2**, etc).

### Detailed Description of the Invention

### Definitions

Nucleomonomer. As used herein, the term "nucleomonomer" means a moiety comprising (1) a base covalently linked to (2) a second moiety. Nucleomonomers include nucleosides and nucleotides. Nucleomonomers can be linked to form oligomers that bind to target or complementary base sequences in nucleic acids in a sequence specific manner.

A "second moiety" as used herein includes a sugar moiety, usually a pentose, and those species which contain modifications of the sugar moiety, for example, wherein one or more of the hydroxyl groups are replaced with a halogen (Cl, Br, F, I), a heteroatom (including N, S and 0), an aliphatic group, or are functionalized as ethers, amines, thiols, and the like. The pentose moiety can be replaced by a hexose or an alternate structure such as a cyclopentane ring, a 6-member morpholino ring and the like. Nucleomonomers as defined herein are also intended to include a base linked to an amino acid and/or an amino acid analog having a free carboxyl group and/or a free amino group and/or protected forms thereof.

Base. "Base" as used herein includes those moieties which contain not only the known purine and pyrimidine heterocycles, but also heterocycle analogs and tautomers thereof. Purines include adenine, guanine and xanthine and exemplary purine analogs include 8-oxo-N⁶-methyladenine and 7-deazaxanthine. Pyrimidines include uracil and cytosine and their analogs such as 5-methylcytosine, 5-(1-propynyluracil), 5-(1-propynylcytosine), 5-methyluracil and 4,4-ethanocytosine.

Nucleoside. As used herein, "nucleoside" means a base covalently attached to a sugar or sugar analog and which may contain a phosphite or phosphine. The term nucleoside includes ribonucleosides, deoxyribonucleosides, or any other nucleoside which is an N-glycoside or C-glycoside of a base. The stereochemistry of the sugar carbons can be other than that of D-ribose.

Nucleosides include those species which contain modifications of the sugar moiety, for example, wherein one or more of the hydroxyl groups are replaced with a halogen, a heteroatom, an aliphatic group, or are functionalized as ethers, amines, thiols, and the like. The pentose moiety can be replaced by a hexose or an alternate structure such as a cyclopentane ring, a 6-member morpholino ring and the like.

The term "nucleoside" will include ribonucleosides, deoxyribonucleosides, or any other nucleoside which is an N-glycoside or C-glycoside of a base, including purine or pyrimidine bases. The stereochemistry of the sugar carbons can be other than that of D-ribose in one or more residues. The pentose moiety can be replaced by a hexose and incorporated into oligomers as described (Augustyns, K., et al Nucl Acids Res (1992) 20:4711-4716). Also included are analogs where the ribose or deoxyribose moiety is replaced by an alternate structure such as a hexose or such as the 6-member morpholino ring described in U.S. patent number 5,034,506. Nucleosides as defined herein also includes a purine or pyrimidine base linked to an amino acid or amino acid analog having a free carboxyl group and a free amino group or protected forms thereof.

Nucleotide. As used herein, "nucleotide" means a nucleoside having a phosphate group or phosphate analog (groups with phosphorus in the same oxidation state as in the phosphate group e.g. thiophosphate, amidate).

Sugar Modification. As used herein, "sugar modification" means any pentose or hexose moiety other than 2'-deoxyribose. Modified sugars include D-ribose, 2'-O-alkyl (1 - 12C), 2'-amino, 2'-halo (F, Cl, Br, I) functionalized pentoses, hexoses and the like. Sugars having a stereochemistry other than that of a D-ribose are also included. For nucleomonomers where the 3' position is not part of the substitute linkage, modified sugars also include 3'-O-alkyl (1 - 12C), 3'-amino and 3'-halo functionalized pentoses, hexoses and the like.

Linkage. As used herein, "linkage" means a phosphodiester moiety (-O-P(O)(O)-O-) that covalently couples adjacent nucleomonomers.

Substitute Linkages. As used herein, "substitute linkage" means any analog of the native phosphodiester group or any suitable moiety that covalently couples adjacent nucleomonomers. Substitute linkages include phosphodiester analogs, e.g. such as phosphorothioate and methylphosphonate, and nonphosphorus containing linkages, e.g. such as acetals and amides. Substitute linkages include the nonphosphorous containing linkages (2',5' linkages) of the invention.

Switchback. As used herein, "switchback" means an oligomer having at least one region of inverted polarity. Switchback oligomers are able to bind to opposite strands of a duplex to form a triplex on both strands of the duplex. The linker ("switchback linker") joining the regions of inverted polarity is a substitute linkage.

Crosslinking moiety. "Crosslinking moiety" includes a group or moiety in an oligomer that forms a covalent bond with a target nucleic acid. Crosslinking moieties include covalent bonding species that covalently link an oligomer to target nucleic acids either spontaneously (e.g. N⁴,N⁴-ethanocytosine) or via photoactivation (e.g. psoralen and the like).

Oligomers. "Oligomers" are defined herein as two or more nucleomonomers covalently coupled to each other by a linkage or substitute linkage. Thus, an oligomer can have as few as two convalently linked nucleomonomers (a dimer). Oligomers can be binding competent and, thus, can base pair with cognate single-stranded or double-stranded nucleic acid sequences. Oligomers (e.g. dimers - hexamers) are also useful as synthons for longer oligomers as described herein. Oligomers can also contain abasic sites and pseudonucleosides.

Oligomer includes oligonucleotides, oligonucleosides, polydeoxyribo-nucleotides (containing 2'-deoxy-D-ribose or modified forms thereof), i.e., DNA, polyribonucleotides (containing D-ribose or modified forms thereof), i.e., RNA, and any other type of polynucleotide which is an N-glycoside or C-glycoside of a purine or pyrimidine base, or modified purine or pyrimidine base. Oligomer as used herein is also intended to include compounds where adjacent nucleomonomers are linked via amide linkages as previously described (Nielsen, P.E., et al, Science (1991) 254:1497-1500). Elements ordinarily found in oligomers, such as the furanose ring and/or the phosphodiester linkage can be replaced with any suitable functionally equivalent element. "Oligomer" is thus intended to include any structure that serves as a scaffold or support for the bases wherein the scaffold permits binding to target nucleic acids in a sequence-dependent manner. Oligomers that are currently known can be defined into four groups that can be characterized as having (i) phosphodiester and phosphodiester analog (phosphorothioate, methylphosphonate, etc) linkages, (ii) substitute linkages that contain a non-phosphorous isostere (riboacetal, formacetal, carbamate, etc), (iii) morpholino residues, carbocyclic residues or other furanose sugars, such as arabinose, or a hexose in place of ribose or deoxyribose and (iv) nucleomonomers linked via amide bonds or acyclic nucleomonomers linked via any suitable substitute linkage.

Blocking Groups. As used herein, "blocking group" refers to a substituent other than H that is conventionally coupled to oligomers or nucleomonomers, either as a protecting group, a coupling group for synthesis, OPO₃⁻², or other conventional conjugate such as a solid support, label, antibody, monoclonal antibody or fragment thereof and the like. As used herein, "blocking group" is not intended to be construed solely as a protecting group, according to slang terminology, but is meant also to include, for example, coupling groups such as a H-phosphonate or a phosphoramidite.

By "protecting group" is meant is any group capable of protecting the O-atom, S-atom or N-atom to which it is attached from participating in a reaction or bonding. Such protecting groups for N-atoms on a base moiety in a nucleomonomer and their introduction are conventionally known in the art. Non-limiting examples of suitable protecting groups include diisobutylformamidine, benzoyl and the like. Suitable "protecting groups" for O-atoms and S-atoms are, for example, DMT, MMT, FMOC or esters.

Protecting group. "Protecting group" as used herein includes any group capable of preventing the O-atom, S-atom or N-atom to which it is attached from participating in a reaction or bonding. Such protecting groups for 0-, S- and N-atoms in nucleomonomers are described and methods for their introduction are conventionally known in the art. Protecting groups also include any group capable of preventing reactions and bonding at carboxylic acids, thiols and the like.

Coupling group. "Coupling group" as used herein means any group suitable for generating a linkage or substitute linkage between nucleomonomers such as a hydrogen phosphonate, a phosphoramidite and a chloromethyl alkyl ether.

Conjugate. "Conjugate" as used herein means any group attached to the oligomer at a terminal end or within the oligomer itself. Conjugates include solid supports, such as silica gel, controlled pore glass and polystyrene; labels, such as fluorescent, chemiluminescent, radioactive atoms or molecules, enzymatic moieties and reporter groups; oligomer transport agents, such as polycations, serum proteins and glycoproteins and polymers and the like.

Synthon. "Synthon" as used herein means a structural unit within a molecule that can be formed and/or assembled by known or conceivable synthetic operations.

Transfection. "Transfection" as used herein refers to any method that is suitable for enhanced delivery of oligomers into cells.

Subject. "Subject" as used herein means a plant or an animal, including a mammal, particularly a human.

By "phosphodiester analog" is meant an analog of the conventional phosphodiester linkage 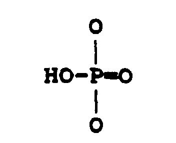 as well as alternative linking groups. These substitute linkage groups contain phosphorus and include, but are not limited to embodiments wherein the P(O)O is replaced with P(S)S, P(O)S, P(O)NR''₂, P(O)R'', P(O)OR''', wherein R'' is H or alkyl (1-6C) and R''' is alkyl (1-6C). The substitute linkages listed below are suitable for Q, formacetal and riboacetal linkages. Suitable riboacetal and formacetal linkages are disclosed in copending application having U.S. patent nos. 5,264,562; application nos. 07/806,710 and 07/990,848, all of which are incorporated herein by reference, and include formacetal linkages such as:
3'-thioformacetal (3' -S-CH₂-O- 5'),
formacetal (3' -O-CH₂-O- 5'),
3'-amino (3' -N-CH₂-CH₂- 5'),
3'-thioketal (3' -S-C(R⁸)₂-O- 5'), and ketal 3' -O-C(R⁸)₂-O- 5'
   where R⁸ is CH₂F or, when both R⁸ are taken together with the atom to which they are attached, form a 4-membered ring or a 6-membered ring where
   (R⁸)₂ is -CH₂-X¹-CH₂-, or -CH₂-CH₂-X¹-CH₂-CH₂-;
and wherein X¹ is selected from the group consisting of S, SO, SO₂, O, CF₂, CHF, NH, NMe, NEt, NPr. Suitable riboacetal linkages include members of the group: 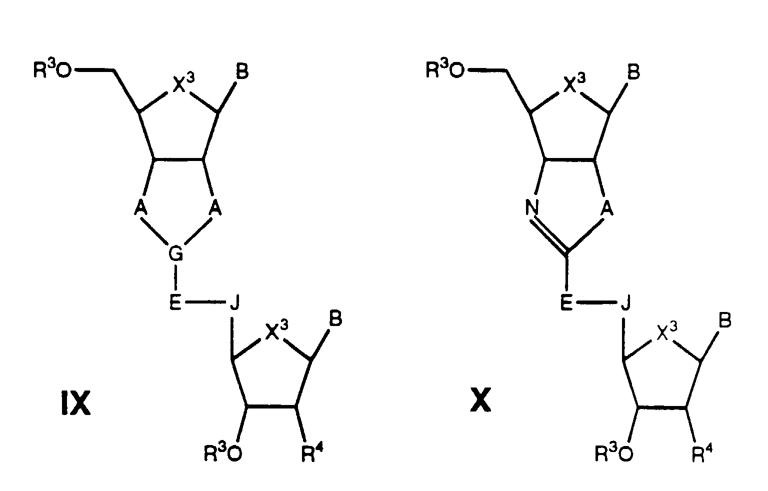 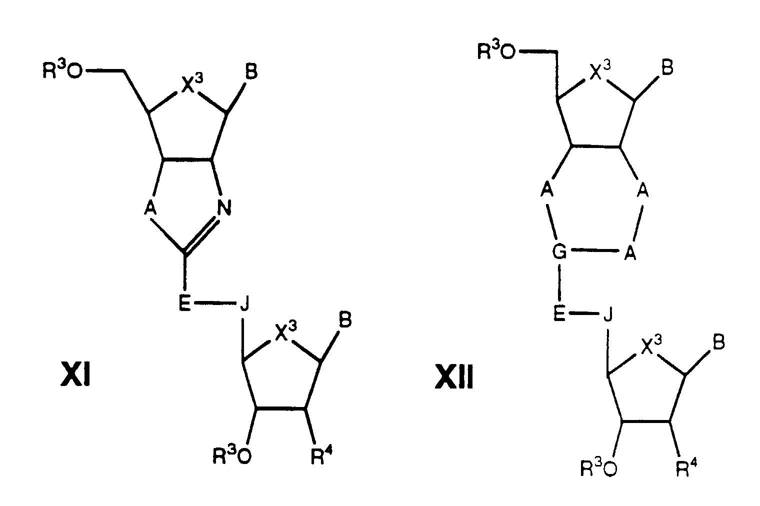 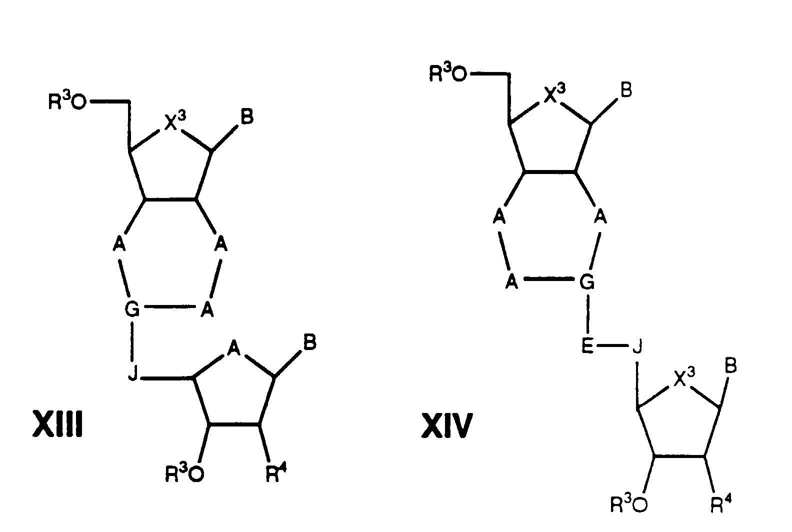 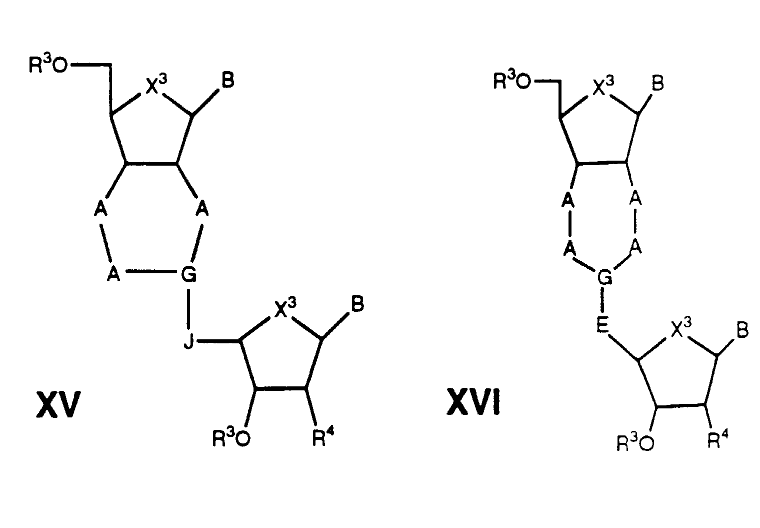 where R³ is independently H, PO₃⁻² or a suitable blocking group such as a dimethoxytrityl (DMT) moiety, a monomethoxytrityl (MMT) moiety, H-phosphonate (OPO₂H), methylphosphonate (OPO₂CH₃) or a phosphoramidite; R⁴ is selected from the group consisting of H, OH, F, OCH₃, OC₂H₅, OCH₂CHCH₂ (O-allyl, OC₃H₅), OC₃H₇ (O-propyl), SCH₃, SC₂H₅, SCH₂CHCH₂ (S-allyl, SC₃H₅), and SC₃H₇ (S-propyl). Methylphosphoramidite and β-cyanoethylphosphoramidite are preferred phosphoramidite groups. B is a purine or pyrimidine base or an analogous form thereof; X³ is independently selected from the group consisting of O, S, CH₂, CF₂ and CFH; A is independently selected from the group consisting of O, S, SO, SO₂, CH₂, CO, CF₂, CS, NH and NR⁵ wherein R⁵ is lower alkyl (1 - 4C; including methyl, ethyl, propyl, isopropyl, butyl and isobutyl) with the proviso that adjacent A are not both O; E is selected from the group consisting of O, S, CH₂, CO, CF₂, CS, NH and NR⁵; J is selected from the group consisting of O, S, CH₂, CO, CF₂ and CS; G is selected from the group consisting of CH, N, CF, CCl, and CR⁷ wherein R⁷ is lower alkyl (1 - 4C; including methyl, ethyl, propyl, isopropyl, butyl and isobutyl) or lower fluoroalkyl (1-4C, 1-5F). Bases (B) that are preferred are adenine, thymine, guanine, cytosine, 8-oxo-N₆-methyladenine, N⁴,N⁴-ethanocytosine, and 5-methylcytosine, 5-propynyluracil, 5-propynylcytosine, 7-methyl-7-deazaguanine, 7-methyl-7-deazaadenine, 7-deazaxanthine and 7-deazaguanine.

The invention is directed to new oligomers which are useful in oligomer-based therapies and intermediates or synthons in their production, as well to methods to synthesize the oligomers and their intermediates. In general, the invention compounds show enhanced stability with respect to nucleases by virtue of their modified linkages, as well as enhanced ability to permeate cells. In oligomers of the invention, at least one 2',5' linkage is present. Desirably, more than one invention substitute linkage (i.e. 2',5' linkage) described herein is present. One substitute linkage may be used repeatedly in this structure, or, if desired a variety of substitute linkages may be used. Though it is preferred that the substitute linkages be non-chiral in nature to enhance the ability to hybridize, useful oligomers of the invention also include those where chiral forms are used.

Preferred 2',5' linkages include the structures for Q shown in Table 1.

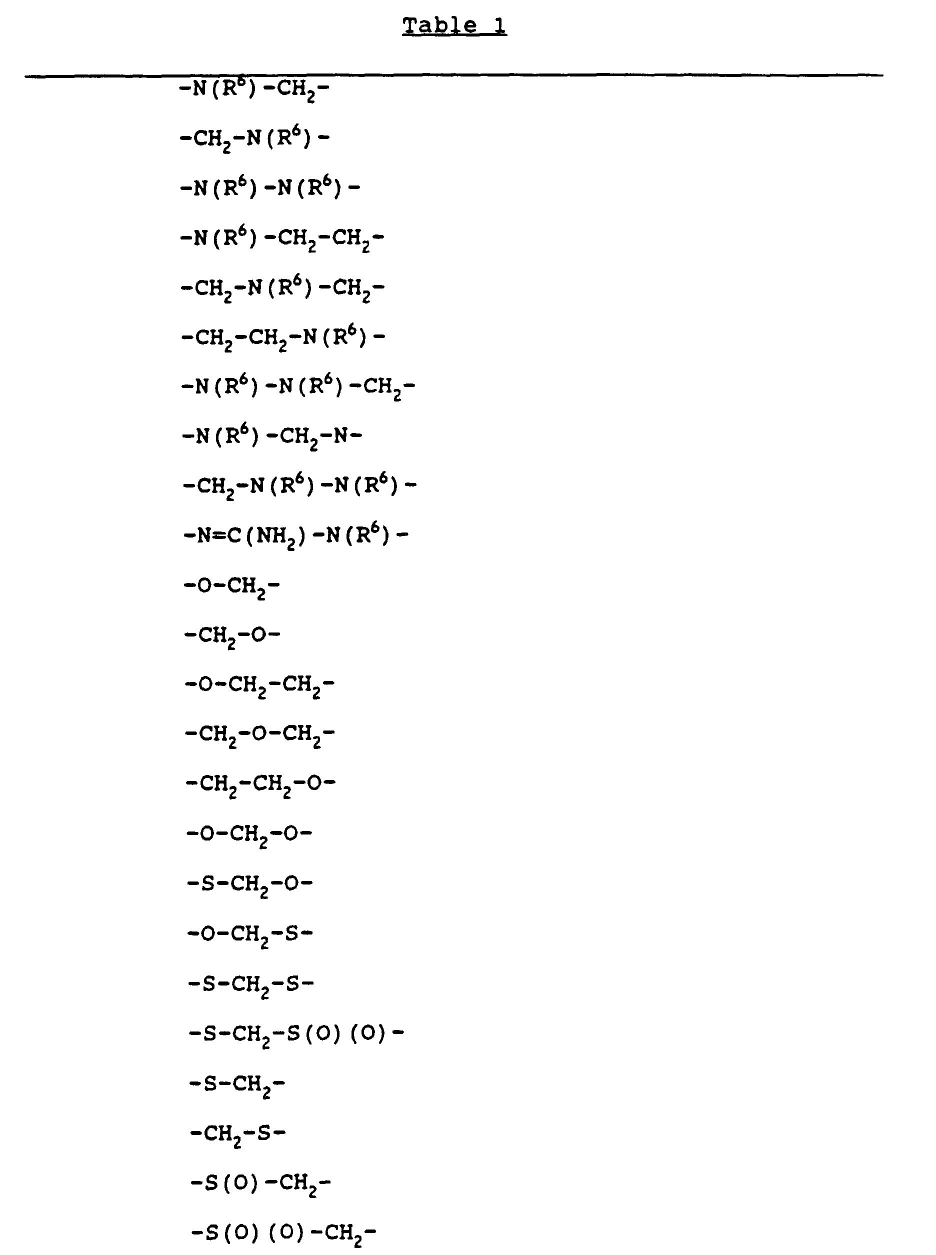

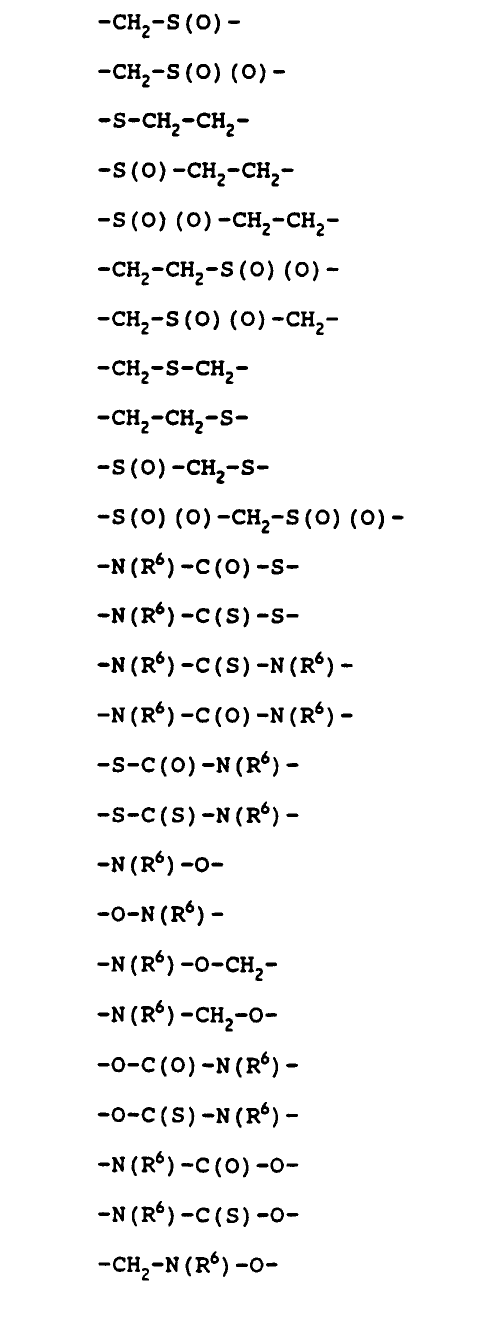

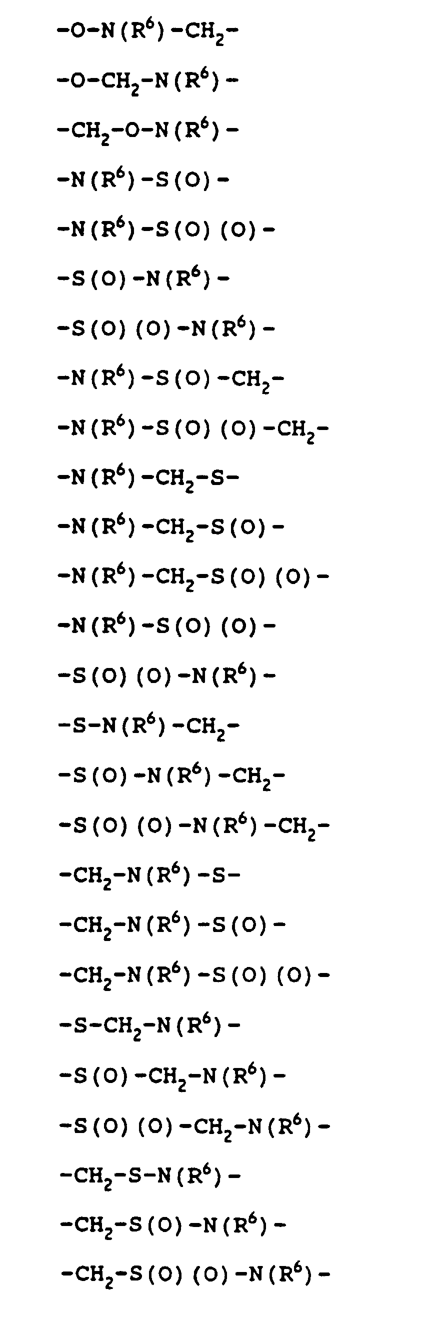

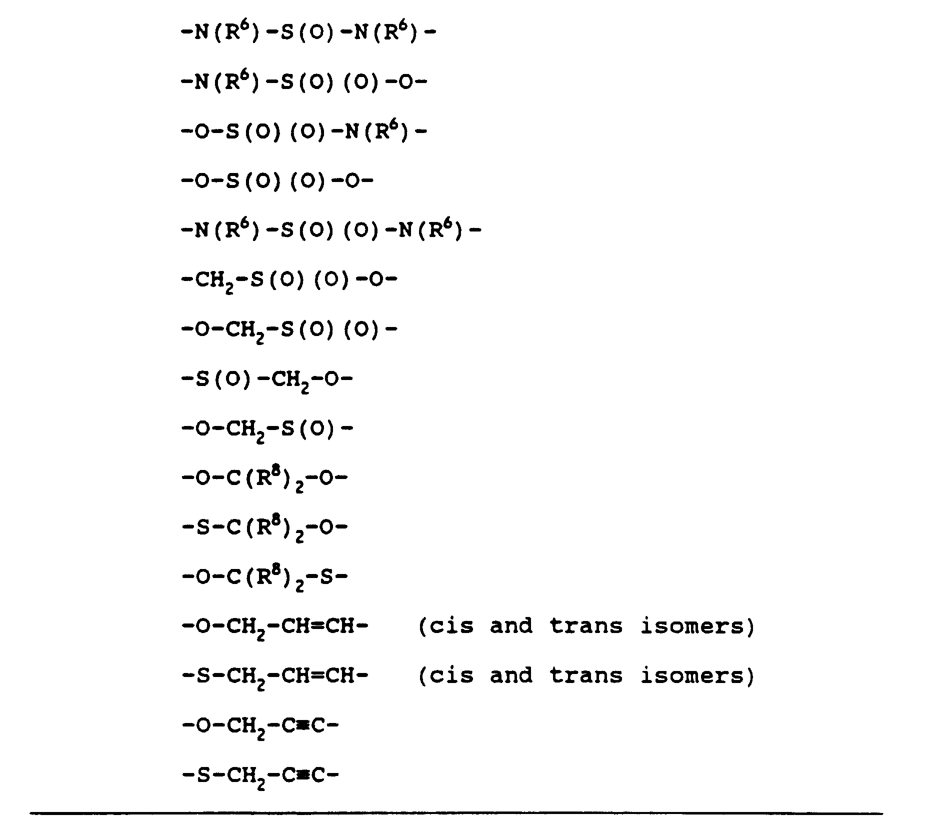

For the 2',5' linkages in Table 1, R⁶ is H, lower alkyl (1-6C), including methyl, ethyl, propyl, isopropyl and butyl, OMe, OH, heteroalkyl (1-6C, 1-2 halo,
S, N or O heteroatoms), or aryl (6-7C); and
wherein R⁸ is CH₂F, or when both R⁸ are taken together with the atom to which they are attached, form a 4-membered or 6-membered ring where (R⁸)₂ is

-CH₂-X¹-CH₂-,

-(CH₂)₂-X¹-(CH₂)₂-;

wherein X¹ is selected from the group consisting of NH, NMe, NEt, NPr, S, SO, SO₂, O, CF₂ and CHF.

Particularly preferred 2',5' linkages include

2' -S-CH₂-CH₂- 5'

2' -S-CH₂- 5'

2' -O-C(S)-NR⁶- 5'

2' -O-CH₂-S- 5'

2' -O-CH₂-O- 5'

2' -O-C(O)-NR⁶- 5'

2' -CH₂-CH₂-S 5'

2' -CH₂-S- 5'

2' -NR⁶-C(S)-O- 5'

2' -S-CH₂-O- 5'

2' -NR⁶-C(O)-O- 5'

2' -O-CH₂-CH=CH- 5'

and

2' -S-CH₂-CH=CH- 5'.

Contemplated equivalents of the invention 2',5' linkages include 3 or 4 atom substitute linkages where the methylene group at the 5' position is substituted with CO, CS, CNH₂, COH, CSH and the like as described in PCT/US92/04294, which is incorporated herein by reference.

It should be clear that the invention oligomers are not limited to oligomers of homogeneous linkage type, and that alternating or randomly distributed substitute linkages including the 2',5' linkages are included. Since the oligomers of the invention can be synthesized one nucleomonomer residue at a time, each individual linkage, and/or substitute linkage, and the nature of each individual "B" substituent can be chosen at will.

The oligomers of the invention may contain any desired number of the substitute linkages of the invention. These may be identical to each other or different by virtue of the embodiments chosen for Q including other noninvention substitute linkages. Since the oligomers are prepared sequentially, any pattern of linkage or substitute linkage types, bases and sugar modifications may be used.

In some preferred embodiments, the substitute linkages alternate in a regular pattern. For example, one invention substitute linkage followed by two phosphodiester linkages followed by one invention substitute linkage etc. Additional alternatives might include, for example, alternating linkages such as a an invention substitute linkage followed by a phosphodiester analog (thioate, etc) followed by an invention substitute linkage followed by a phosphodiester analog, etc., so that there is a one-by-one alternation of the two types of substitute linkages. A variety of different patterns is readily derived.

It is also clear that selected sugar modifications may be made to one or more nucleomonomer residues; however, for the most part, a 2'-5' nucleotide linkage between 2'-deoxyribose residues is most convenient. Where this is the case, further abbreviation of the structures may be used. For example, in standard DNA (or RNA) the sequences are generally denoted by the sequence of bases alone, such as, for example, ATG CGC TGA. In general, it is simply stated in advance whether this represents an RNA or DNA sequence. In the compounds of the invention, similar notation will be used for modifications of otherwise physiological DNA or RNA molecules but the 3',5' phosphodiester linkages replaced by the invention substitute linkages will be noted. Thus, 5'-TCTCme(O-C(O)-NR)TCme(O-C(O)-NR)TCme(O-C(O)-NR)TCme(O-C(O)-NR)TTTT-2' indicates an oligonucleotide TCTCmeTCmeTCmeTCmeTTTT (the Cme denoting 5-methylcytosine) with each of the four 3',5' phosphodiester linkages replaced in the noted positions with 2',5' substitute linkages.

We have found in general that several chemical advantages appear to be inherent to the 2',5' substitute linkages, at least when compared to the corresponding 3',5' linkages. These advantages provide the 2',5' with significantly more flexibility. Specifically, the 3'-deoxy sugar is synthesized de novo to be appended to any aglycone of choice; therefore, one is not limited to modifications of naturally occurring nucleosides. Also, the presence of a 2'-acyl group on the sugar (compound #108, scheme #16) insures the formation of the β-anomer preferentially, in good yield, when appended via the Vorbruggen method (Niedballa, U. et al., J Org Chem (1974) 39:3654). If the corresponding 3'-acyl-2'-deoxy carbohydrate was subjected to the same conditions, a mixture of α- and β-anomers would be obtained.

Furthermore, the presence of a 2'-hydroxyl group or oxygen is also known to stabilize the glycosidic linkage. Studies (See, Michelson, A.M. in "The Chemistry of Nucleosides and Nucleotides," 1963, Academic Press, N.Y., p. 26-27, and Garrett, E.R., Mehta, P.J., J. Am. Chem. Soc., 1972, 94, 8532) have shown that the 2'-deoxy nucleosides are 1000 times more susceptible to acid catalyzed deglycosylation when compared to the corresponding ribonucleoside. On the contrary, 3'-deoxy nucleosides are only 2-3 times less stable to acid-catalyzed deglycosylation when compared to the parent riboside. Therefore, the presence of the 2'-hydroxyl not only renders the nucleomonomer more stable to synthetic conditions, but also allows access into nucleomonomers containing a modified aglycone which otherwise would render the glycosidic bond too unstable for practical use.

### Additional Nucleomonomer Modifications

Oligomers that are comprised of nucleomonomers can also contain various modifications in addition to the substitute linkages of the invention. A non-limiting exemplary list of such additional modifications includes oligomers where (i) one or more nucleomonomer residues are modified at the 2' position, (ii) one or more covalent crosslinking moieties are incorporated, (iii) inverted polarity linkers (switchback linkers) are incorporated, (iv) other noninvention substitute linkages are included, (v) other base analogs, such as 8-oxo-N⁶-methyladenine, are included and (vi) conjugates such as intercalating agents or polylysine that respectively enhance binding affinity to target nucleic acid sequences or that enhance association of the oligomer with cells are included.

The binding competence of the invention oligomers for single-stranded and duplex targets is compatible with further modifications to the oligomer. These further modifications may also confer other useful properties such as stability to nuclease cleavage (e.g. in a domain of an invention oligomer having phosphodiester linkages), or enhance their ability to permeate cell membranes, and the like.

Also included are oligomers containing one or more substitute linkages such as sulfide or sulfone linkages (Benner, S.A., International Publication No. WO 89/12060), sulfamate linkages (International Publication No. WO 91/15500), carbamate or other substitute linkages in morpholino-linked oligomers (Stirchak, E.P. et al Nucleic Acids Res (1989) 17:6129-6141; Summerton, J., et al International Publication No. 216 860) and related linkages.

Thus, exemplary embodiments of invention oligomers include oligomers having (1) at least one substitute linkage and a 5-member or 6-member ring that is linked to an adjacent C4' atom through a tone, two or three atom bridge, and (2) one or more non-invention substitute linkages selected from the group consisting of pbosphorothioate, methylphosphonate and thionomethylphosphonate and/or (3) one or more phosphodiester linkages and/or (4) purine or pyrimidine analogs that enhance binding affinity for complementary target sequences. Other exemplary oligomers would include (1) an oligomer having invention substitute linkages at the 3' and/or 5' ends and phosphorothioate linkages elsewhere in the oligomer; (2) oligomers having invention substitute linkages and standard purine or pyrimidine bases (e.g. adenine, guanine, cytosine, thymine, or uracil); (3) oligomers having invention substitute linkages and one or more bases that enhance binding affinity or permeation competence of the oligomer (e.g. 5-methylcytosine, 5-(1-propynyl)uracil, 5-(1-propynyl)cytosine, and the like); and (4) oligomers having invention substitute linkages and one or more 2'-modified nucleomonomers (e.g. 2'-O-allyl, 2'-fluoro, and the like).

Related linkages such as amide linkages and 2',5' linkages are described in patent application numbers WO 93/24507 and WO 93/24508 incorporated herein by reference in their entirety.

Also included are oligomers containing nucleomonomer residues linked via amide bonds. Exemplary linkages have been described (Nielsen, P.E., et al, Science (1991) 254: 500; commonly owned patent application no. WO 93/24507, incorporated herein by reference).

### Oligomers

The oligomers of the invention can be formed using invention and conventional nucleomonomers and synthesized using standard solid phase (or solution phase) oligomer synthesis techniques, which are now commercially available. In general, the invention oligomers can be synthesized by a method comprising the steps of: synthesizing a nucleomonomer or oligomer synthon having a protecting group and a base and a coupling group capable of coupling to a nucleomonomer or oligomer; coupling the nucleomonomer or oligomer synthon to an acceptor nucleomonomer or an acceptor oligomer; removing the protecting group; and repeating the cycle as needed until the desired oligomer is synthesized.

The oligomers of the present invention can be of any length including those of greater than 40, 50, 100, 200 or 500 nucleomonomers. In general, preferred oligomers contain 2-30 nucleomonomers. Lengths of greater than or equal to about 8 to 20 nucleomonomers are useful for therapeutic or diagnostic applications. Short oligomers containing 2, 3, 4 or 5 nucleomonomers are specifically included in the present invention and are useful as synthons.

Oligomers having a randomized sequence and containing about 6,7 or 8 nucleomonomers are useful for primers that are used in cloning or amplification protocols that use random sequence primers, provided that the oligomer contains about 1 or 2 residues at the 3' end that can serve as a primer for polymerases or reverse transcriptases or that otherwise do not interfere with polymerase activity.

Oligomers can contain conventional phosphodiester linkages or can contain other noninvention substitute linkages such as phosphoramidate linkages in addition to the invention substitute linkages. These substitute linkages include, but are not limited to, embodiments wherein a moiety of the formula -O-P(O)(S)-O-("phosphorothioate"), -O-P(S)(S)-O-("phosphorodithioate"), -O-P(O)(NR¹¹₂)-X², -O-P(O)(R¹¹)-O-, -O-P(S)(R¹¹)-O- ("thionoalkylphosphonate"), -P(O)(OR⁹)-X², -O-C(O)-X², or -O-C(O)(NR¹¹₂)-X²-, wherein R¹¹ is H (or a salt) or alkyl (1-12C including methyl and ethyl) and R⁹ is alkyl (1-9C) and the linkage is joined to adjacent nucleomonomers through an -O- or -S- bonded to a carbon of the nucleomonomer and X² is O or S. Phosphorothioate and phosphodiester linkages are well known. Particularly preferred substitute linkages for use in the oligomers of the present invention include phosphodiester, phosphorothioate, methylphosphonate and thionomethylphosphonate substitute linkages. Phosphorothioate and methylphosphonate substitute linkages confer added stability to the oligomer in physiological environments. While not all such substitute linkages in the same oligomer need be identical, particularly preferred oligomers of the invention contain one or more phosphorothioate or methylphosphonate substitute linkages.

### Pharmaceutically Acceptable Salts

Any pharmaceutically acceptable salt can be used and such salt forming materials are well known in the art.

Pharmaceutically acceptable salts are preferably metal or ammonium salts of the oligomers of the invention and include alkali or alkaline earth metal salts, e.g., the sodium, potassium, magnesium or calcium salt; or advantageously easily crystallizing ammonium salts derived from ammonia or organic amines, such as mono-, di- or tri-lower (alkyl, cycloalkyl or hydroxyalkyl)-amides, lower alkylenediamines or lower (hydroxyalkyl or arylalkyl)-alkylammonium bases, e.g. methylamine, diethylamine, triethylamine, dicyclohexylamine, triethanolamine, ethylenediamine, tris-(hydroxymethyl)-aminomethane or benzyltrimethylammonium hydroxide. The oligomers of the invention form acid addition salts, which are preferably such of therapeutically acceptable inorganic or organic acids, such as strong mineral acids, for example hydrohalic, e.g., hydrochloric or hydrobromic acid; sulfuric, phosphoric; aliphatic or aromatic carboxylic or sulfonic acids, e.g., formic, acetic, propionic, succinic, glycollic, lactic, malic, tartaric, gluconic, citric, ascorbic, maleic, fumaric, hydroxymaleic, pyruvic, phenylacetic, benzoic, 4-aminobenzoic, anthranilic, 4-hydroxybenzoic, salicylic, 4-aminosalicylic, methanesulfonic, ethanesulfonic, hydroxyethanesulfonic, benzenesulfonic, sulfanilic or cyclohexylsulfamic acid and the like.

### Blocking Groups

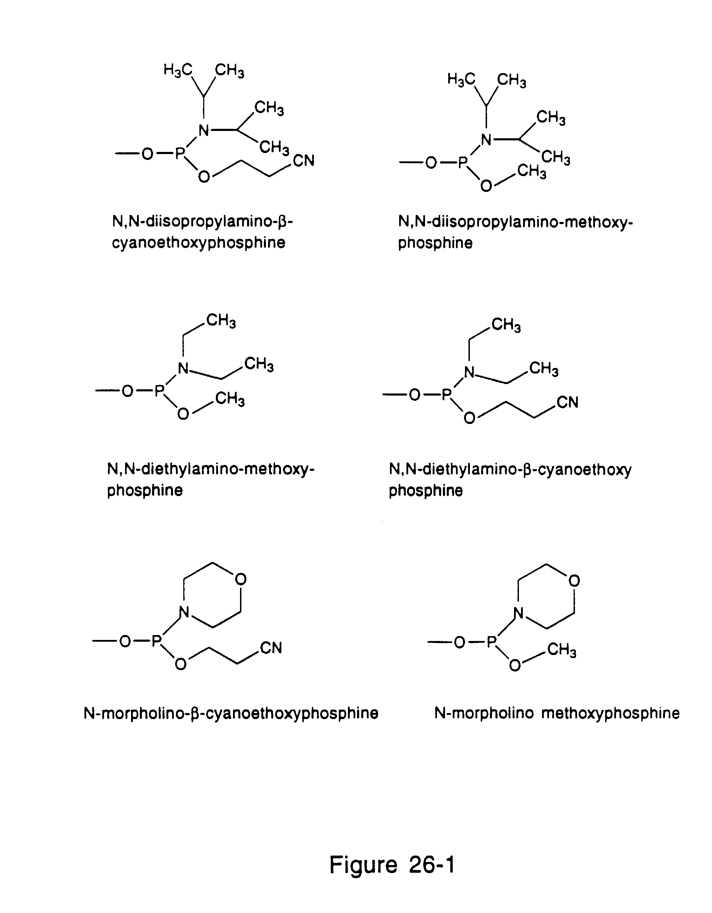

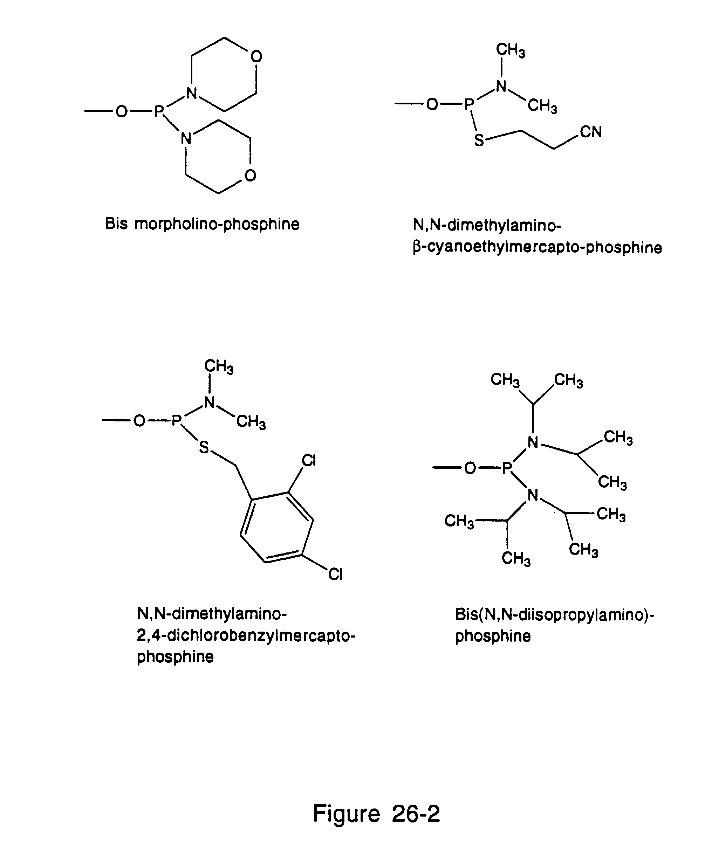

1. Coupling Groups. Suitable coupling groups are, for example, H-phosphonate, a methylphosphonamidite, or a phosphoramidite. Phosphoramidites that can be used include β-cyanoethylphosphoramidites (preferred). Methylphosphonamidites, alkylphosphonamidites (including ethylphosphonamidites and propylphosphonamidites) can also be used. Exemplary phosphoramidites are shown in Figures 26-1 and 26-2.

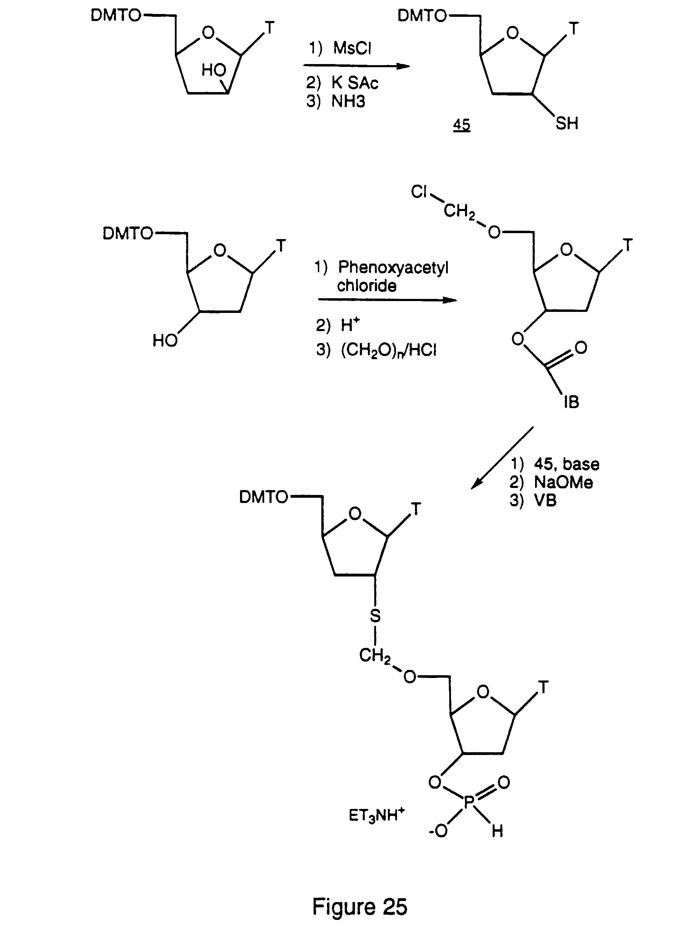

Suitable "coupling groups" at the 3', 2' or 5' position for oligomer synthesis via phosphoramidite triester chemistry, referred to herein as "amidite" chemistry, include N,N-diisopropylamino-β-cyanoethoxyphosphine, N,N-diisopropylaminomethoxyphosphine, N,N-diethylamino-β-cyanoethoxyphosphine, (N-morpholino)-β-cyanoethoxyphosphine, and (N-morpholino)-methoxyphosphine (Moore, M.F. et al, J Org Chem (1985) 50:2019-2025; Uznanski, A.W., et al, Tet Lett (1987) 28:3401-3404; Bjergarde, K., et al, Nucl Acids Res (1991)19:5843-5850; Dahl, O. Sulfur Reports (1991) 11:167-192). Related coupling groups such as N,N-diisopropylamino-methylphosphine or N,N-diethylamino-methyl-phosphine can also be used to prepare methylphosphonates (Fig 25-4). Methylphosphonate oligomers can be conveniently synthesized using coupling groups such as N,N-diisopropylamino-methylphosphonamidite, and N,N-diethylamino-methylphosponamidite. Synthesis of nucleomonomer amidites of the invention can be accomplished by conventional methods (for example, Gryaznov, S.M., et al, Nucl Acids Res (1992) 20:1879-1882; Vinayak, R., et al, Nucl Acids Res (1992) 20:1265-1269; Sinha, N.D., et al, Nucl Acids Res (1984) 12:4539-4557; and other references cited herein).

Suitable coupling groups at the 3', 2' (or 5') position for oligomer synthesis via phosphate triester chemistry, referred to herein as "triester" chemistry, include 2-chlorophenyl phosphate, 4-chlorophenyl phosphate, 2,4-dichlorophenyl phosphate and 2,4,-dibromophenyl phosphate nucleotide diester derivatives or, for synthesis of phosphorothioate linkages, the thiono derivatives thereof (Marugg, J.E., et al, Nucl Acids Res (1984) 12:9095-9110; Kemal, O., et al, J Chem Soc Chem Commun (1983) 591-593; Kamer, P.C.J., et al, Tet Lett (1989) 30:6757-6760).

2. Protecting Groups. Protecting groups such as diisobutylformamidine, benzoyl, isobutyryl, FMOC, dialkylformamidine, dialkylacetamidine or other groups known in the art can be used to protect the exocyclic nitrogen of the cytosine, adenine or guanine heterocycles. Alternatively, cytidine can be directly incorporated into oligomers without a protecting group at the exocyclic nitrogen using described methods (Gryaznov, S.M. et al, J Amer Chem Soc (1991) 113:5876-5877; Gryaznov, S.M., et al, Nucl Acids Res (1992) 20:1879-1882; Kung, P.-P., et al, Tetrahedron Letters (1992) 33:5869-5872).

Suitable protecting groups are DMT (dimethoxy trityl), Bz (benzoyl), iBu (isobutyryl), phenoxyacetyl, MMT (monomethoxytrityl) or FMOC at the 5' terminus and/or hydrogen phosphonate, methyl phosphoramidite, methyl phosphonamidite, β-cyanoethylphosphoramidite, TBS (t-butyldimethylsilyl) or TBDPS (t-butyldiphenylsilyl) at the 3'- terminus.

Preferred protecting groups are Bz (benzoyl), DMT (dimethoxytrityl), MMT (monomethoxytrityl) or FMOC at the 5' terminus or position and/or TBS, hydrogen phosphonate, methylphosphoramidite, methylphosphonamidite, β-cyanoethylphosphoramidite at the 3'-terminus. However, it is intended that the position of the blocking groups can be reversed as needed (e.g., a phosphoramidite at the 5'- position and DMT at the 3'-position). In general, the nucleomonomers and oligomers of the invention can be derivatized to such "blocking groups" as indicated in the relevant formulas by methods known in the art.

### Conjugates

Also included are "conjugates" of oligomers. "Conjugates" of the oligomers include those conventionally recognized in the art. For instance, the oligomers can be covalently linked to various moieties such as, intercalators, and substances which interact specifically with the minor groove of the DNA double helix. Other chosen conjugate moieties, can be labels such as radioactive, fluorescent, enzyme, or moieties which facilitate cell association using cleavable linkers and the like. Suitable radiolabels include ³²P, ³⁵S, ³H, ¹³¹I and ¹⁴C; and suitable fluorescent labels include fluorescein, resorufin, rhodamine, BODIPY (Molecular Probes) and texas red; suitable enzymes include alkaline phosphatase and horseradish peroxidase. Other compounds which can be used as covalently linked moieties include biotin, antibodies or antibody fragments, asialoglycoprotein, transferrin and the HIV Tat protein can also conveniently be linked to the oligomers of the invention.

These additional moieties can be derivatized through any convenient moiety. For example, intercalators, such as acridine or psoralen can be linked to the oligomers of the invention through any available -OH or -SH, e.g., at the terminal 5'- position of the oligomer, the 2'- positions of RNA, or an OH, NH₂, COOH or SH incorporated into the 5- position of pyrimidines. A derivatized form which contains, for example, -CH₂CH₂NH₂, -CH₂CH₂CH₂OH or -CH₂CH₂CH₂SH in the 5- position of pyrimidines is convenient. Conjugates including polylysine or lysine can be synthesized as described and can further enhance the binding affinity of an oligomer to its target nucleic acid sequence (Lemaitre, M. et al., Proc Natl Acad Sci (1987) 84:648-652; Lemaitre, M. et al., Nucleosides and Nucleotides (1987) 6:311-315).

A wide variety of substituents can be attached, including those bound through linkages or substitute linkages. The -OH moieties in the oligomers can be replaced by phosphate groups, protected by standard protecting groups, or coupling groups to prepare additional linkages to other nucleomonomers, or can be bound to the conjugated substituent. The 5'- terminal OH can be phosphorylated; the 2'-OH or OH substituents at the 3'- terminus can also be phosphorylated. The hydroxyls can also be derivatized to standard protecting groups.

Oligomers of the invention can be covalently derivatized to moieties that facilitate cell association using cleavable linkers. Linkers used for such conjugates can include disulfide linkages that are reduced after the oligomer-transport agent conjugate has entered a cell. Appropriate molecular linkers include for example, -Y¹-X⁸CH₂CHR¹⁰-SS-CHR¹⁰CH₂X⁸-Y¹- wherein each Y¹ is independently alkylene (1-9C; including methylene, ethylene and propylene), or CO, each X⁸ is independently O, S(O)(O), S(O), NR¹⁰, CH₂, C(R¹⁰)₂ or CO; R¹⁰ wherein each R¹⁰ is independently H, alkyl (1-6C; including methyl, ethyl and propyl), or 6-10C aryl and which linkers have been previously described (International Publication No. WO 91/14696). Disulfide-containing linkers of this type have a controllable t_{1/2} in vivo, facilitating its use as a prodrug/transport component. Such linkers are stable under extracellular conditions relative to intracellular conditions due to the redox potential of the disulfide linkage.

Suitable conjugates also include solid supports for oligomer synthesis and to facilitate detection of nucleic acid sequences. Solid supports include, but are not limited to, silica gel, controlled pore glass, polystyrene, and magnetic glass beads.

### Sugar Modifications

Derivatives can be made by substitution on the sugars. Among the preferred derivatives of the oligomers of the invention are the 2'-0-allyl or 3'-0-allyl derivatives. The presence of the 2'- or 3'-O-allyl group appears to enhance permeation ability and stability to nuclease degradation, but does not appear to diminish the affinity of the oligomer for single chain or duplex targets.

Furthermore, as the a anomer binds to duplex DNA or single-stranded RNA in a manner similar to that for the β anomers but with a reversed polarity, oligomers can contain nucleomonomers having this epimer or a domain thereof (Praseuth, D., et al., Proc Natl Acad Sci (USA) (1988) 85:1349-1353; Sun, J.S. et al, Proc Natl Acad Sci (1991) 88:6023-6027; Debart, F., et al, Nucl Acids Res (1992) 20:1193-1200). α-Anomeric oligomers containing the substitute linkages described herein represent a class of modified oligomers included in the present invention.

### Noninvention Substitute Linkages

The oligomers of the invention can also contain one or more "substitute linkages", in addition to the 2',5' linkages disclosed herein, which are generally understood in the art. These "substitute linkages" include phosphorothioate, methylphosphonate, thionomethylphosphonate, phosphorodithioate, alkylphosphonates, morpholino carbamate, morpholino sulfamate, morpholino sulfamide, boranophosphate (-O-P(OCH₃)(BH₃)-O-), siloxane (-O-Ci(X⁴)(X⁴)-O-; X⁴ is 1 - 6C alkyl or phenyl) and phosphoramidate (methoxyethylamine (-O-P(OCH₂CH₂OCH₃) (O)-O-) and the like), and are synthesized as described in the generally available literature including the following references (Sood, A., et al, J Am Chem Soc (1990) 112:9000-9001; WO 91/08213; WO 90/15065; WO 91/15500; Stirchak, E.P. et al Nucleic Acid Res (1989) 17:6129-614 U.S. Patent 5,034,506; U.S. Patent 5,142,047; Hewitt, J. et al, Nucleosides and Nucleotides (1992) 11:1661-1666; Summerton, J., et al International Publication No. 216 860). Substitute linkages that can be used in the oligomers disclosed herein also include the sulfonamide (-O-SO₂-NH-), sulfide (-CH₂-S-CH₂-), sulfonate (-O-SO₂-CH₂-), carbamate (-O-C(O)-NH-, -NH-C(O)-O-), dimethylhydrazino (-CH₂-NCH₃-NCH₃-), sulfamate (-O-S(O)(O)-N-; -N-S(O)(O)-N-), 3'-thioformacetal (-S-CH₂-O-), formacetal (-O-CH₂-O-), 3'-amine (-NH-CH₂-CH₂-), N-methylhydroxylamine (-CH₂-NCH₃-O-) and 2'5' linkages (such as 2',5' carbamate (2' -N(H)-C(O)-O- 5'), 5',2' carbamate (2' -O-C(O)-N(H)- 5'), 5',2' methylcarbamate (2' -O-C(O)-N(CH₃)- 5') and 5',2' thioformacetal (2' -O-CH₂-S- 5'). Substitute linkages are disclosed and claimed in U.S. Patent no. 5,264,562 and application no. WO 92/05186 incorporated herein by reference in their entirety. Additional substitute linkages that are suitable include amide linkages described by Buchardt, O. et al, (International Publication No. WO 92/20702), and those described by Cook, P.D. et al, (International Publication No. WO 92/20822), De Mesmaeker, A. at al, (International Publication No. WO 92/20823) and as described in PCT/US92/04294.

Except where specifically indicated, the substitute linkages, such as a formacetal linkage, -O-CH₂-O-, are linked to either the 3' or 2' carbon of a nucleomonomer on the left side and to the 5' carbon of a nucleomonomer on the right side. Thus a formacetal linkage can be indicated as 3' -O-CH₂-O- 5' or 2' -O-CH₂-O- 5'. The designations of a 3', 2' or 5' carbon can be modified accordingly when a structure other than ribose, deoxyribose or arabinose is linked to an adjacent nucleomonomer. Such structures include xylose, a hexose, morpholino ring, carbocyclic ring (e.g. cyclopentane) and the like.

The use of carbamate, carbonate, sulfide, sulfoxide, sulfone, N-methylhydroxylamine and dimethylhydrazino linkages in synthons or oligomers has been described (Vaseur, J-J. et al, J Amer Chem Soc (1992) 114:4006-4007; WO 89/12060; Musicki, B. et al, J Org Chem (1990) 55:4231-4233; Reynolds, R. C., et al J Org Chem (1992) 57:2983-2985; Mertes, M. P., et al, J Med Chem (1969) 12:154-157; Mungall, W. S., et al, J Org Chem (1977) 42:703-706; Stirchak, E. P., et al, J Org Chem (1987) 52:4202-4206; Wang, H., et al, Tet Lett (1991) 32:7385-7388; International Application No. PCT US91/03680). Substitute linkage(s) can be utilized in the oligomers for a number of purposes such as to further facilitate binding with complementary target nucleic acid sequences and/or to increase the stability of the oligomers toward nucleases.

### Nucleosides

Exemplary nucleosides suitable for synthesis of amide linked nucleomonomers have been described (Nielsen, P.E. ibid; Buchardt, O. et al, International Publication No. WO 92/20702; commonly owned patent application no. WO 93/24507, and incorporated herein by reference in its entirety).

"Nucleosides" also include those moieties which contain modifications of the sugar, for example, wherein one or more of the hydroxyl groups are replaced with halogen, aliphatic groups, or functionalized as ethers, amines, and the like. Such structures include a hexose, morpholino ring, carbocyclic ring (e.g. cyclopentane) and the like.

### Base

Suitable bases for use within the present invention include not only the known purine and pyrimidine bases, but also analogs of these heterocyclic bases and tautomers thereof. Such analogs include alkylated purines or pyrimidines, acylated purines or pyrimidines, or other heterocycles. Such "analogous purines" and "analogous pyrimidines" or purine or pyrimidine analogs are those generally known in the art, some of which are used as chemotherapeutic agents. An exemplary, but not exhaustive, list includes N⁴,N⁴-ethanocytosine, 7-deazaxanthosine, 7-deazaguanosine, 8-oxo-N⁶-methyladenine, 4-acetylcytosine, 5-(carboxyhydroxylmethyl) uracil, 5-fluorouracil, 5-bromouracil, 5-carboxymethylaminomethyl-2-thiouracil, 5-carboxymethylaminomethyl uracil, inosine, N⁶-isopentenyl-adenine, 1-methyladenine, 2-methylguanine, 5-methylcytosine, N⁶-methyladenine, 7-methylguanine, 5-methylaminomethyl uracil, 5-methoxy aminomethyl-2-thiouracil, 5-methoxyuracil, pseudouracil, 5-methyl-2-thiouracil, 2-thiouracil, 4-thiouracil, 5-(1-propynyl)-4-thiouracil, 5-(1-propynyl)-2-thiouracil, 5-(1-propynyl)-2-thiocytosine, 2-thiocytosine, and 2,6-diaminopurine. In addition to these base analogs, pyrimidine analogs including 6-azacytosine, 6-azathymidine and 5-trifluoromethyluracil described in Cook, D. P., et al, International Publication No. WO 92/02258 (incorporated herein by reference) can be conveniently incorporated into the invention oligomers.

Incorporation of 4-thiouridine and 2-thiothymidine into oligomers has been described (Nikiforov, T. T., et al, Tet Lett (1992) 33:2379-2382; Clivio, P., et al Tet Lett (1992) 33:65-68; Nikiforov, T. T., et al, Tet Lett (1991) 32:2505-2508; Xu, Y.-Z., et al Tet Lett (1991) 32:2817-2820; Clivio, P., et al Tet Lett (1992) 33:69-72; Connolly, B.A., et al., Nucl. Acids Res. (1989) 17:4957-4974).

Preferred bases include adenine, guanine, thymine, uracil, cytosine, 5-methylcytosine, 5-(1-propynyl)uracil, 5-(1-propynyl)cytosine, 8-oxo-N⁶-methyladenine, 7-deaza-7-methylguanine, 7-deaza-7-methyladenine and 7-deazaxanthosine. Synthesis and use of oligomers that bind to duplex DNA sequences via GT binding motif containing 7-deazaxanthosine is described in patent application no.WO 93/09127 which is incorporated herein by reference in its entirety.

### Covalent Bonding Moiety

Included in some of the oligomers of the invention is a moiety which is capable of effecting at least one covalent bond between the oligomer and the duplex. Multiple covalent bonds can also be formed by providing a multiplicity of such crosslinking moieties. The covalent bond is preferably to a base residue in the target strand, but can also be made with other portions of the target, including the saccharide or phosphodiester. The reaction nature of the moiety which effects crosslinking determines the nature of the target in the duplex. Preferred crosslinking moieties include acylating and alkylating agents, and, in particular, those positioned relative to the sequence specificity-conferring portion so as to permit reaction with the target location in the strand. Crosslinking moieties are disclosed and claimed in commonly owned pending application no. 640,654.

In one embodiment of the invention, a switchback oligonucleotide containing crosslinking moieties at either end can be used to bridge the strands of the duplex with at least two covalent bonds. In addition, nucleotide sequences of inverted polarity can be arranged in tandem with a multiplicity of crosslinking moieties to strengthen the complex. Exemplary of crosslinking moieties that are useful in the invention include N⁴,N⁴-ethanocytosine and N⁶,N⁶-ethanoadenine.

It is clear that the heterocycle need not be a purine or pyrimidine; indeed the pseudo-base to which the reactive function is attached need not be a heterocycle at all. Any means of attaching the reactive group is satisfactory so long as the positioning is correct.

### Inverted Polarity

In their most general form, inverted polarity oligomers, that can incorporate one or more nucleomonomers described above, contain at least one segment along their length of the formula:

3'----→5'--C--5'-----3' (1)

or

5'---→3'--C--3'-----5' (2)

where -C- symbolizes any method of coupling the nucleomonomer sequences of opposite polarity (Froehler, B.C., et al Biochemistry (1992) 31:1603-1609; Horne, D.A., et al J Am Chem Soc (1990) 112:2435-2437; Beal, P.A., et al J Am Chem Soc (1992) 114:4976-4978).

In these formulas, the symbol 3'----5' indicates a stretch of oligomer in which the linkages are consistently formed between the 5'- hydroxyl of the ribosyl residue of the nucleomonomer to the left with the 3'- (or 2'- for oligomers having 2', 5' linkages) hydroxyl of the ribosyl residue of the nucleomonomer to the right (i.e., a region of uniform polarity), thus leaving the 5'- hydroxyl of the rightmost nucleomonomer ribosyl residue free for additional conjugation. Analogously, 5'----3' indicates a stretch of oligomer in the opposite orientation wherein the linkages are formed between the 3'- hydroxyl of the ribosyl residue of the left nucleomonomer and the 5'- hydroxyl of the ribosyl residue of the nucleomonomer on the right, thus leaving the 3'- hydroxyl of the rightmost nucleomonomer ribosyl residue free for additional conjugation.

The linkage, symbolized by -C-, can be formed so as to link the 5'- hydroxyls of the adjacent ribosyl residues in formula (1) or the 3' hydroxyls of the adjacent ribosyl residues in formula (2), or the "-C-" linkage can conjugate other portions of the adjacent nucleomonomers so as to link the inverted polarity strands. "-C-" can represent a linker moiety, or simply a covalent bond.

It should be noted that if the linkage between strands of inverted polarity involves a sugar residue, either the 3'- or 2'- position can be involved in the linkage, and either of these positions can be in either R or S configuration. The choice of configuration will in part determine the geometry of the oligomer in the vicinity of the linkage. Thus, for example, if adjacent 3'- positions are used to effect a covalent linkage, less severe deformation of the oligomer chain will generally occur if both 3'- hydroxyls involved in the linkage are in the conventional R configuration. If they are both in the S configuration, this will result in a favorable "kink" in the chain.

### 2' Modified Oligomers

Oligomers within the present invention include nucleomonomers having modifications of the ribose or deoxyribose sugar. 2'-O-methyl-, 2'-O-ethyl- and 2'-O-allyl oligomers have been synthesized and shown to bind to single-stranded complementary nucleic acid sequences (Cotten, M., et al., Nucleic Acids Res (1990) 19:2629-2635; Blencowe, B.J., et al., Cell (1989) 59:531-539; Sproat, B.S., et al., Nucleic Acids Res (1989) 17:3373-3386; Inoue, H., et al., Nucleic Acids Res (1987) 15:6131-6148; Morisawa, H., et al., European Patent Serial No. 0339842; Chavis, C., et al., J Organic Chem (1982) 47:202-206; Sproat, B.S., et al, Nucleic Acids Res (1991) 19:733-738). The 2'-modified oligomers were reported to be relatively nuclease stable compared to unmodified controls. Synthesis of 2' fluoro nucleomonomers and their incorporation into oligomers has also been described (Codington, J.F., et al, J Org Chem (1964) 29:558-564; Fazakerley, G.V., et al, FEBS Lett (1985) 182:365-369). Synthesis of oligomer analogs containing the modified bases described herein would be based on methods described. Synthesis of oligomers containing 2'- amino nucleomonomers has been described (Pieken, W.A., et al, Science (1991) 253:314-317).

In an additional use of substitute linkages of the invention, 2'- or 3'-O-allyl modified sugar forms of the nucleomonomers can be included in the oligomer. The 2'- and 3'-0-allyl nucleomonomers can be prepared and incorporated into oligomers using standard methods.

### Synthesis

Oligomers or the segments thereof are conventionally synthesized. The synthetic methods known in the art and described herein can be used to synthesize oligomers containing substitute linkages of the invention, as well as other linkages or substitute linkages known in the art, using appropriately protected nucleomonomers. Methods for the synthesis of oligomers having phosphorous containing linkages are found, for example, in Froehler, B., et al., Nucleic Acids Res (1986) 14:5399-5467; Nucleic Acids Res (1988) 16:4831-4839; Nucleosides and Nucleotides (1987) 6:287-291; Froehler, B., Tetrahedron Letters (1986) 27:5575-5578; Caruthers, M. H. in Oligodeoxynucleotides-Antisense Inhibitions of Gene Expression (1989), J. S. Cohen, editor, CRC Press, Boca Raton, p7-24; Reese, C.B. et al, Tetrahedron Letters (1985) 26:2245-2248. Synthesis of the methylphosphonate linked oligomers via methyl phosphonamidite chemistry has also been described (Agrawal, S. et al., Tetrahedron Letters (1987) 28:3539-3542; Klem, R. E., et al, International Publication Number WO 92/07864).

Oligomers containing nonphosphorous based substitute linkages that have been previously described in commonly owned US Patent no. 5,495,009 and commonly owned international publications WO 91/06629 and WO 92/05186, are preferably synthesized using suitably blocked dimer synthons as a starting material. Oligomers containing linkages of the present invention are also conveniently synthesized by preparation of dimer or trimer compounds by solution phase chemistry followed by conversion of the synthon to a derivative that is incorporated into oligomers by either solid or solution phase chemistry. Typical synthons are 5' DMT or MMT blocked 3' phosphonate or phosphoramidate derivatives which are prepared by standard methods (see: Gait, M.J. ed., Oligonucleotide Synthesis; A Practical Approach (1984) IRL Press, Oxford).

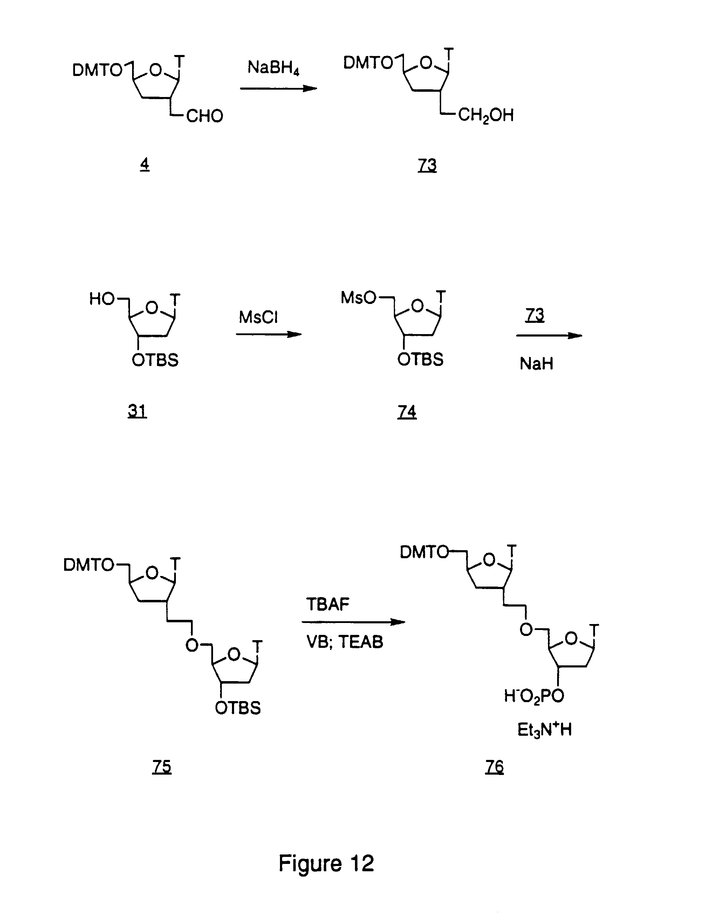

Oligomers having phosphorous-containing linkages or segments thereof are conventionally synthesized. Methods known in the art and described herein can be used to synthesize oligomers containing bases of the invention, as well as other bases known in the art, using appropriately protected nucleomonomers (see Figure 12). Methods for the synthesis of oligomers are found, for example, in Froehler, B., et al., Nucleic Acids Res (1986) 14:5399-5467; Nucleic Acids Res (1988) 16:4831-4839; Nucleosides and Nucleotides (1987) 6:287-291; Froehler, B., Tetrahedron Letters (1986) 27:5575-5578; Caruthers, M. H. in Oligodeoxynucleotides-Antisense Inhibitions of Gene Expression (1989), J. S. Cohen, editor, CRC Press, Boca Raton, p7-24; Reese, C.B. et al, Tetrahedron Letters (1985) 26:2245-2248. Synthesis of the methylphosphonate linked oligomers via methyl phosphonamidite chemistry has also been described (Agrawal, S. et al., Tetrahedron Letters (1987) 28:3539-3542; Klem, R. E., et al, International Publication Number WO 92/07864).

Synthons that are included in the scope of the present invention include dimers, trimers, tetramers, hexamers and longer oligomers made by solid or solution phase synthesis. Trimers and longer synthons may contain more than one type of linkage. The synthons may include any base as described above or 2', 3' and 5' groups such as OH, DMTO, MMTO, O-allyl, phosphate, a phosphonate or an amidite as described above.

### Utility and Administration

As the oligomers of the invention are capable of significant single-stranded or double-stranded target nucleic acid binding activity to form duplexes, triplexes or other forms of stable association, these oligomers are useful in diagnosis and therapy of diseases that are associated with expression of one or more genes such as those associated with pathological conditions. Therapeutic applications can employ the oligomers to specifically inhibit the expression of genes (or inhibit translation of RNA sequences encoded by those genes) that are associated with either the establishment or the maintenance of a pathological condition. Exemplary genes or RNAs encoded by those genes that can be targeted include those that encode enzymes, hormones, serum proteins, transmembrane proteins, adhesion molecules (LFA-1, GPII_{b}/IIIₐ, ELAM-1, VACM-1, ICAM-1, E-selectin, and the like), receptor molecules including cytokine receptors, cytokines (IL-1, IL-2, IL-3, IL-4, IL-6 and the like), oncogenes, growth factors, and interleukins. Target genes or RNAs can be associated with any pathological condition such as those associated with inflammatory conditions, cardiovascular disorders, immune reactions, cancer, viral infections, bacterial infections, yeast infections, parasite infections and the like.

Oligomers of the present invention are suitable for use in both in vivo and ex vivo therapeutic applications. Indications for ex vivo uses include treatment of cells such as bone marrow or peripheral blood in conditions such as leukemia (chronic myelogenous leukemia, acute lymphocytic leukemia) or viral infection. Target genes or RNAs encoded by those genes that can serve as targets for cancer treatments include oncogenes, such as ras, k-ras, bcl-2, c-myb, bcr, c-myc, c-abl or overexpressed sequences such as mdm2, oncostatin M, IL-6 (Kaposi's sarcoma), HER-2 and translocations such as bcr/abl. Viral gene sequences or RNAs encoded by those genes such as polymerase or reverse transcriptase genes of herpesviruses such as CMV, HSV-1, HSV-2, retroviruses such as HTLV-1, HIV-1, HIV-2, or other DNA or RNA viruses such as HBV, HPV, VZV, influenza virus, adenoviruses, flaviviruses, rhinovirus and the like are also suitable targets. Application of specifically binding oligomers can be used in conjunction with other therapeutic treatments. Other therapeutic indications for oligomers of the invention include (1) modulation of inflammatory responses by modulating expression of genes such as IL-1 receptor,
IL-1, ICAM-1 or E-Selectin that play a role in mediating inflammation and (2) modulation of cellular proliferation in conditions such as arterial occlusion (restenosis) after angioplasty by modulating the expression of (a) growth or mitogenic factors such as non-muscle myosin, myc, fos, PCNA, PDGF or FGF or their receptors, or (b) cell proliferation factors such as c-myb. Other suitable proliferation factors or signal transduction factors such as TGFα, IL-6, γINF, protein kinase C, tyrosine kinases (such as p210, p190), may be targeted for treatment of psoriasis or other conditions. In addition, EGF receptor, TGFa or MHC alleles may be targeted in autoimmune diseases.

Delivery of oligomers of the invention into cells can be enhanced by any suitable method including calcium phosphate, DMSO, glycerol or dextran transfection, electroporation or by the use of cationic anionic and/or neutral lipid compositions or liposomes by methods described (International Publication Nos. WO 90/14074, WO 91/16024, WO 91/17424, U.S. Patent 4,897,355). The oligomers can be introduced into cells by complexation with cationic lipids such as DOTMA (which may or may not form liposomes) which complex is then contacted with the cells. Suitable cationic lipids include but are not limited to N-(2,3-di(9-(Z)-octadecenyloxyl))-prop-1-yl-N,N,N-trimethylammonium (DOTMA) and its salts, 1-O-oleyl-2-O-oleyl-3-dimethylaminopropyl-β-hydroxyethylammonium and its salts and 1,2-bis(oleyloxy)-3-(trimethylammonio) propane and its salts.

Enhanced delivery of the invention oligomers can also be mediated by the use of (i) viruses such as Sendai virus (Bartzatt, R., Biotechnol Appl Biochem (1989) 11:133-135) or adenovirus (Wagner, E., et al, Proc Natl Acad Sci (1992) 89:6099-6013; (ii) polyamine or polycation conjugates using compounds such as polylysine, protamine or N1, N12-bis(ethyl)spermine (Wagner, E., et al, Proc Natl Acad Sci (1991) 88:4255-4259; Zenke, M., et al, Proc Natl Acad Sci (1990) 87:3655-3659; Chank, B.K., et al, Biochem Biophys Res Commun (1988) 157:264-270; U.S. Patent 5,138,045); (iii) lipopolyamine complexes using compounds such as lipospermine (Behr, J.-P., et al, Proc Natl Acad Sci (1989) 86:6982-6986; Loeffler, J.P., et al J Neurochem (1990) 54:1812-1815); (iv) anionic, neutral or pH sensitive lipids using compounds including anionic phospholipids such as phosphatidyl glycerol, cardiolipin, phosphatidic acid or phosphatidylethanolamine (Lee, K.-D., et al, Biochim Biophys ACTA (1992) 1103:185-197; Cheddar, G., et al, Arch Biochem Biophys (1992) 294:188-192; Yoshimura, T., et al, Biochem Int (1990) 20:697-706); (v) conjugates with compounds such as transferrin or biotin or (vi) conjugates with compounds such as serum proteins (including albumin or antibodies), glycoproteins or polymers (including polyethylene glycol) that enhance pharmacokinetic properties of oligomers in a subject. As used herein, transfection refers to any method that is suitable for delivery of oligomers into cells. Any reagent such as a lipid or any agent such as a virus that can be used in transfection protocols is collectively referred to herein as a "permeation enhancing agent". Delivery of the oligomers into cells can be via cotransfection with other nucleic acids such as (i) expressable DNA fragments encoding a protein(s) or a protein fragment or (ii) translatable RNAs that encode a protein(s) or a protein fragment.

The oligomers can thus be incorporated into any suitable formulation that enhances delivery of the oligomers into cells. Suitable pharmaceutical formulations also include those commonly used in applications where compounds are delivered into cells or tissues by topical administration. Compounds such as polyethylene glycol, propylene glycol, azone, nonoxonyl-9, oleic acid, DMSO, polyamines or lipopolyamines can be used in topical preparations that contain the oligomers.

The invention oligomers can be conveniently used as reagents for research or production purposes where inhibition of gene expression is desired. There are currently very few reagents available that efficiently and specifically inhibit the expression of a target gene by any mechanism. Oligomers that have been previously reported to inhibit target gene expression frequently have nonspecific effects and/or do not reduce target gene expression to very low levels (less than about 40% of uninhibited levels).

Thus, the oligomers as described herein constitute a reagent that can be used in methods of inhibiting expression of a selected protein or proteins in a subject or in cells wherein the proteins are encoded by DNA sequences and the proteins are translated from RNA sequences, comprising the steps of: introducing an oligomer of the invention into the cells; and permitting the oligomer to form a triplex with the DNA or RNA or a duplex with the DNA or RNA whereby expression of the protein or proteins is inhibited. The methods and oligomers of the present invention are suitable for modulating gene expression in both procaryotic and eucaryotic cells such as bacterial, fungal parasite, yeast and mammalian cells.

RNase H "competent" or RNase H "incompetent" oligomers can be easily designed using the substitute linkages of the invention. RNase H competent oligomers can comprise one or more RNase H competent domains comprised of linked RNase H competent nucleomonomers. Oligomers having modifications such as 2'- substitutions (2'-O-allyl and the like) or certain uncharged linkages (methylphosphonate, phosphoramidate and the like) are usually incompetent as a substrate that is recognized by and/or acted on by RNase H. RNase H competence can facilitate antisense oligomer function by degrading the target RNA in an RNA-oligomer duplex (Dagle, J.M., et al, Nucl Acids Res (1990) 18:4751-4757; Walder, J.A. et al, International Publication Number WO 89/05358). The enzyme cleaves RNA in RNA-DNA duplexes.

In order to retain RNase H competence, an oligomer requires a RNase H competent domain of three or more competent contiguous nucleomonomers located within it (Quartin, R.S., et al, Nucl Acids Res (1989) 17:7253-7262). Design of oligomers resistant to nuclease digestion will have terminal linkage, sugar and/or base modifications to effect nuclease resistance. Thus, the oligomers can be designed to have modified nucleomonomer residues at either or both the 5'- and/or 3'- ends, while having an internal RNase H competent domain. Exemplary oligomers that retain RNase H competence would generally have uniform polarity and would comprise about 2 to about 12 nucleomonomers at the 5'- end and at the 3'- end which stabilize the oligomer to nuclease degradation and about three to about 26 nucleomonomers that function as a RNase H competent domain between the RNase H incompetent 3' and 5'- ends. Variations on such an oligomer would include (1) a shorter RNase H competent domain comprising 1 or 2 RNase H competent linkages or substitute linkages, (2) a longer RNase H incompetent domain comprising up to 15, 20 or more substitute linkages or nucleomonomers, (3) a longer RNase H competent domain comprising up to 30, 40 or more linkages, (4) oligomers with only a single RNase H incompetent domain at the 3' end or at the 5' end, or (5) oligomers having more than one RNase H competent domain. RNase H competence also applies as a consideration to oligomers having one or more regions of inverted polarity, to circular oligomers and to other types of oligomers.

Oligomers containing as few as about 8 nucleomonomers can be used to effect inhibition of target protein(s) expression by formation of duplex or triplex structures with target nucleic acid sequences. However, linear oligomers used to inhibit target protein expression via duplex or triplex formation will preferably have from about 10 to about 20 nucleomonomer residues.

Oligomers containing substitute linkages of the invention can be conveniently circularized as described (International Publication No. WO 92/19732; Kool, E.T. J Am Chem Soc (1991) 113:6265-6266; Prakash, G., et al. J Am Chem Soc (1992) 114:3523-3527). Such oligomers are suitable for binding to single-stranded or double-stranded nucleic acid targets. Circular oligomers can be of various sizes. Such oligomers in a size range of about 22-50 nucleomonomers can be conveniently prepared. The circular oligomers can have from about three to about six nucleomonomer residues in the loop region that separate binding domains of the oligomer as described (Prakash, G. ibid). Oligomers can be enzymatically circularized through a terminal phosphate by ligase or by chemical means via linkage through the 5'- and 3'-terminal sugars and/or bases.

The oligomers can be utilized to modulate target gene expression by inhibiting the interaction of nucleic acid binding proteins responsible for modulating transcription (Maher, L. J., et al, Science (1989) 245:725-730) or translation. The oligomers are thus suitable as sequence-specific agents that compete with nucleic acid binding proteins (including ribosomes, RNA polymerases, DNA polymerases, translational initiation factors, transcription factors that either increase or decrease transcription, protein-hormone transcription factors and the like). Appropriately designed oligomers can thus be used to increase target protein synthesis through mechanisms such as binding to or near a regulatory site that transcription factors use to repress expression or by inhibiting the expression of a selected repressor protein itself.

The invention oligomers, comprising additional modifications that enhance binding affinity can be designed to contain secondary or tertiary structures, such as pseudoknots or pseudo-half-knots (Ecker, D.J., et al, Science (1992) 257:958-961). Such structures can have a more stable secondary or tertiary structure than corresponding unmodified oligomers. The enhanced stability of such structures would rely on the increased binding affinity between regions of self complementarity in a single oligomer or regions of complementarity between two or more oligomers that form a given structure. Such structures can be used to mimic structures such as the HIV TAR structure in order to interfere with binding by the HIV Tat protein (a protein that binds to TAR). A similar approach can be utilized with other transcription or translation factors that recognize higher nucleic acid structures such as stems, loops, hairpins, knots and the like. Alternatively, the invention oligomers can be used to (1) disrupt or (2) bind to such structures as a method to (1) interfere with or (2) enhance the binding of proteins to nucleic acid structures.

In addition to their use in antisense or triple helix therapies, the oligomers of the invention can also be applied as therapeutic or diagnostic agents that function by direct displacement of one strand in a duplex nucleic acid. Displacement of a strand in a natural duplex such as chromosomal DNA or duplex viral DNA, RNA or hybrid DNA/RNA is possible for oligomers with a high binding affinity for their complementary target sequences. Therapeutic applications of oligomers by this method of use, referred to herein as D-looping or "D-loop therapy" has not previously been possible because the affinity of natural DNA or RNA for its complementary sequence is not great enough to efficiently displace a DNA or RNA strand in a duplex. Therapeutic efficacy of oligomers that function by D-looping would result from high affinity binding to a complementary sequence that results in modulation of the normal biological function associated with the nucleic acid target. Types of target nucleic acids include but are not limited to (i) gene sequences including exons, introns, exon/intron junctions, promoter/enhancer regions and 5' or 3' untranslated regions, (ii) regions of nucleic acids that utilize secondary structure in order to function (e.g. the HIV TAR stem-loop element or tRNAs), (iii) nucleic acids that serve structural or other functions such as telomeres, centromeres or replication origins (virus, bacteria and the like) and (iv) any other duplex region. It is clear that oligomers can be synthesized with discrete functional domains wherein one region of an oligomer binds to a target by D-looping while an adjacent region binds a target molecule by say, forming a triple helix or binding as an aptamer to a protein.

Alternatively, a D-looping oligomer can bind to each strand in a duplex by switching the strand to which the oligomer binds (i.e. by having one region of the oligomer that binds to one strand and another region that binds to the complementary strand). The controlling elements that dictate the mode of binding (i.e. triple helix or D-loop) are the sequence of the oligomer and the inherent affinity built into the oligomer. Base recognition rules in Watson-Crick duplex binding differ from those in Hoogsteen controlled triplex binding. Because of this, the oligomer base sequence can be used to dictate the type of binding rules an oligomer will utilize. D-loop structures are formed in nature by enzyme-mediated processes (Harris, L.D. et al., J Biol Chem (1987) 262: 9285-9292) or are associated with regions where DNA replication occurs (Jacobs, H.T. et al., Nucl Acids Res (1989) 17:8949-8966). D-loops that arise from the binding of oligomers can result from a one or two step process. Direct displacement of a target strand will give rise to a D-loop by a single binding event. However, D-looping can also occur by forming a triple helix which facilitates a strand displacement event leading to the D-loop.

Ribozymes containing substitute linkages of the invention can be designed in order to design species with altered characteristics. Ribozymes that cleave single stranded RNA or DNA (Robertson, D.L., et al Nature (1990) 344:467-468) have been described. Therapeutic applications for ribozymes have been postulated (Sarver, N. et al, Science (1990) 247:1222-1225; International Publication Number WO 91/04319). Secondary or tertiary structure necessary for ribozyme function can be affected by design of appropriate oligomer sequences. For example, ribozymes having nuclease stable targeting domains containing substitute linkages of the invention can have higher affinity, while maintaining base pairing specificity, for target sequences. Because of the higher affinity and/or nuclease stability of the invention substitute linkages shorter recognition domains in a ribozyme (an advantage in manufacturing) can be designed which can lead to more favorable substrate turnover (an advantage in ribozyme function).

In therapeutic applications, the oligomers are utilized in a manner appropriate for treatment of a variety of conditions by inhibiting expression of appropriate target genes. For such therapy, the oligomers can be formulated for a variety of modes of administration, including systemic, topical or localized administration. Techniques and formulations generally can be found in Remington's Pharmaceutical Sciences, Mack Publishing Co., Easton, PA, latest edition. The oligomer active ingredient is generally combined with a carrier such as a diluent or excipient which can include fillers, extenders, binders, wetting agents, disintegrants, surface-active agents, or lubricants, depending on the nature of the mode of administration and dosage forms. Typical dosage forms include tablets, powders, liquid preparations including suspensions, emulsions and solutions, granules, capsules and suppositories, as well as liquid preparations for injections, including liposome preparations.

For systemic administration, injection is preferred, including intramuscular, intravenous, intraperitoneal, and subcutaneous. For injection, the oligomers of the invention are formulated in liquid solutions, preferably in physiologically compatible buffers such as Hank's solution or Ringer's solution. In addition, the oligomers can be formulated in solid form and redissolved or suspended immediately prior to use. Lyophilized forms are also included. Dosages that can be used for systemic administration preferably range from about 0.01 mg/Kg to 50 mg/Kg administered once or twice per day. However, different dosing schedules can be utilized depending on (i) the potency of an individual oligomer at inhibiting the activity of its target DNA or RNA, (ii) the severity or extent of a pathological disease state associated with a given target gene, or (iii) the pharmacokinetic behavior of a given oligomer.

Systemic administration can also be by transmucosal or transdermal means, or the compounds can be administered orally. For transmucosal or transdermal administration, penetrants appropriate to the barrier to be permeated are used in the formulation. Such penetrants are generally known in the art, and include, for example, bile salts and fusidic acid derivatives for transmucosal administration. In addition, detergents can be used to facilitate permeation. Transmucosal administration can be through use of nasal sprays, for example, or suppositories. For oral administration, the oligomers are formulated into conventional oral administration forms such as capsules, tablets, and tonics.

For topical administration, the oligomers of the invention are formulated into ointments, salves, gels, or creams, as is generally known in the art. Formulation of the invention oligomers for ocular indications such as viral infections would be based on standard compositions known in the art.

In addition to use in therapy, the oligomers of the invention can be used as diagnostic reagents to detect the presence or absence of the target nucleic acid sequences to which they specifically bind. The enhanced binding affinity of the invention oligomers is an advantage for their use as primers and probes.
Diagnostic tests cab be conducted by hybridization through either double or triple helix formation which is then detected by conventional means. For example, the oligomers can be labeled using radioactive, fluorescent, or chromogenic labels and the presence of label bound to solid support detected. Alternatively, the presence of a double or triple helix can be detected by antibodies which specifically recognize these forms. Means for conducting assays using such oligomers as probes are generally known.

The use of oligomers containing the invention substitute linkages as diagnostic agents by triple helix formation is advantageous since triple helices form under mild conditions and the assays can thus be carried out without subjecting test specimens to harsh conditions. Diagnostic assays based on detection of RNA for identification of bacteria, fungi or protozoa sequences often require isolation of RNA from samples or organisms grown in the laboratory, which is laborious and time consuming, as RNA is extremely sensitive to ubiquitous nucleases.

The oligomer probes can also incorporate additional modifications such as modified sugars and/or substitute linkages that render the oligomer especially nuclease stable, and would thus be useful for assays conducted in the presence of cell or tissue extracts which normally contain nuclease activity. Oligomers containing terminal modifications often retain their capacity to bind to complementary sequences without loss of specificity (Uhlmann et al., Chemical Reviews (1990) 90:543-584). As set forth above, the invention probes can also contain linkers that permit specific binding to alternate DNA strands by incorporating a linker that permits such binding (Froehler, B.C., et al, Biochemistry (1992) 31:1603-1609); Horne et al., J Am Chem Soc (1990) 112:2435-2437).

Incorporation of substitute linkages of the present invention into probes that also contain covalent crosslinking agents has the potential to increase sensitivity and reduce background in diagnostic or detection assays. In addition, the use of crosslinking agents will permit novel assay modifications such as (1) the use of the crosslink to increase probe discrimination, (2) incorporation of a denaturing wash step to reduce background and (3) carrying out hybridization and crosslinking at or near the melting temperature of the hybrid to reduce secondary structure in the target and to increase probe specificity. Modifications of hybridization conditions have been previously described (Gamper et al., Nucleic Acids Res (1986) 14:9943).

Oligomers of the invention are suitable for use in diagnostic assays that employ methods wherein either the oligomer or nucleic acid to be detected are covalently attached to a solid support as described (U.S. Patent No. 4,775,619). The oligomers are also suitable for use in diagnostic assays that rely on polymerase chain reaction techniques to amplify target sequences according to described methods (European Patent Publication No. 0 393 744). Oligomers of the invention containing a 3' terminus that can serve as a primer are compatible with polymerases used in polymerase chain reaction methods such as the Taq or Vent™ (New England Biolabs) polymerase. Oligomers of the invention can thus be utilized as primers in PCR protocols.

The oligomers are useful as primers that are discrete sequences or as primers with a random sequence. Random sequence primers can be generally about 6, 7, or 8 nucieomonomers in length. Such primers can be used in various nucleic acid amplification protocols (PCR, ligase chain reaction, etc) or in cloning protocols. The substitute linkages of the invention generally do not interfere with the capacity of the oligomer to function as a primer. Oligomers of the invention having 2'-modifications at sites other than the 3' terminal residue, other modifications that render the oligomer RNase H incompetent or otherwise nuclease stable can be advantageously used as probes or primers for RNA or DNA sequences in cellular extracts or other solutions that contain nucleases. Thus, the oligomers can be used in protocols for amplifying nucleic acid in a sample by mixing the oligomer with a sample containing target nucleic acid, followed by hybridization of the oligomer with the target nucleic acid and amplifying the target nucleic acid by PCR, LCR or other suitable methods.

The oligomers derivatized to chelating agents such as EDTA, DTPA or analogs of 1,2-diaminocyclohexane acetic acid can be utilized in various in vitro diagnostic assays as described (U.S. Patent Nos. 4,772,548, 4,707,440 and 4,707,352). Alternatively, oligomers of the invention can be derivatized with crosslinking agents such as 5-(3-iodoacetamidoprop-1-yl)-2'-deoxyuridine or 5-(3-(4-bromobutyramido)prop-1-yl)-2'-deoxyuridine and used in various assay methods or kits as described (International Publication No. WO 90/14353).

In addition to the foregoing uses, the ability of the oligomers to inhibit gene expression can be verified in in vitro systems by measuring the levels of expression in subject cells or in recombinant systems, by any suitable method (Graessmann, M., et al., Nucleic Acids Res (1991) 19:53-59).

All references cited herein are incorporated herein by reference in their entirety.

### Synthesis of the Oligomers

The oligomers of the invention can be synthesized using reactions known in the art of oligonucleotide derivative synthesis. See e.g. Flandor, J. and Yam, S.Y., Tet Letts (1990) 31:597-600; Mattson, R.J. et al., J Org Chem (1990) 55:2552-2554; Chung, C.K et al., J Org Chem (1989) 54:2767-2769.

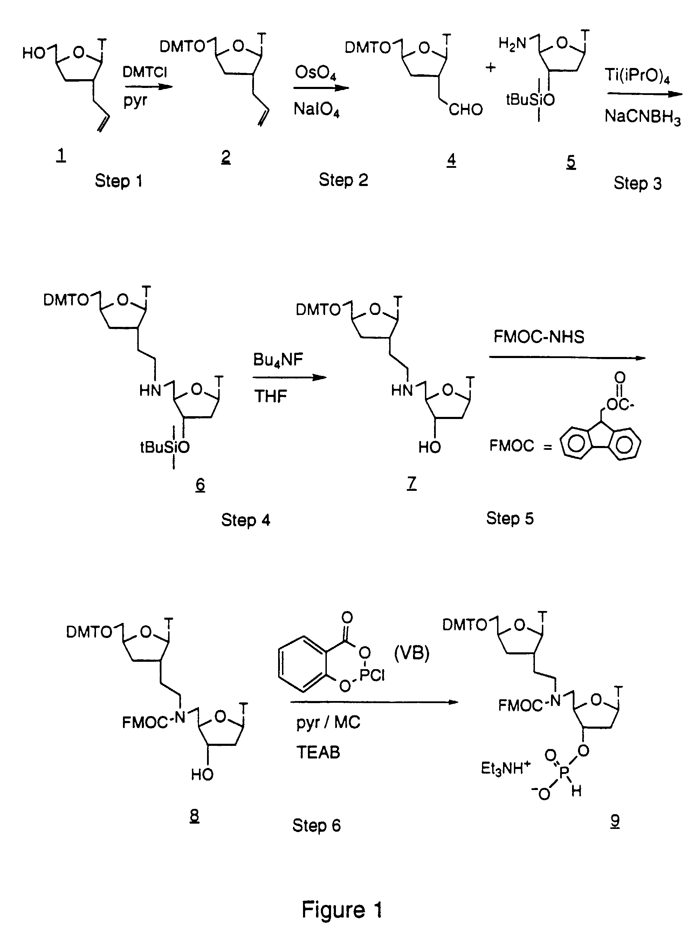

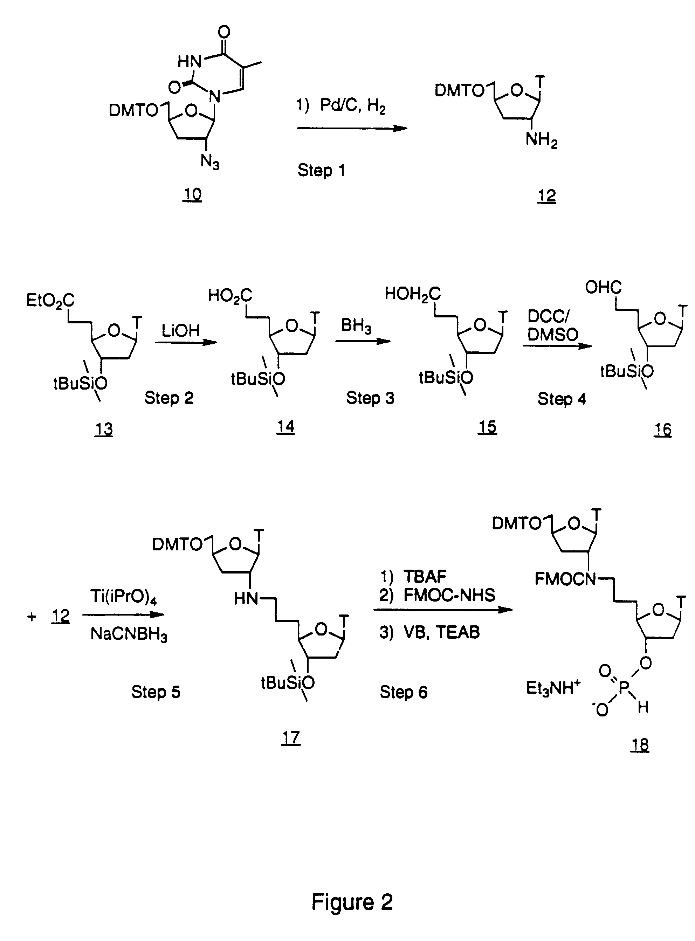

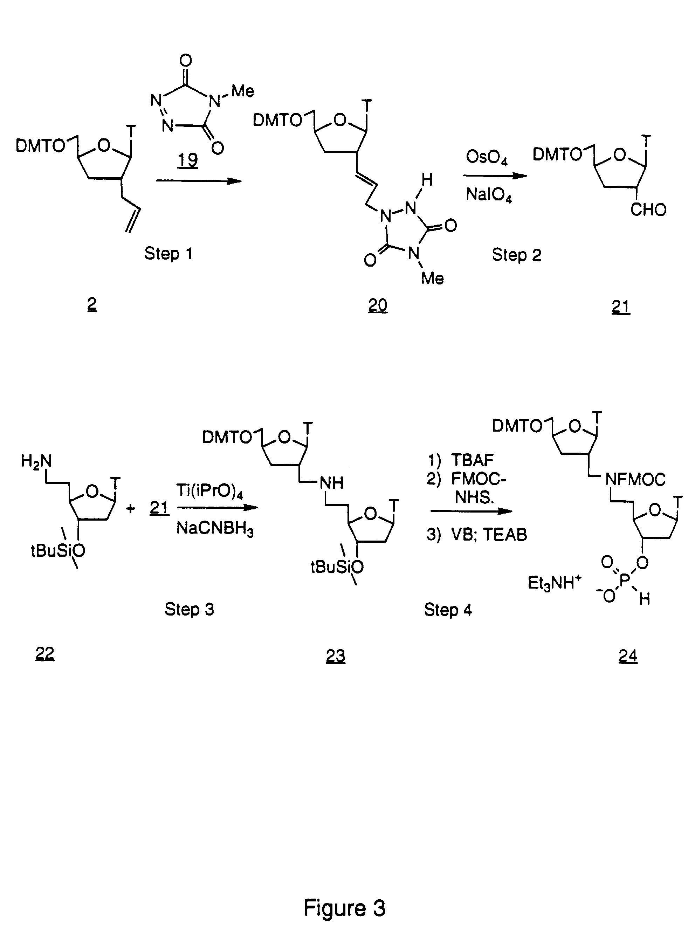

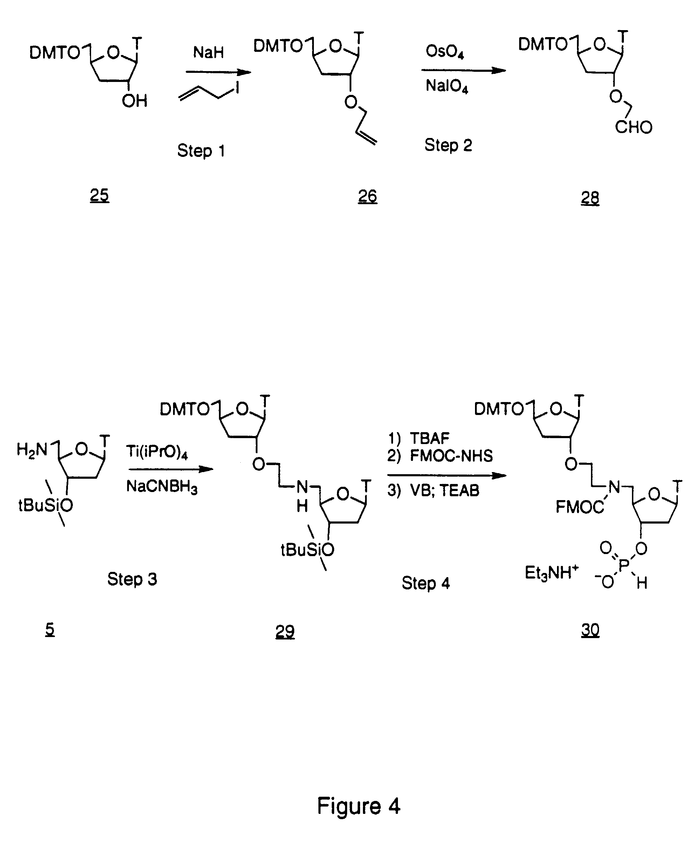

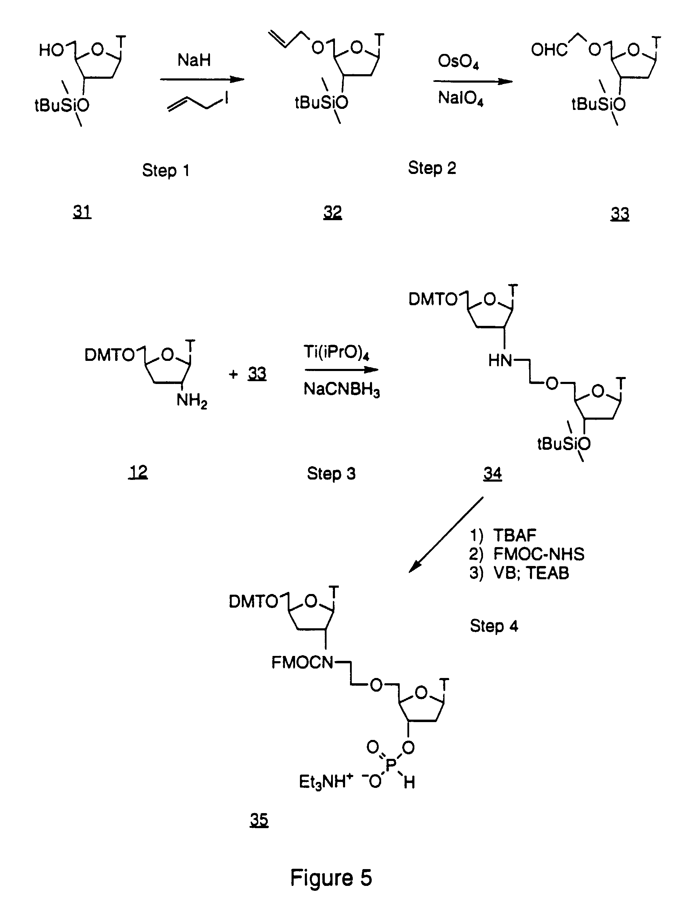

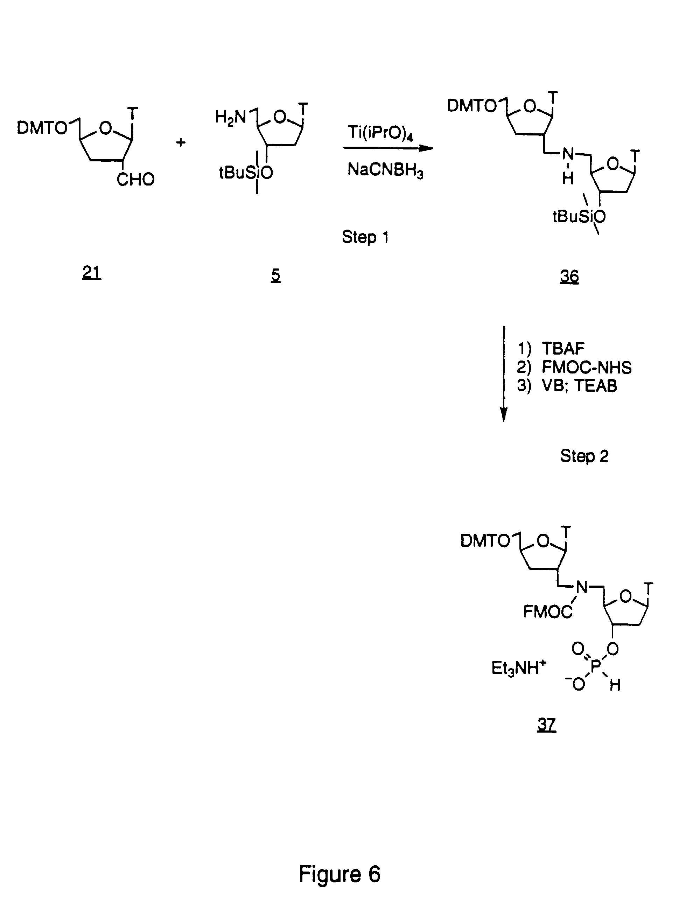

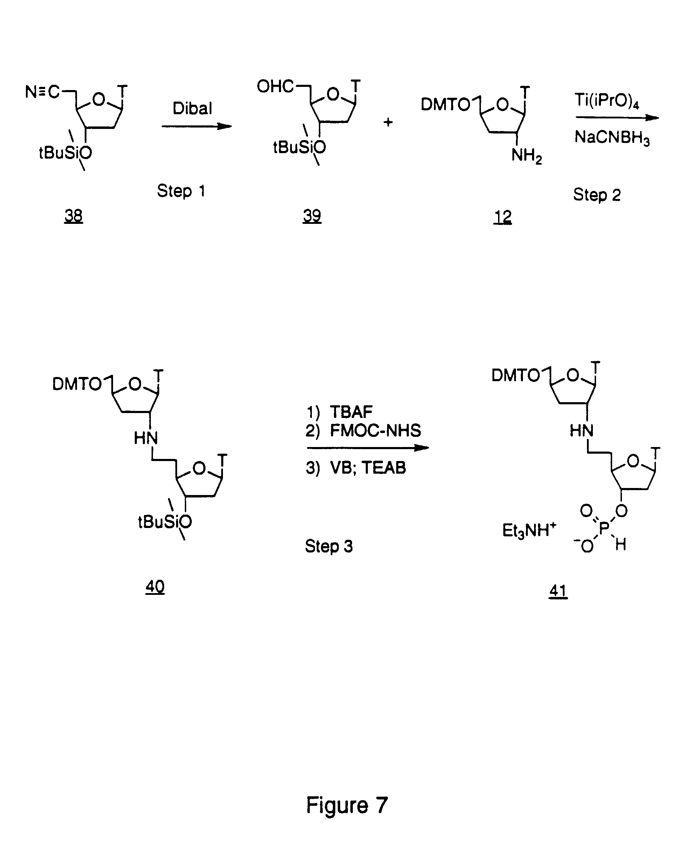

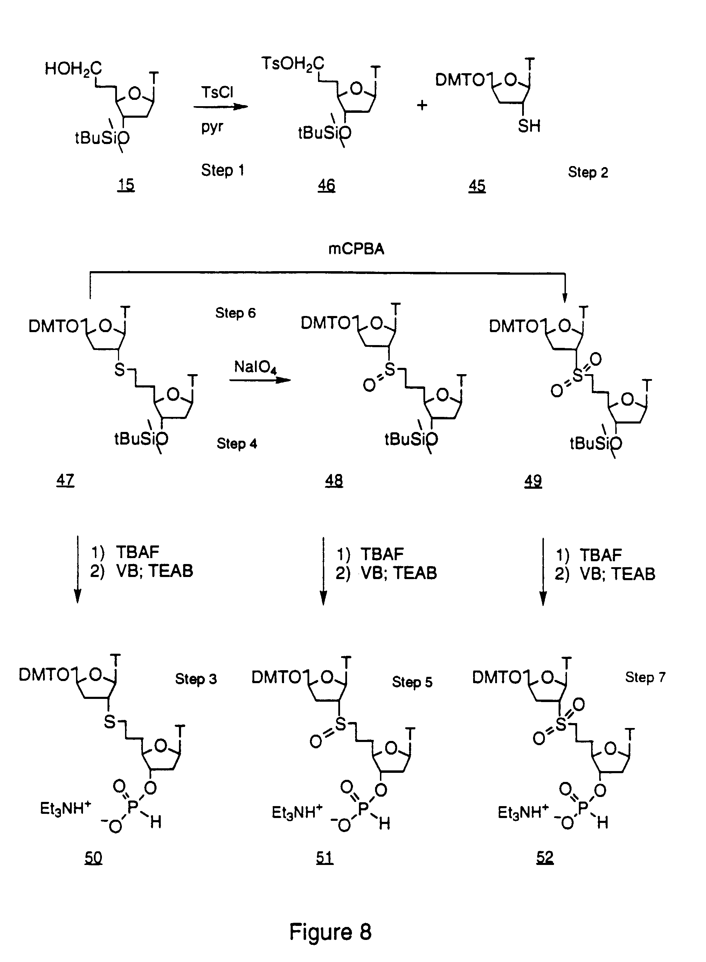

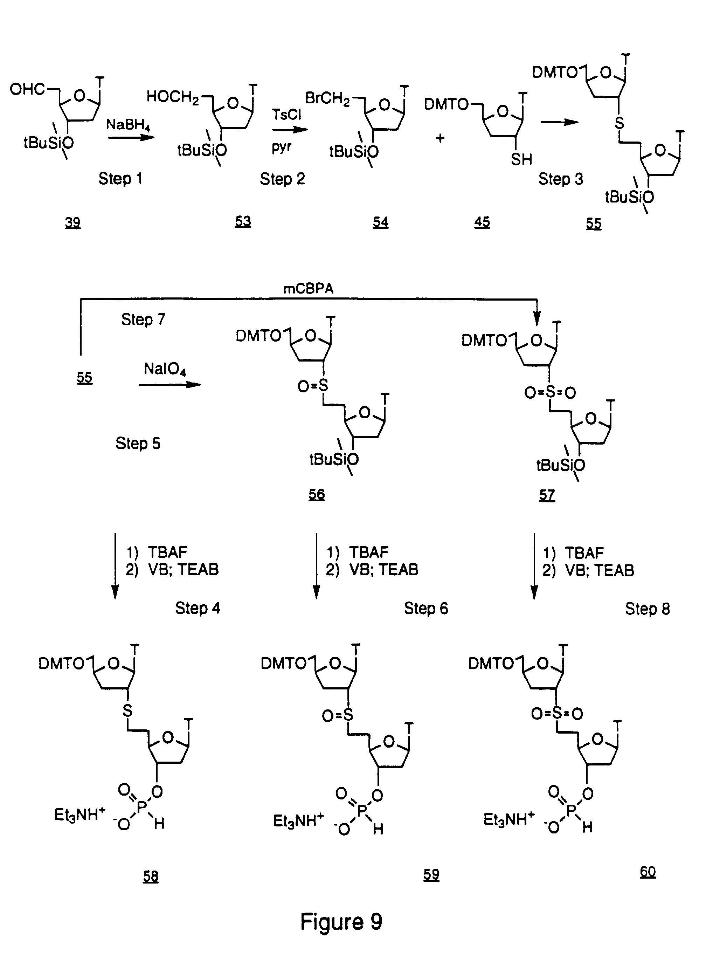

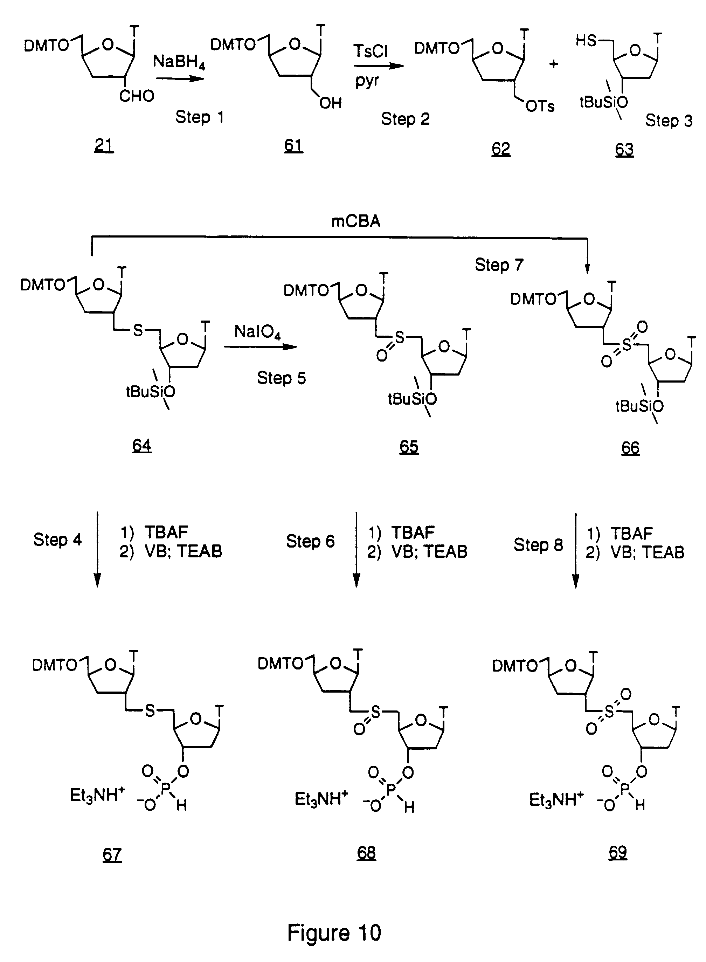

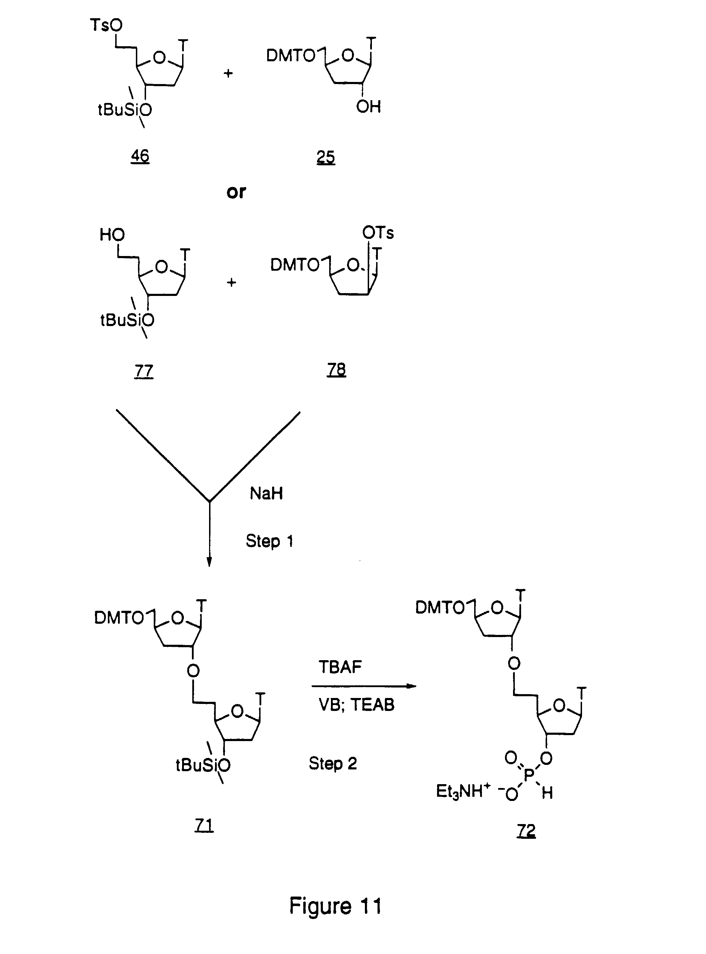

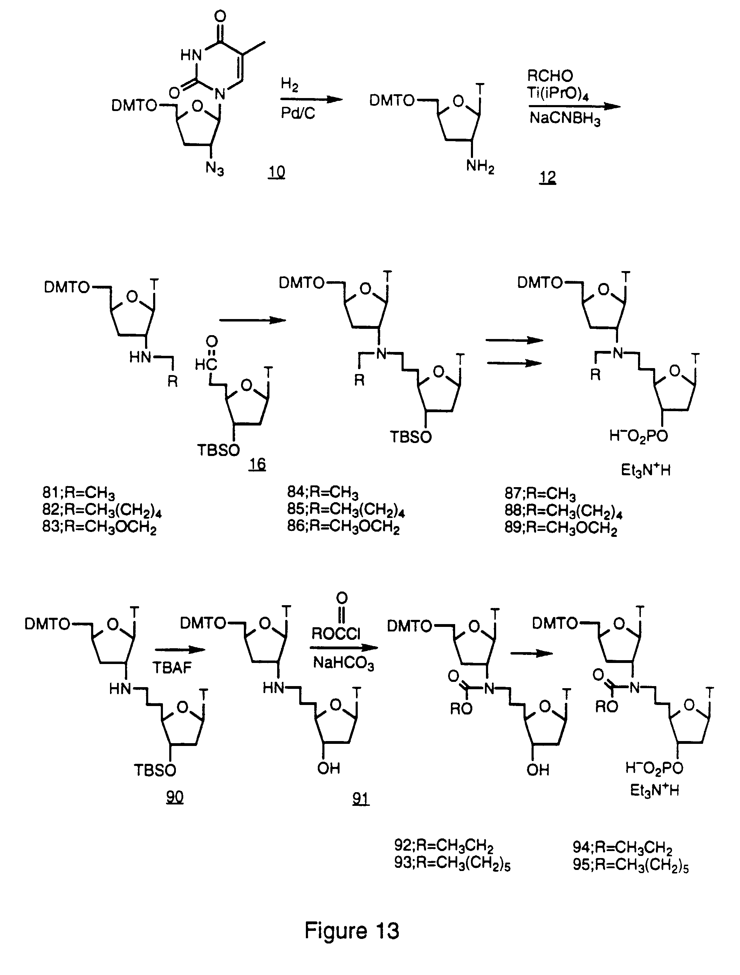

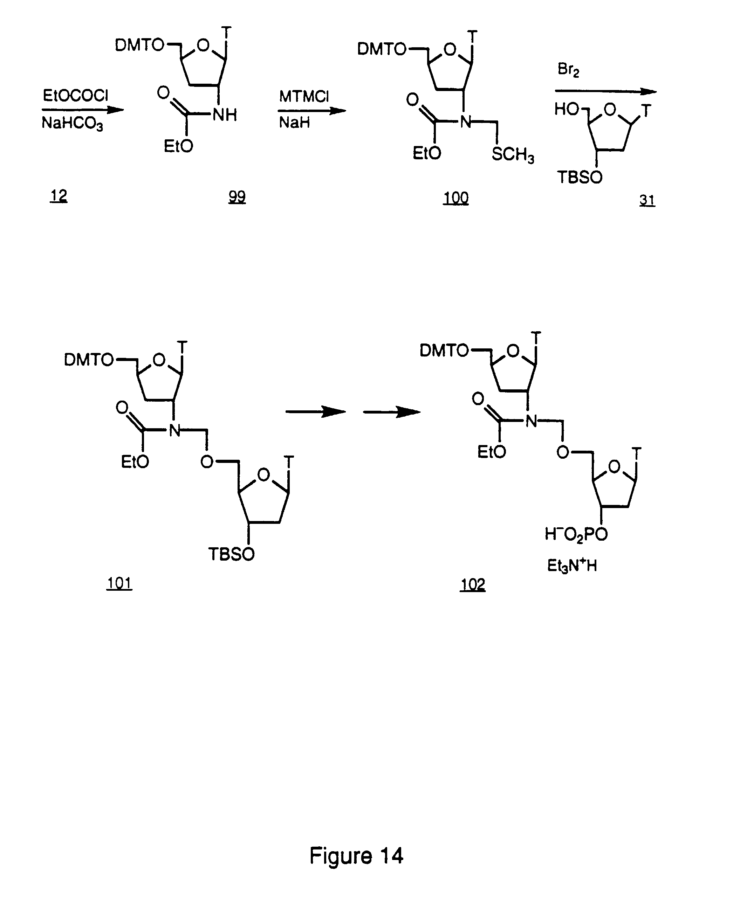

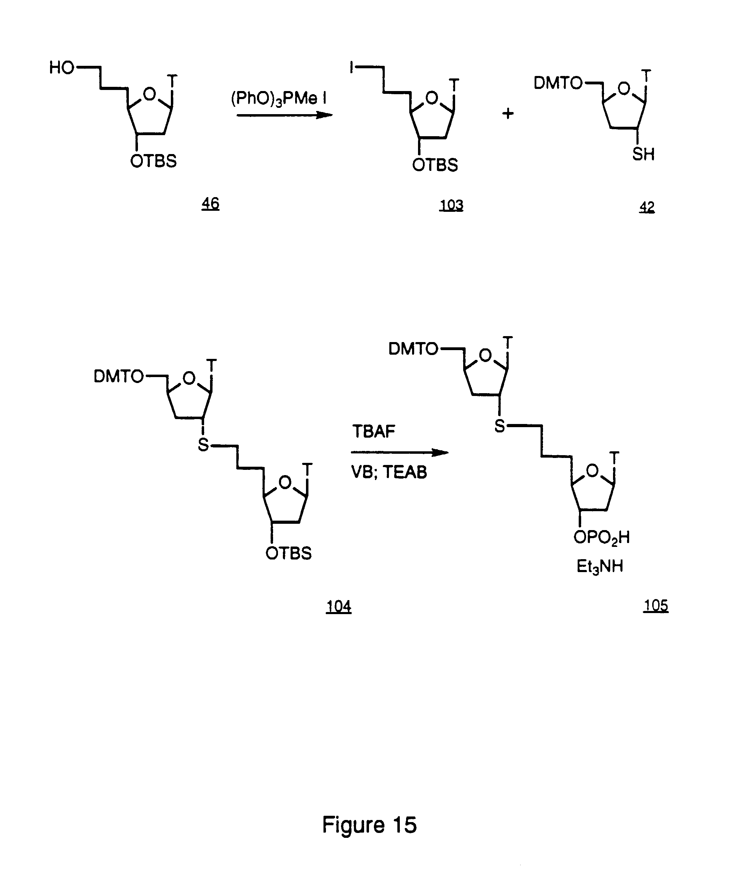

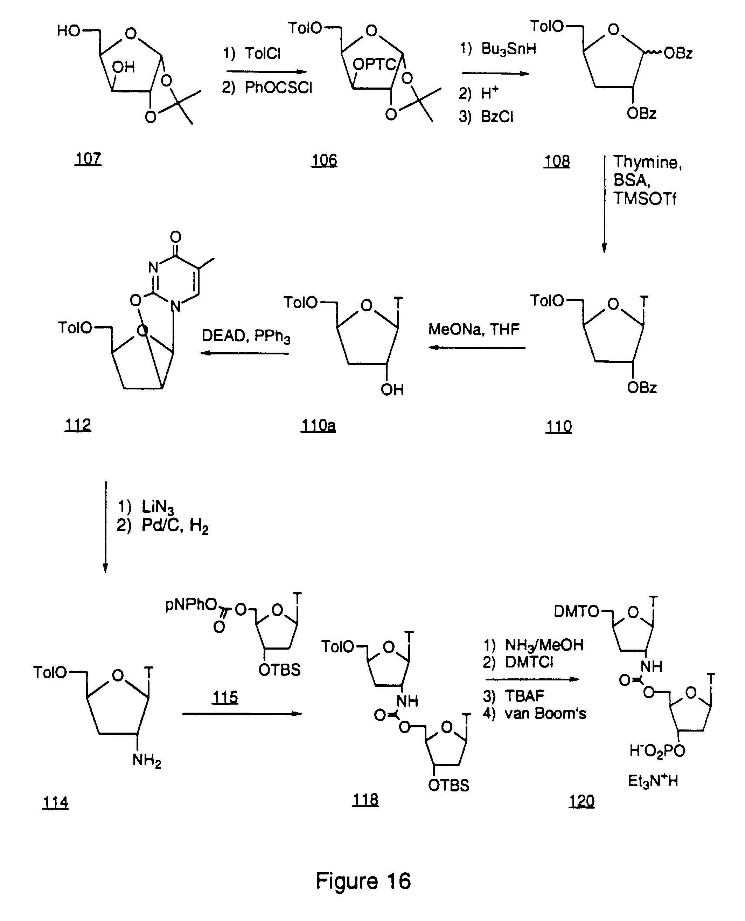

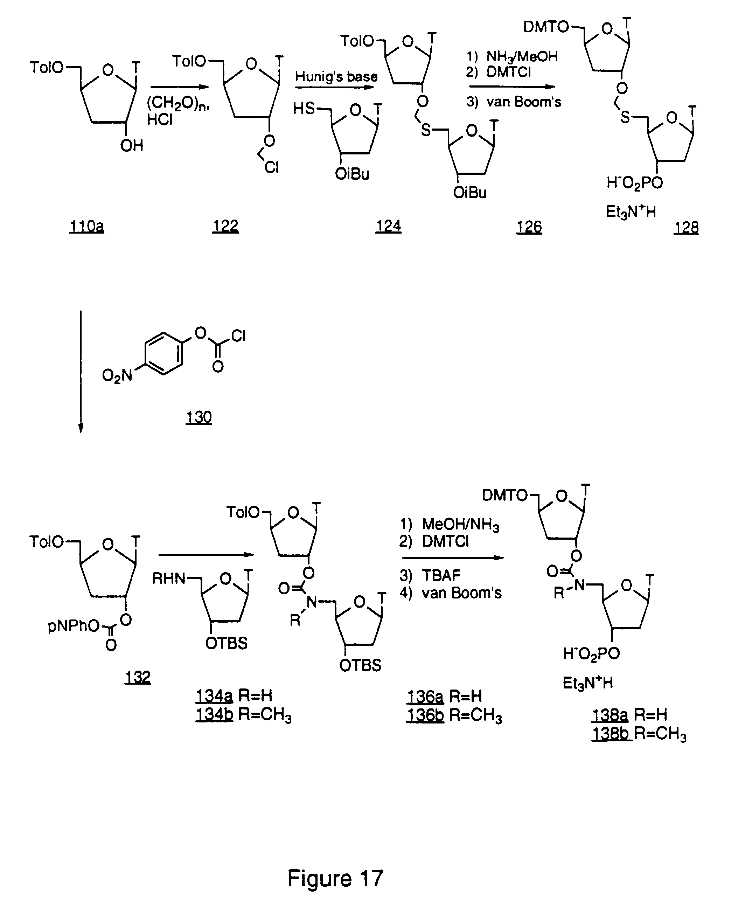

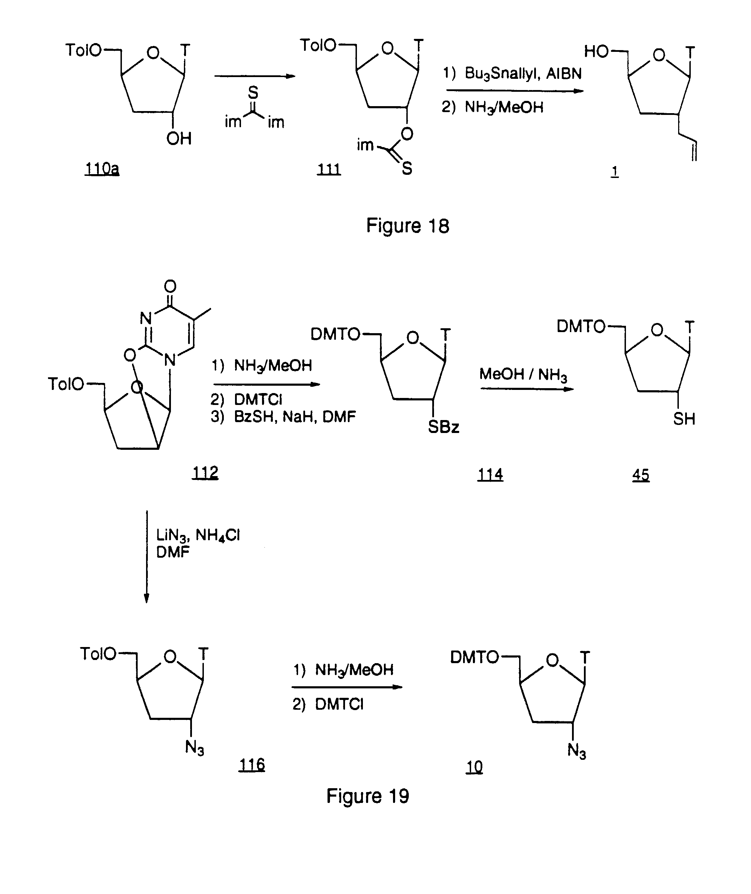

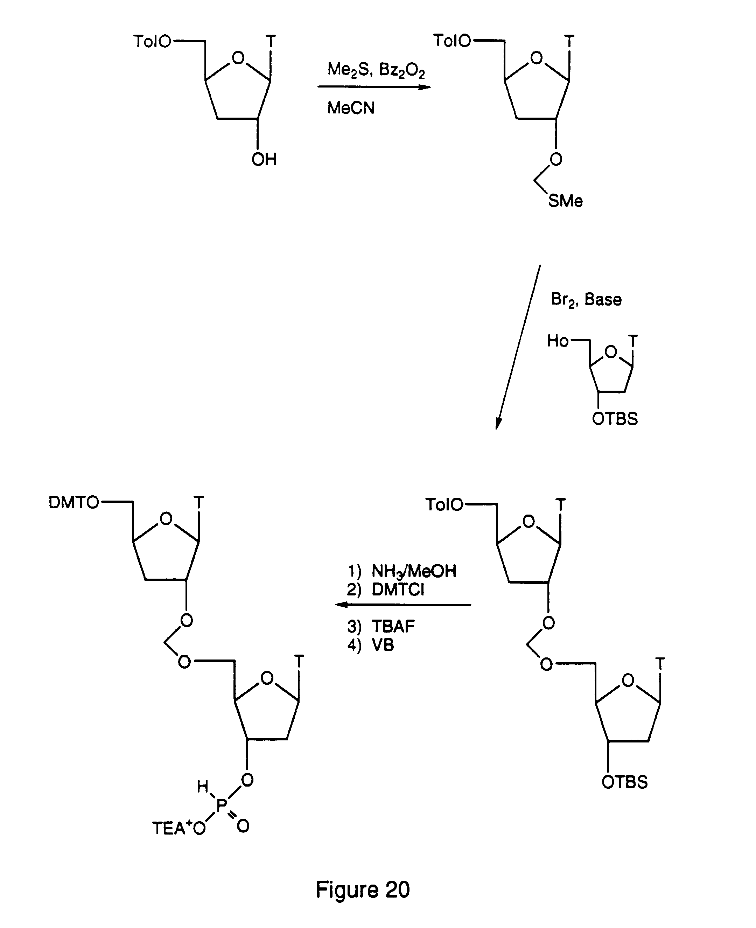

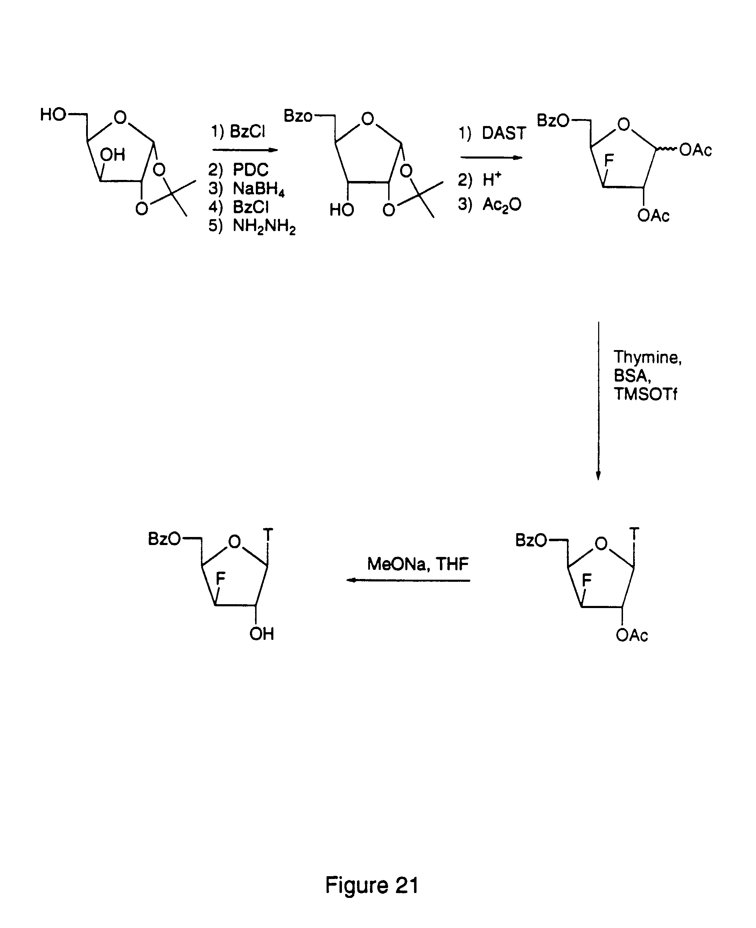

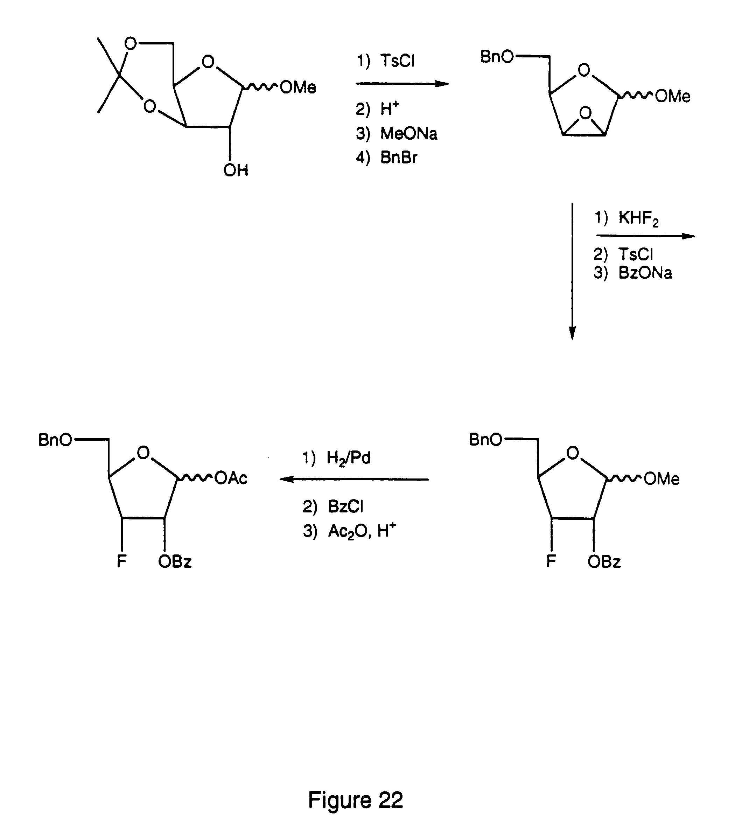

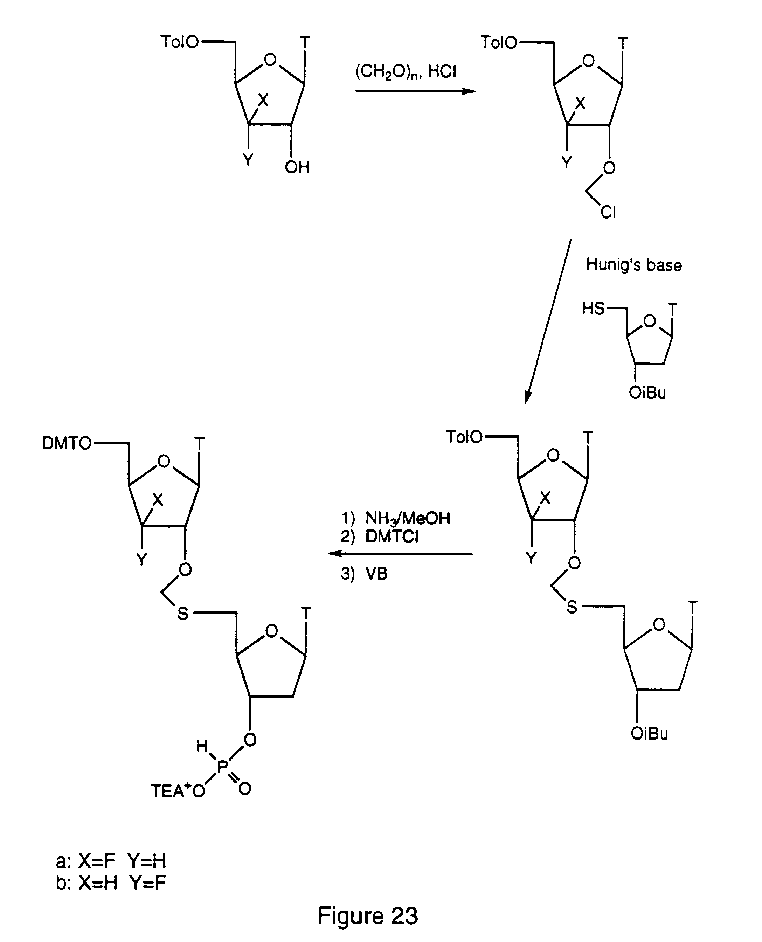

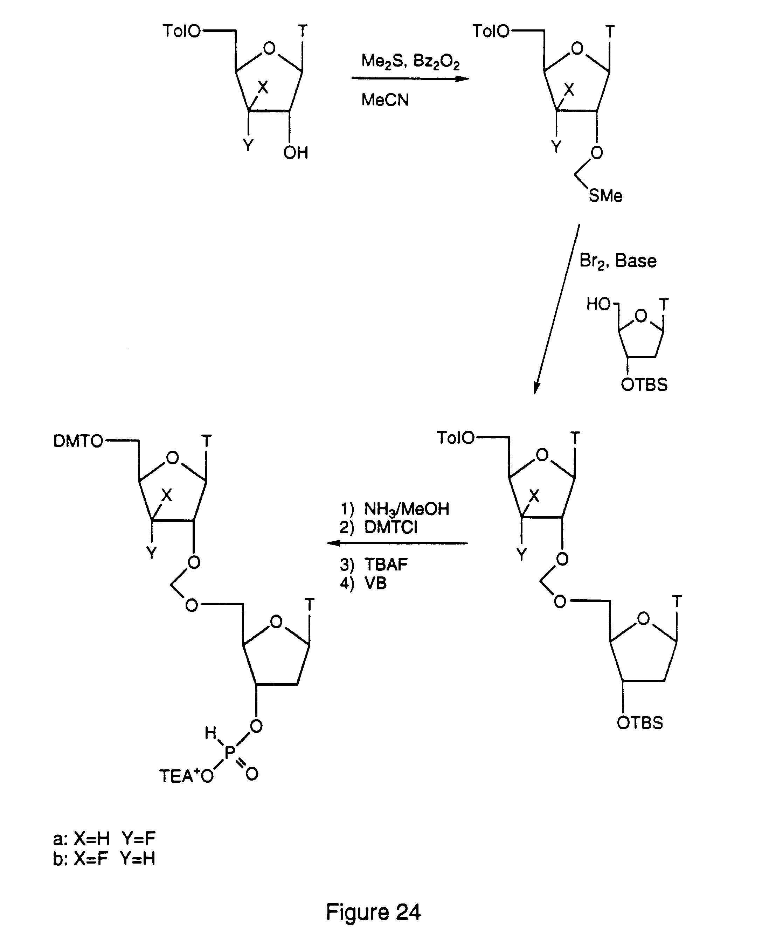

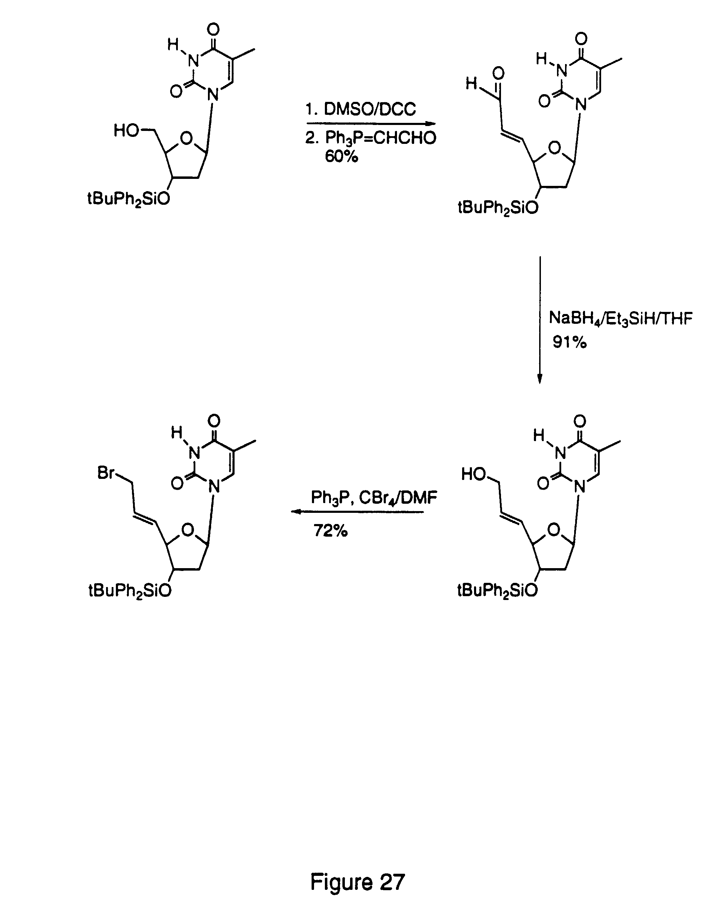

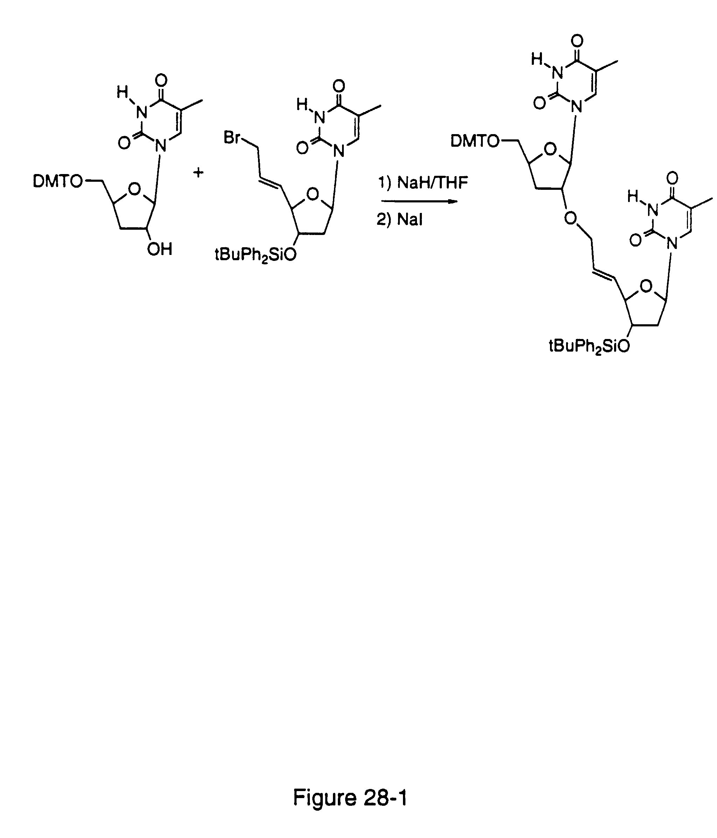

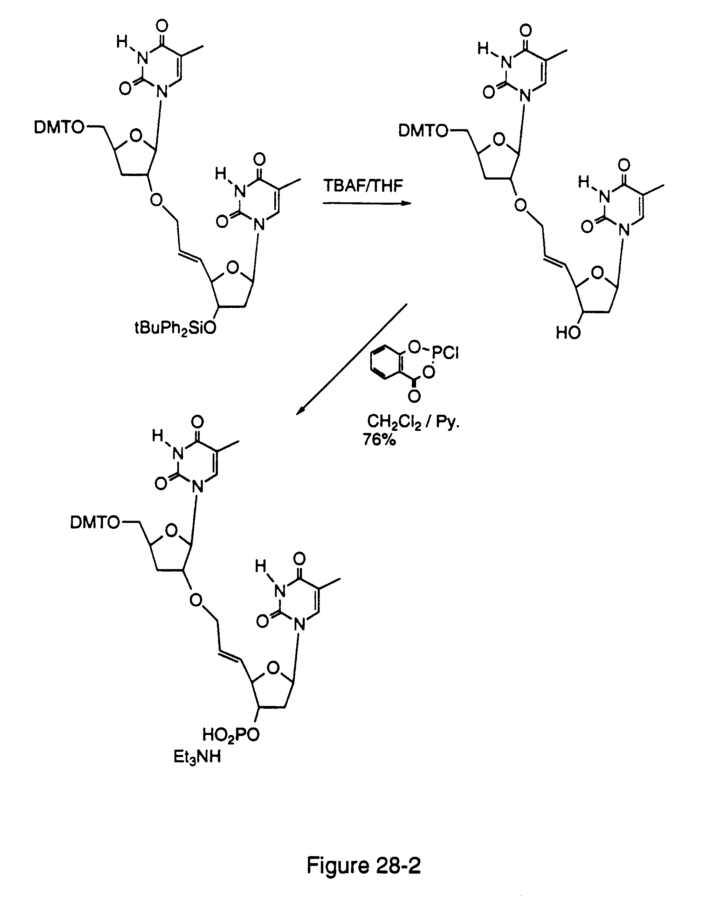

As can be seen from the variety of substitute linkages specifically listed in Table 1, the substitute linkages of the invention can vary so as to contain one or more nitrogen, sulfur, and/or oxygen atoms in their structure. The positions of these atoms in the substitute linkage can vary from the "5'" end, to the "middle" to the "2'" end. In this section, a series of representative synthesis reaction schemes are set forth which provide routes to various locations and combinations of nitrogen, oxygen, and sulfur atoms within the substitute linkages. Specifically, Scheme 1 shown in Figure 1, shows the formation of a dimer containing a three atom long substitute linkage with a nitrogen at the 5' end of the 2' nucleomonomer. Scheme 2, depicted in Figure 2, shows the formation of a three atom long substitute linkage with a nitrogen at the 2' end of the 5' nucleomonomer. Scheme 3, shown in Figure 3, depicts the formation of a three atom long substitute linkage with a nitrogen in the middle. Scheme 4, shown in Figure 4, depicts the formation of a four atom long substitute linkage with oxygen at the 2' end and nitrogen at the 5' end. Scheme 5, depicted in Figure 5, shows the formation of a four atom long substitute linkage with nitrogen at the 2' end and oxygen at the 5' end. Scheme 6, shown in Figure 6, depicts the formation of a two atom long substitute linkage with nitrogen at the 5' end. Scheme 7, depicted in Figure 7, shows the formation of a two atom long substitute linkage with nitrogen at the 2' end. Scheme 8, represented in Figure 8, shows the formation of three different three atom long substitute linkages with sulfur at the 2' end. Scheme 9, represented in Figure 9, depicts the formation of three different two atom long substitute linkages with sulfur at the 2' end. Scheme 10, depicted in Figure 10, shows the formation of three different two atom long substitute linkages with sulfur at the 5' end. Scheme 11, shown in Figure 11, depicts the formation of a two atom long substitute linkage with oxygen at the 2' end. Scheme 12 as shown in Figure 12 depicts the formation of a three atom long substitute linkage with oxygen at the 5' end. Scheme 13, depicted in Figure 13, shows the formation of several alkyl derivatives of a three atom long substitute linkage with nitrogen at the 2' end. Scheme 14, shown in Figure 14, shows the formation of a three atom long aminal derivative. Scheme 15, depicted in Figure 15, demonstrates the preparation of a three atom long substitute linkage with sulfur at the 2' end. Scheme 16, shown in Figure 16, shows the preparation of a three atom long substitute linkage with a nitrogen at the 2' end of the 5' nucleoside. Scheme 17, shown in Figure 17, shows the preparation of two three atom long substitute linkages having oxygen atoms at the 2' end of the substitute linkage. These substitute linkages have either an oxygen or a sulfur at the 5' end and a carbon or carbonyl midlinkage. Figures 18 and 19 show the formation of several starting nucleomonomers for several linkages of the invention. Figure 20 shows a scheme for synthesis of a 2',5' formacetal substitute linkage. Figures 21 and 22 show the synthesis of a xylosofluoro (Puech, F. et al Tet Lett (1989) 30:3171-3174) and ribofluoro (Mikhailopulo, I., et al, J Med Chem (1991) 34:2195-2202) nucleomonomer and Figures 23 and 24 show the incorporation of these nucleomonomers into dimers linked via 5',2' thioformacetal and formacetal substitute linkages. Figure 25 shows the synthesis of a 2', 5' thioformacetal substitute linkage. Figure 26 shows the synthesis of a nucleomonomer used for formation of a linkage containing a double bond and Figures 27 and 28 show synthesis of dimers containing a double bond.

These schemes can be modified as is known to those practicing in the area of oligonucleotide chemistry. For example, although protection of the bases is not always indicated in the synthesis schemes, such may be desireable and can be accomplished using reagents and techniques known in the art. See, e.g. Protective Groups in Organic Synthesis (Theodora W. Greene, John Wiley and Sons, 1981). Similarly, although the use of protective groups is shown in some cases, it is not always necessary to block the reactants in order to synthesize the exemplified invention oligomers.

Turning to Figure 1, the first two steps shown in Scheme 1 relate to the derivatization of thymine to a protected nucleoside. The third and subsequent steps in Scheme 1 are directed to the synthesis of the substitute linkage. The starting materials such as the material shown as compound 1 in Scheme 1 are 2'-deoxy-2'-(2-allyl) nucleosides. These allyl materials are analogous to the 3'-deoxy-3'-(2-propanyl) thymidyl derivatives described in Flandor, J. and Yam, S.Y., supra.

In step 1 of Scheme 1, the reactive 5'-hydroxyl in the nucleoside sugar is reacted with dimethoxytritylchloride (DMTCl) to protect it and yields compound **2**. Other equivalent protecting groups may be used. In the next step, the 2'-allyl group of Compound 2 is oxidized with OsO₄/NaIO₄ to yield the aldehyde intermediate **4**. The aldehyde **4** is then reacted with a 5-deoxy-5'-amino-3'-protected nucleoside, which can be selected from a range of known compounds and the resulting imine is reduced. This reductive alkylation reaction can be advantageously carried out using a suitable catalyst such as titanium isopropoxide and cyanoborohydride (see Mattson, R.J. et al., supra). This yields a protected dimer joined through a 2'-CH₂-CH₂-NH-5' substitute linkage. Compound **6** in Scheme 1 is representative.

Thereafter, the 3'-hydroxyl protecting group is removed to yield compound **7**. The amine group in the substitute linkage is protected, such as with an FMOC group to yield compound **8** and a phosphonate group is added to the 3'-hydroxyl with Van Boom's reagent (VB) (Marugg, J.E. et al., Tet Letters (1986) 27:2661-2664). This yields dimer **9** which has two nucleosides joined through a -CH₂-CH₂-N(FMOC)- substitute linkage, a free 3'-phosphonate group and a blocked 5' position. This dimer can then be added into a growing oligomer using conventional chemistry. Alternatively, the resulting dimer or oligomer may be succinylated as a convenient linker for coupling to a solid support, such as controlled pore glass (CPG). The coupled oligomer can be used as a starting material for standard oligonucleotide synthesis, as, for example, using H-phosphonate chemistry as described by Froehler, B., et al., Nucleic Acids Res (1986) 14:5399.

This synthesis involves deprotection of the 5'-hydroxyl using dichloroacetic acid in methylene chloride and treatment with a 5'-DMT-protected base 3'-phosphonate in the presence of acetyl chloride/pyrimidine/acetonitrile, and repetition of this deprotection and linkage protocol for any desired number of times.

Alternatively, the liberated 3'-OH can be linked via an ester linkage to a solid support analogous to standard oligonucleotide synthesis (Matteucci, M. et al., J Am Chem Soc (1981) 103:3185) for extension of oligonucleotide. The final product is removed from the solid support by standard procedures, such as treatment with iodine in a basic aqueous medium containing THF or other inert solvent, followed by treatment with ammonium hydroxide. Deprotection of the bases attached to the added nucleotides is also conducted by standard procedures. Similarly, the FMOC group protecting the nitrogen present in the substitute linkage can be removed conventionally and, if desired, replaced by other R groups as set forth herein.

The substitute linkage can be included at any arbitrary position in an oligomer by substituting for a conventional monomer in the sequential synthesis, a protected dimer containing the substitute linkage which has been synthesized, for example, by the steps set forth in Scheme 1 shown in Figure 1.

Any DNA synthesis chemistry such as phosphoramidate or phosphonate chemistry can be used to link monomers or dimers in a manner analogous to that set forth above.

Turning to Figure 2, a representative route (Scheme 2) is provided for generating a three atom long substitute linkage with a nitrogen at the 2' position is shown. In Step 1 the N₃ group is reduced to an amine such as with hydrogen and a hydrogenitive catalyst to yield compound **12**. Step 2 begins with an ester compound **13**. This material is treated in Step 2 with base to hydrolyze the ester, and treated with acid to yield the free acid **14**. The acid is then selectively reduced to the alcohol **15** using for example a borane reducing agent. The alcohol **15** is converted in Step 4 to the aldehyde **16** such as by using a carbodiimide and DMSO. Aldehyde **16** and amine **12** are then coupled in Step 5 and converted to phosphonate **18** in a manner analogous to that used in Scheme 1 by treatment with TBAF (Tetrabutyl ammonium fluoride), FMOC-NHS and Van Boom's reagent plus TEAB.

In Reaction Scheme 3 (shown in Figure 3) the starting material is a 2'-alkyl substituted protected nucleoside such as **2**. In Step 1 the alkyl double bond is isomerized by coupling the alkyl group to **19**. Step 2 can be used to generate a 2'-aldehyde substituent present in compound **21**. This aldehyde can then be coupled to the known amine **22** in Step 3 and converted to the phosphonate in Step 4 which are analogous to the steps described in Schemes 1 and 2.

In Figure 4 a route for producing an oxygen- and nitrogen-containing substitute linkage is given. A free 2' hydroxyl is reacted in Step 1 with allyl iodide in the presence of sodium hydride to couple the allyl group to the free hydroxyl and yield compound **26**. The allyl group in **26** is then oxidized to an aldehyde **28** which is reacted with amine-substituted nucleoside derivative **5** in Step 3 to give the two nucleosides coupled through a substitute linkage of the invention and yield "dimer **29**" which is converted to the phosphonate form **30** using the methodology set out in Scheme 1.

Scheme 5, shown in Figure 5, is essentially the "reverse" of Scheme 4 in that the nitrogen is placed in the 2' position and the oxygen in the 5' position. Essentially the same reactions are conducted using different blocking and substitution patterns to achieve the reverse orientation.

Scheme 6, shown in Figure 6, provides a two atom long substitute linkage. It employs as representative nucleoside analog starting materials, aldehyde **21** (produced in Scheme 3) and amine **5** (noted as available in Scheme 1). These materials are coupled and converted to a phosphonate in Steps 1 and 2 which are analogous to Steps 5 and 6 of Scheme 2.

Scheme 7 shown in Figure 7 also involves a 2 atom substitute linkage, this time with a nitrogen at the "5'" end. This reaction sequence starts with the known 5' nitrile 38 (Meyer, W., et al Angew. Chem (1976) 88:512-513; Etzold, G., et al Chem Commun (1968) 7:422) which is converted to an aldehyde **39** in Step 1. This aldehyde then is coupled to amine **12** (previously prepared) in Step 2 and converted to a phosphonate in Step 3, again analogous to Steps 5 and 6 of Scheme 2.

Scheme 8, shown in Figure 8, provides a route to three atom long substitute linkages having sulfur in various oxidation states at the 2' end. The scheme begins with the thiol 45 which was synthesized as shown in Figure 19. In Step 1 the alcohol group on compound 15 (produced in Scheme 2) is reacted with tosyl chloride. Tosylate **46** is then coupled with thiol **45** in Step 2 to yield sulfur-containing "dimer" **47**. Dimer **47**, having sulfur as -S- can be converted directly to a phosphonate as shown in Step 3. Alternatively the sulfur can be partially oxidized with NaIO₄ (Step 4) to -S(O)- or with an MCPBA (Step 6) to -S(O)(O)- and then converted to the respective phosphonates as shown in Steps 5 and 7.

In Scheme 9 several two atom long sulfur-containing substitute linkages are constructed. Aldehyde **39**, prepared in Scheme 7 is reduced to alcohol **53** with a borohydride reducing agent. The alcohol is converted to a tosylate **54** which is then coupled to the thiol **45** from Scheme 8 in Step 3 to yield "dimer" **55**. Dimer **55** is then converted to the phosphonate with or without oxidation in Steps 4, 5-6 and 7-8 respectively.

Figure 10 shows Scheme 10 which is directly analogous to Schemes 8 and 9 just described with variation in the position of the aldehyde group and thiol group. Again, this scheme gives rise to 3 families of materials **67**, **68** and **69** which differ from one another in terms of sulfur oxidation state.

Schemes 11 and 12 are representative routes to nucleomonomers linked with oxygen present at the 2' and 5' ends of the linking group.

In Scheme 11, two routes are shown. In one a "5"' tosylate **46** is reacted with a "2"' alcohol **25** to yield dimer **71** which is converted to a phosphonate to yield **72**. Alternatively a 2' tosylate **78** can be reacted with a 5' alcohol **77** to yield **71**.

In Scheme 12, 2' aldehyde **4** is reduced to 2' alcohol **73** which is coupled to 5' mesylate **74** to give oxygen-containing linked material **75** which is converted to phosphonate **76**.

Figure 13, Scheme 13, shows the synthesis of alkyl derivatives of a 2' amine of a three atom long substitute linkage. Azide **10** is hydrogenated to deliver the amine **12**. Amines **81**, **82** and **83** are treated with acetaldehyde toluene, and titanium isopropoxide and the products coupled with aldehyde **16**, as described for amine **12**, to yield dimers **84-86** which are in turn converted to the corresponding phosphonates **87-89**. Acylated derivatives of the 2' amine begin with dimer **90**, which is prepared as explained for compound **17**. The products are ultimately converted to phosphonates as described further below.

The synthesis of an aminal-containing substitute linkage (Figure 14, Scheme 14) begins with amine **12**, which is acylated to yield carbamate **99**, which is alkylated to produce thioaminal **100** and is ultimately converted to the corresponding phosphonate.

Figure 15, Scheme 15, shows the preparation of a three atom long substitute linkage with a 2' sulfur. Alcohol **46** (in DMF and pyridine) is reacted within methyltriphenoxyphosphonium iodide. The product is saturated with sodium thiosulfate to yield iodide **103**. Thiol **42** and acetonitrile are combined with acetamide and DMF, and iodide added, to ultimately yield dimer **104** which is converted to a phosphonate **105** as described for compound **18**.

The following examples are intended to illustrate but not to limit the invention.

### Experimental

### Example 1

The compounds of this example are shown in Scheme 1, shown in Figure 1.

To a flask containing compound **1** (which may be produced using Scheme 18 as shown in Figure 18) is added pyridine and the solution is evaporated to dryness. Pyridine is added again followed by DMT-Cl; the solution is stirred for 18 hours and poured in 10% aq sodium bicarbonate solution. The crude product is extracted with CHCl₃, dried (Na₂SO₄), stripped to dryness, and chromatographed on silica gel (5% MeOH/MC) (methylene chloride) to yield the product **2**.

To a solution of **2** in dioxane and 1% aqueous sodium bicarbonate is added osmium tetroxide (2.5 wt% solution in t-butyl alcohol), and the solution stirred for 5 minutes. Sodium periodate is added in four portions, and the mixture stirred. The solution is poured into 10% aqueous saturated bicarbonate and the crude product is extracted with chloroform; dried (Na₂SO₄); and concentrated. The resulting oil is taken up in methylene chloride; filtered through celite and concentrated. To this aldehyde is added, 5'-amino, 3-(O-t butyldimethylsilyl)thymidine, toluene, and titanium tetraisopropoxide. After stirring for 1 hour, ethanol (20 ml abs) and sodium cyanoborohydride are added and the reaction stirred. The solution is poured into 10% aq sodium bicarbonate solution and the crude product extracted with chloroform; dried (Na₂SO₄); stripped to dryness, and chromatographed on silica (1% Et₃N/5 to 10% methanol/MC) to yield the product **6**.

Compound **6** is dissolved in THF and tetrabutylammonium fluoride is added. The reaction solution is stirred, concentrated and applied to a silica gel column and chromatographed (1% Et₃N/5 to 10 to 15% MeOH/MC) to yield the product **7**.

To a solution of compound **7** in acetonitrile and methanol is added N-(9-Fluorenylmethoxycarbonyloxy) succinimide, and the solution stirred. The crude product is concentrated to dryness; toluene is then added and the solution is again evaporated to dryness to deliver the product **8**.

Compound **8** is dried by azeotropic distillation with pyridine. To a solution of **8** in pyridine and MC at 0°C is added a solution of 2-chloro-4H-1,3,2-benzodioxaphosphorin-4-one (PA) (1 M in methylene chloride). The solution is stirred and quenched with pH 7.5 triethyl ammonium bicarbonate (TEAB). The crude product is extracted with 4:1 MC/n-butanol, dried (Na₂SO₄), and diluted with acetonitrile. The solution is concentrated and chromatographed on silica gel (1% pyr/O to 20% H₂O/acetonitrile). The product-containing fractions are concentrated, diluted with toluene and concentrated again. The product is then dissolved in 3:1 MC/n-butanol and back extracted with pH 7.5 triethylammonium bicarbonate. The organic layer is dried (Na₂SO₄), diluted with acetonitrile, and concentrated to afford the final product **9**. The FMOC group can be substituted using conventional techniques.

### Example 2

The compounds used and generated in this example are shown in Scheme **2**, Figure 2. A mixture of compound **10** obtained as shown in Figure 19, Scheme 19, 10% palladium on carbon, ethyl acetate, and methanol is hydrogenated at atmospheric pressure. The reaction mixture is filtered through celite, and the solvent is evaporated. The crude product is chromatographed on silica gel (0.5% TEA/5% MeOH/MC) to yield the product **12**.

Compound **13** is dissolved in dioxane and water and treated with lithium hydroxide. The solution is poured into ice cold 0.1M H₃PO₄ and chloroform. The crude product is extracted with chloroform, dried over Na₂SO₄, concentrated, and chromatographed on silica gel (5% methanol/MC) to yield the carboxylic acid **14**.

To a solution of carboxylic acid **14** in tetrahydrofuran at 0°C is added BH₃-THF (1.OM in THF) in three portions. The mixture is slowly poured into ice cold aqueous sodium bicarbonate. The product is extracted with chloroform, dried over sodium sulfate, and concentrated to provide alcohol **15**.

A solution of **15** in DMSO is treated with N,N'dicyclohexyl carbodiimide (DCC) and dichloroacetic acid, and the mixture stirred. The reaction mixture is poured into 5% aqueous bicarbonate, and the crude product extracted with chloroform, dried over sodium sulfate, concentrated, and chromatographed on silica gel (5% MeOH/MC) to afford the aldehyde **16**.

The aldehyde **16** and amine **12** may be coupled and then converted into the phosphonate **18** in analogous fashion as described for compound **6** (Example 1). Following synthesis, the FMOC group can be replaced using conventional methods.

### Example 3

The compounds used and generated in this example are shown in Scheme **3**, Figure 3.

Preparation of **20**: To a dry (azeotroped from pyridine at reduced pressure) sample of compound 2 is added dry CHCl₃ (ethanol-free) and stirred at room temperature until a solution results. To this solution is added 4-methyl-1,2,4-triazoline-3,5-dione. The resulting red solution should be protected from light and allowed to stir at room temperature overnight. More 4-methyl-1,2,4-triazoline-3,5-dione is added, and the reaction mixture is protected from the light and allowed to stir at room temperature overnight. The reaction mixture is diluted with CHCl₃ and the organic phase washed with saturated aqueous NaHCO₃, separated, and dried over Na₂SO₄. Removal of solvents affords a dark yellow oil, which is purified by column chromatography with Baker, Inc. silica gel, using a step gradient of 4%-20% isopropyl alcohol in CH₂Cl₂ as eluent. This will afford a clear oil, whose 'H NMR spectral properties are consistent with the structure of **20**.

Compound **20** is oxidized to **21**. Compound **21** is coupled with amine **22** and may be subsequently converted into the phosphonate **24** in a similar manner to that described for compound **2**.

The FMOC group may be substituted using conventional methods.

### Example 4

The compounds used and generated in this example are shown in Scheme **4**, Figure 4.

To a solution of **25** in THF is added NaH (60% dispersion in oil), and the solution stirred. Allyl iodide is added, and the solution stirred for an additional period. The reaction mixture is poured in 5% aqueous bicarbonate, and the crude product is extracted with MC, washed with saturated brine, dried over sodium sulfate, and concentrated to deliver the product **26** as a crisp yellow foam.

Compound **26** is converted into aldehyde **28** in the manner previously described for compound **2**. Aldehyde **28** is coupled with compound **5** and subsequently converted to the phosphonate **30** as described above.

The FMOC group may be substituted using conventional methods.

### Example 5

### Preparation of 5'-TCTCme(CH₂-CH₂-NH)TCme(CH₂-CH2-NH)TCme(CH₂-CH₂-NH)TCme(CH₂-CH₂-NH)TTTT-2'

The oligomer of this example is synthesized using the conventional techniques described by Froehler, B.C. et al., Nucleic Acids Res (1986) 14:5399, but with the incorporation of the Cme(CH₂-CH₂-NFMOC)T dimer synthon. This dimer is constructed using the technique described in Example 1. The oligomers resulting from the synthesis may be deblocked with concentrated ammonia and gel purified using conventional techniques.

### Example 6

### Preparation of 5'-TCTCme(O-CH₂-CH₂-NH)TCme(O-CH₂-CH₂-NH)TCme(O-CH₂-CH₂-NH)TCme(O-CH₂-CH₂-NH)TTTT-2'

The oligomer of this example is synthesized as in Example 6, using the conventional techniques described by Froehler, B.C. et al., Nucleic Acids Res (1986) 14:5399, but with the incorporation of the Cme(O-CH₂-CH₂-NFMOC)T dimer synthon. This dimer is constructed using the technique described in Example 4. The oligomer resulting from the synthesis is deblocked with concentrated ammonia and gel purified using conventional techniques.

### Example 7

### Preparation of 5'-TCTCTC(CH₂-CH₂-O)TC(CH₂-CH₂-O) TCTTTT-2'

The oligomer prepared in this example consisted of conventional nucleotides as well as modified internucleoside linkages wherein the C preceding each of the modified linkers is a hydroxyethyl morpholino cytidine. This oligomer is synthesized as in Example 6, using the conventional techniques described by Froehler, B.C. et al., Nucleic Acids Res (1986) 14:5399, but with the incorporation of the morpholine C(CH₂-CH₂-O)T dimer synthon. This dimer is constructed using the technique described in Example 5. The oligomers resulting from the synthesis may be deblocked with concentrated ammonia and gel purified using conventional techniques.

### Example 8

### Preparation of T (NR-CH₂-CH₂) T

The preparation of alkyl derivatives of the 2' amine, as shown in Scheme 13, Figure 13 begins with azide **10**. Compound **10** in methanol with 10% palladium on carbon is hydrogenated. The catalyst may be removed by filtration and the solvent removed by rotary evaporation to deliver the amine **12**. To a solution of amine **12**, acetaldehyde and toluene is added titanium isopropoxide and the solution stirred. At this point absolute ethanol (25 mmol) and sodium cyanoborohydride may be added. The mixture is subsequently stirred and stripped to dryness.

The crude product is chromatographed on silica gel (1% Et₃N/3 to 5 to 8% 2-propanol/MC) to deliver the product as a white foam. In a similar manner, amines **82** and **83** may be prepared. Compounds **81-83** are then coupled with aldehyde **16** as described for amine **12** to deliver dimers **84-86**, which may then converted to the corresponding phosphonates **87-89** as described for compound **18**.

The preparation of acylated derivatives of the 2' amine may begin with the dimer **90**, which is prepared as described for compound **17**. Dimer **90** is deprotected with tetrabutylammonium fluoride as described for compound **7** to yield dimer **91**. To a solution of amine **91**, ethyl acetate and 5% aqueous sodium bicarbonate is added ethyl chloroformate. The organic layer is separated, dried over sodium sulfate, and concentrated. The crude product is chromatographed on silica gel (3 to 5 to 10 to 15% 2-propanol/MC) to yield the product **92**. Likewise, carbamate **93** is prepared. Compounds **92** and **93** are subsequently converted to the phosphonates **94** and **95** as described above.

### Example 9

As is shown in Scheme 14, shown in Figure 14, the aminal derivative **101** is prepared from amine **12**, which is acylated with ethyl chloroformate to give carbamate **99**. The carbamate **99** is alkylated with chloromethyl methylsulfide in the presence of sodium hydride to afford thioaminal **100**. Compound **100** is activated with bromine in the presence of alcohol **31** to deliver dimer **101**, which is then converted to the corresponding phosphonate **102** using the method described above.

### Example 10

The compounds of this example are shown in Scheme 15, Figure 15. To a solution of alcohol **46** in DMF and pyridine is added methylthiophenoxyphosphonium iodide, and the reaction stirred. The reaction is quenched with methanol and the solvents removed on the rotary evaporator. The crude product is dissolved in methylene chloride; is extracted with aqueous saturated sodium thiosulfate and aqueous saturated sodium bicarbonate; dried; concentrated; and chromatographed on silica gel to deliver the iodide **103**.

To a solution of thiol **42** (which may be produced using Scheme 18 in Figure 18) and acetonitrile is added bis(trimethylsilyl) acetamide. The solvent may be evaporated; DMF and iodide 103 are added. The reaction is stirred and then quenched with aqueous saturated sodium bicarbonate. The crude product is extracted with methylene chloride; dried; concentrated; and chromatographed on silica gel to deliver dimer **104**. Dimer **104** is converted to the phosphonate **105** as described above.

### Example 11

### Preparation of Compound 120

This Example shows the preparation of Compound **120**. The synthesis is found in Figure 16.

Compound **107** (Aldrich) was treated with toluyl chloride in pyridine/CH₂Cl₂ followed by an aqueous work-up. The resulting syrup was crystallized from ether/hexane to yield white needles (89%). This 5-toluyl compound was then treated with phenoxythionocarbonyl chloride and DMAP in acetonitrile followed by an aqueous work-up to yield a tan solid (**106**).

Compound **106** was treated with tributyltin hydride and AIBN in toluene at 80°C for 4 hours. The solvent was removed in vacuo and the resulting oil subjected to column chromatography and eluted with 15% EtOAc/hexane to yield a clear, colorless syrup. This syrup was dissolved in dioxane/1N HCl and heated at 65°C for 1 hour. The solvent was cooled and neutralized by addition of saturated aqueous NaHCO₃ (pH=6). The solution was then reduced in vacuo until a two phase solution was observed. The solution was diluted with EtOAc and washed with saturated aqueous NaHCO₃ and brine. The organic phase was decanted, dried, and reduced to a yellow syrup. This syrup was dissolved in pyridine/CH₂Cl₂ and treated with benzoyl chloride for 12 hours. The solvent was removed and the residue subjected to an aqueous work-up. The resulting syrup was subjected to column chromatography and eluted with 15% EtOAc/hexane.

Thymine was silylated in acetonitrile with BSA at 70°C and treated with a solution of compound 108 followed by TMSOTf. The solution was stirred for 1 hour, cooled, and subjected to an aqueous work-up. The resulting white foam was subjected to column chromatography and eluted with 55% EtOAc/hexane.

Compound 110 was dissolved in anhydrous THF and treated with MeONa followed by neutralization and an aqueous work-up. The resulting white foam was crystallized from Et₂O to yield a white powder.

Compound 110a was dissolved in THF and treated with PPh₃ and DEAD at 0°C for 30 minutes. The solvent was removed and the resultant oil subjected to column chromatography and eluted with 7% MeOH/CH₂Cl₂.

Compound 112 was dissolved in DMF and treated with LiN₃ and NH₄Cl at 100°C for 8 hours. The solution was cooled and the solvent removed and the residue subjected to an aqueous work-up. The resultant foam was dissolved in EtOH and treated with 10% Pd/C. The suspension was then hydrogenated at 414 kPa (60psi) H₂ for 12 hours. The suspension was filtered and the filtrate reduced to a white foam.

Compound 114 was dissolved in DMF and compound 115 and TEA were added. This solution was stirred at room temperature for 90 minutes. The solvent was removed and the residue subjected to column chromatography and eluted with 4% MeOH/CH₂Cl₂.

Compound **118** was dissolved in MeOH/NH₃ and the sealed flask was heated at 70°C for 12 hours. The solvent was removed and the residual foam was crystallized from Et₂O to yield a white powder. This powder was dissolved in pyridine and treated with DMTCl for 3 hours. The solvent was removed and the residue subjected to an aqueous work-up. The residual oil was subjected to column chromatography and eluted with 3% MeOH/CH₂Cl₂. The resultant white foam was dissolved in THF and treated with Bu₄NF for 1 hour and the solvent was removed in vacuo. The resultant white foam was subjected to column chromatography and eluted with 8% MeOH/CH₂Cl_{2 -} to yield a white powder. This powder was dissolved in pyridine/CH₂Cl₂ and cooled to 0°C and treated with van Boom's reagent for 30 minutes. The solution was neutralized with TEAB (1N, pH=7) and extracted with CH₂Cl₂ and reduced to a white foam. This foam was subjected to column chromatography and eluted with 12% MeOH/CH₂Cl₂/0.5%TEA to recover compound **120**.

### Example 12

### Preparation of Compounds 128, 138a, 138b

This Example, depicted in Figure 17, shows two synthesis reactions for the preparation either of 5',2' carbamate or 5',2' methyl carbamate linkages.

Compound **110a** (prepared using the procedure shown in Figure 16) was dissolved in CH₂Cl₂ and cooled to 0°C. Paraformaldehyde was added and HCl (anhydrous) was passed through the suspension until a solution resulted. The flask was sealed and stored at 5°C for 16 hours. After this time the solvent was removed to yield a white foam (compound **122**) that was used without further purification in the following steps.

Compound **122** and compound **124** were dissolved in CH₂Cl₂ and Hunig's base was added. The resulting solution was stirred at room temperature for 3 hours. The solution was diluted with CH₂Cl₂ and subjected to an aqueous work-up. The resultant foam was subjected to column chromatography and eluted with 4%iPA/CH₂Cl₂ to yield a white foam containing compound **126**.

Compound **126** was dissolved in MeOH and treated with MeONa (trace) at 50°C for 1 hour. The solvent was removed and the solid subjected to column chromatography and eluted with 10%MeOH/CH₂Cl₂ to yield a white foam. This foam was dissolved in pyridine and treated with DMTCl and stirred at room temperature for 2 hours. The solvent was then removed and the residue treated to an aqueous work-up and the residual foam subjected to column chromatography and eluted with 6%MeOH/CH₂Cl₂ to yield a white foam. This foam was dissolved in pyridine/CH₂Cl₂ and cooled to 0°C and treated with van Boom's reagent and stirred for 30 minutes. The solution was neutralized with TEAB (1N, pH=7) and extracted. The resulting white foam was subjected to column chromatography and eluted with 12% MeOH/0.5% TEA/CH₂Cl₂ to yield a white foam (compound **128**).

In the other synthesis route depicted in Figure 17, compound **110a** was dissolved in pyridine and treated with p-nitrophenylchlorocarbonate and stirred at room temperature for 12 hours. The solvent was removed and the resulting foam subjected to column chromatography and eluted with 50%EtOAc/hexane to yield a white foam (compound **132**).

Compound **132** was dissolved in DMF and treated with compound **134a** and TEA. The solution was stirred at room temperature for 24 hours. The solvent was removed and the resulting yellow syrup subjected to column chromatography and eluted with 4%MeOH/CH₂Cl₂ to yield a white foam.

Compound **136b** was prepared using the procedure described for compound **134a** except that compound **134b** was used as the reactant.

Compound **136a** was dissolved in MeOH/NH₃ and heated in a sealed flask at 65°C for 16 hours. The solvent was removed and the resulting white foam was crystallized from Et₂O to yield a white powder. This powder was dissolved in pyridine and treated with DMTCl and stirred at room temperature for 3 hours. The solvent was removed and the residue treated to an aqueous work-up and subjected to column chromatography and eluted with 4%MeOH/CH₂Cl₂ to yield a white foam. This foam was dissolved in THF and treated with TBAF and stirred at room temperature for 45 minutes. The solution was diluted with EtOAc and subjected to an aqueous work-up. The resulting white foam was then crystallized from Et₂O to yield a white powder. This powder was dissolved in pyridine/CH₂Cl₂ and cooled to 0°C and treated with van Boom's reagent and stirred for 30 minutes. The solution was neutralized with TEAB (1N, pH=7) and extracted. The resulting white foam was subjected to column chromatography and eluted with 12%MeOH/0.5%TEA/CH₂Cl₂ to yield a white foam containing compound **138a**.

Compound **136b** was used in the same fashion as described just above in the preparation of compound **138a** to yield compound **138b**.

### Example 13

RNA and DNA duplex and DNA-triplex experiments utilizing certain desirable substitute linkages of this invention were conducted to determine those linkages' effect on the Tm values of the resulting oligomers.

These experiments were carried out in a buffered solution (140 mM KCl, 5 mM Na₂HPO₄, and 1 mM MgCl₂) at pH=6.6 (except for the 2',5'-carbamate which was buffered at pH=7.0) according to the following protocol: 0.15 ODs of the target RNA/DNA was combined with 0.1 OD of the oligomer being assayed in a sterile eppendorf tube and dried. To this mixture was added 300 λ of Tₘ buffer and the solution was stirred. Tₘ values were then determined by a first derivative plot of absorbance versus temperature. Thermal denaturation analysis was carried out with a heating rate of 0.25°C/min and absorbance was monitored at 260 nm.

The test oligomers that were synthesized for analysis were of the following sequence: 5'-TCᵐTCᵐTCᵐTCᵐTCᵐTᴬTTᴬTT-3' where T = thymidine, C^{m} = 5-methyl-2'-deoxycytidine, and T^{A}T = a thymidine-thymidine dimer with an experimental linkage of the structure detailed in Table 2 below. All other linkages were phosphodiester.

Target Duplex Sequence (DNA)

5′ AGAGAGAGAGAAAAA 3′ ← target strand
3′ TCTCTCTCTCTTTTT 5′ ← complement of target

Single Stranded Target (DNA or RNA)
↓
5′ AAAAAGAGAGAGAGA 3′          T = U for RNA

The linkage designated 5',2' thioformacetal has sulfur linked to the 5' carbon and the linkage designated 2',5' thioformacetal has sulfur linked to the 2' carbon. Similarly, the linkages designated 5',2' carbamate and 5',2' methyl carbamate have nitrogen linked to the 5' carbon atom (e.g. compounds **138a** and **138b**) and the 2',5' carbamate linkage has nitrogen linked to the 2' carbon atom (e.g. compound **120**).

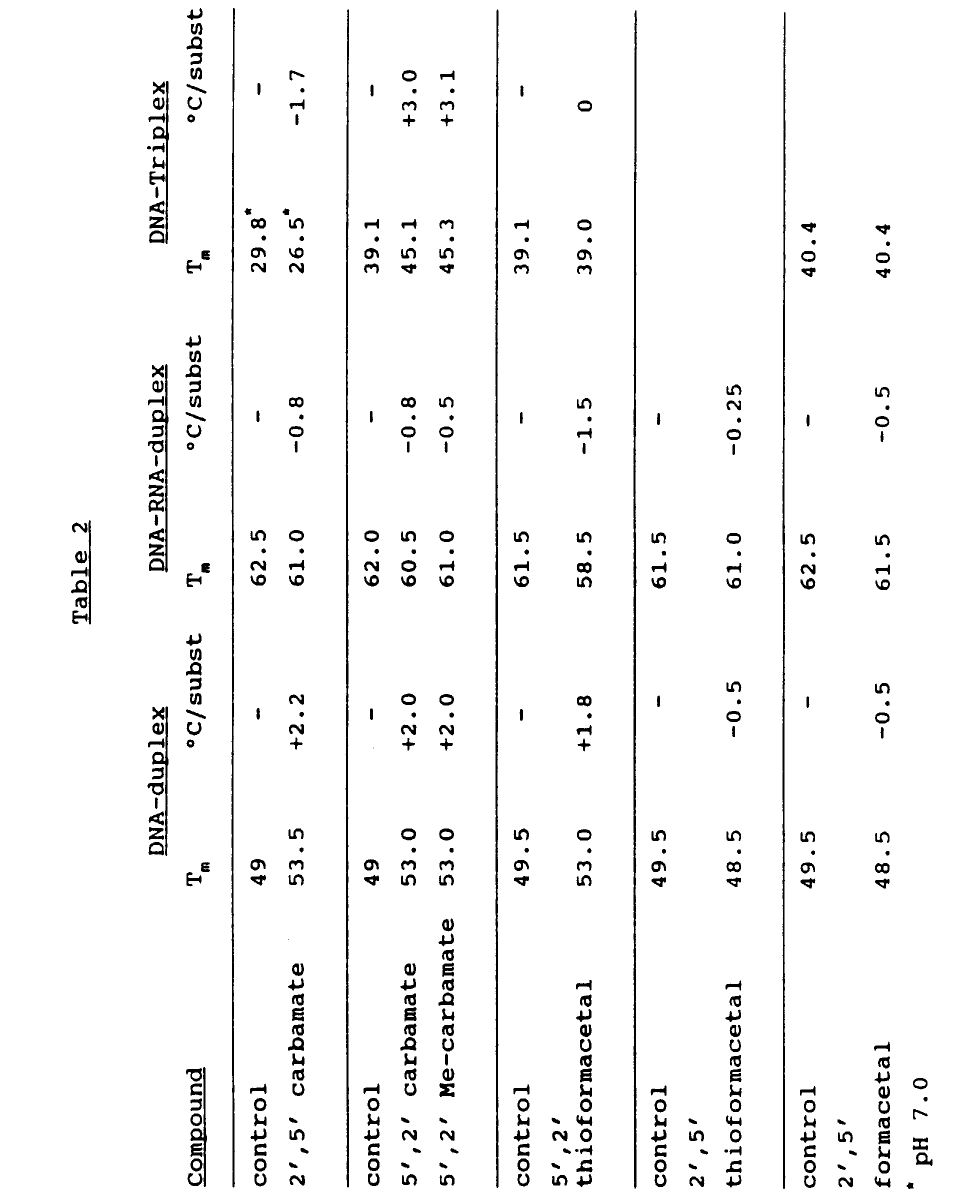

### Example 14

The dimer, **55**, shown in Figure 9 having a two atom long sulfur-containing linkage was synthesized as follows. The nitrile **38** was synthesized by adding to a solution of 3'-silylthymidine (2.33 g, 5.0 mmol) in DMF (25 mL), methyltriphenoxyphosphonium iodide (2.92 g, 6.5 mmol). The solution was then stirred for 18 h. Sodium cyanide (490 mg, 100 mmol) was added and the solution was stirred for 18 h. Methanol was added, and the solution was concentrated. The solution was partitioned between ethyl acetate (EtOAc) (100 mL) and Satd. NaHCO₃ (100 mL) and the crude product was washed with aqueous thiosulfate (100 mL), dried, concentrated, and chromatographed (EtOAc:hexane 4:6) to deliver the product **38** (1.14 g, 49%).

The aldehyde, **39**, was synthesized as follows. To a solution of nitrile (1.14 g, 2.45 mmol) in toluene at -78°C, was added diisobutyl aluminum hydride (Dibal) (1.5 M, 7.5 mL), and the solution was stirred for 30 min. Ethanol (2 mL) was added, followed by sodium fluoride (3.0 g), and water (2 mL). The mixture was filtered through celite, and the crude product was extracted with EtOAc (100 mL), dried, concentrated, and chromatographed (EtOAc:Hexane 4:6) to yield the product (625 mg).

**53**. 0.49 g (1 mmole) of aldehyde **39** was treated with NaBH₄ (38 mg, 1 mmole) in 5 mL of EtOH at rt. for 1 h. After removal of EtOH, the residue was extracted with EtOAc and washed with 1M citric acid. The organic layer was washed then with water, dried over Na₂SO₄, evaporated and finally purified by flash chromatography in methanol/methylene chloride (0:100-1:99) to obtain 0.43 g (88%) of 53.

**54**. 0.4 g (0.8 mmole) of **53** was dissolved in 5 mL of dry DMF. At rt. 0.42 g (1.6 mmole) of triphenylphosphine and 0.53 g (1.6 mmole) of carbon tetrabromide were added into the reaction mixture. Two hours later TLC show completion of the reaction. The mixture was evaporated to dryness and extracted with EtOAc/water. The organic layer was dried over Na₂SO₄ and purified after concentration by flash chromatography in hexane/EtOAc (10:1-1:1) to yield 0.34 g (76%) of **54**.

**Thymidine-thymidine 2'S thioether 55**. A solution of 0.14 g (0.25 mmole) of 54 was syringed to a 0°C solution of 2'-mercapto-5'-O-DMT-2',3'-dideoxythymidine (**45**) and 0.5 mL of sodium trimethylsilanolate (1M solution in THF) in methylene chloride (5.0 mL) under nitrogen. After stirring for 1 h, the reaction was added 10 mL of methylene chloride and quenched with water. The organic layer was separated, dried (Na₂SO₄), concentrated, and purified by flash chromatography in methanol/methylene chloride (0:100-5:95) to yield 0.13 g (51%) of T*T dimer **55**.

### Example 15

The formacetal linkage shown in Figure 20 was synthesized as follows.

**5'-Toluyl-2'-methylthiomethylthymidine (2' MTMT)**: 5'-Toluylthymidine ((0.12 g, 0.33 mmol) was dissolved in MeCN (4 mL) and Me₂S (0.24 mL, 3.3 mmol) was added and the solution was cooled to 0°C. Bz₂O₂ (0.32 g, 1.3 mmol) was added and the resulting solution was allowed to warm to 20°C and stirred for 15 hours. Met anol was added and the solvent was removed in vacuo. The residue was dissolved in CH₂Cl₂ and washed with NaHCO₃ (sat) and brine. The organic layer was decanted, dried over Na₂SO₄, filtered, and reduced and the residue purified by column chromatography to yield a white foam.

**T-T formacetal dimer: 2' MTMT** (0.34 g, 0.81 mmol) and 3'-TBSthymidine (0.58 g, 1.63 mmol) were disolved in benzene/CH₂Cl₂ (1/1, 10 mL) and stirred with 4A molecular sieves for 2 hours. The solution was then cooled to -10°C and Br₂ (0.12 g, 0.77 mmol, in 0.5 mL CH₂Cl₂) was added and the solution allowed to warm to RT and stirred for 16 hours. The solution was then diluted with EtOAc and washed with NaHCO₃, H₂O, and brine. Organic layer decanted, dried over Na₂SO₄, filtered, and reduced to a foam. The foam was dissolved in NH₃/MeOH (10mL) and heated at 60°C for 12 hours. The solvent was removed and the residue was subjected to column chromatography and eluted with MeOH/CH₂Cl₂ (2-3-4-6% of MeOH) to yield a whited foam; yield=0.12 g (26%).

**5-DMT-3'-TBS t-t formacetal dimer**: The 5'-OH dimer (0.12 g, 0.13 mmol) was protected as described previously except the product was purified by column chromatography and eluted with MeOH/CH₂Cl₂ (1-2-3-4% MeOH) to yield a white foam; yield=0.15 g (83%).

**5'-DMT-T-T formacetal dimer-H-Phosphonate**: 5'-DMT-3'-TBS T-T dimer was desilated as described before with TBAF but was purified by crystallization from Et₂O to yield a white powder; yield=0.08 g (80%). The H-phosphonate was prepared as described before but the compound was purified by column chromatography and was eluted with MeOH/CH₂Cl₂/TEA (5-10-15% MeOH/0.5% TEA) to yield a white foam; yield=0.062 g (74%).

### Example 16

The 2',5' thioformacetal linkage shown in Figure 25 was synthesized as follows.

**3'-Deoxy-5'-O-DMT-2'-O-methanesulfonyl-β-D-arabinosylthymine**. 2.18 g (4.0 mmole) of 3'-Deoxy-5'-O-DMT-B-D-arabinosylthymine (Webb, T. R., Mitsuya, H., Broder, S., J. Med. Chem. **1988**, 31, 1475) was dissolved in 20 mL of dry pyridine. At 0°C, 0.5 mL of methane sulfonyl chloride (1.5 eq) was added into the solution, dropwise. After 2 h the reaction mixture was poured into ice water, the precipitate collected, then dissolved in methylene chloride, washed with water, dried over Na₂SO₄, and evaporated to dryness. The residue was purified on silica gel column in methanol/methylene chloride (0:100-2:98) to yield 2.2 (90%) of 3'-Deoxy-5'-O-DMT-2'-O-methanesulfonyl-β-D-arabinosylthymine.

**2'-S-Acetyl-2',3'-dideoxy-5'-O-DMT-thymidine**. 3'-Deoxy-5'-O-DMT-2'-O-methanesulfonyl-β-D-arabinosyl thymine (0.31 g, 0.5 mmole) was heated with potassium thioacetate (0.3 g, 5.2 mmole) in 5 mL of dry DMF for 2 h. The reaction mixture was evaporated to dryness. The residue was then dissolved in 20 mL of methylene chloride and washed with 20 mL 10% sodium bicarbonate solution and with 20 mL of water. The organic phase was dried over Na₂SO₄ and evaporated to dryness. The residue was purified on silica gel column in methanol/methylene chloride (0:100-2:98) to yield 0.24 (79%) of 2'-S-acetyl-2',3'-dideoxy-5'-O-DMT-thymidine.

**2'-Mercapto-2',3'-dideoxy-5'-O-DMT-thymidine**. 2'-S-acetyl-2',3'-dideoxy-5'-O-DMT-thymidine was treated with saturated ammonia in cold methanol for 30 min. Then the mixture was evaporated under reduced pressure with the exclusion of oxygen. Proton NMR shows 95% conversion to 2'-Mercapto product. The residue was used for the next step without further purification.

**3'-O-Isobutyrylthymidine**. To a solution of 5'-O-DMT-Thymidine (5.44 g, 10.0 mmol) in pyridine (30 mL) was added isobutyric anhydride (3.3 mL, 20.0 mmol) dropwise and the mixture was stirred at 20°C for 18 h. The reaction was quenched with methanol (2.5 mL) and concentrated in vacuo. The crude product was extracted with methylene chloride (30.0 mL), washed with saturated aqueous sodium bicarbonate (30.0 mL), dried over Na₂SO₄ and concentrated. The residual oil was dissolved in 25% methanol/methylene chloride (100 mL) and treated with p-toluenesulfonic acid (2.85 g, 15 mmol) at 0°C. After 0.5 h, the orange-red solution was quenched with saturated aqueous sodium bicarbonate (300 mL), and the organic layer was dried (Na₂SO₄) and concentrated. The crude product was dissolved in ethylacetate (25 mL) and precipitated by addition of hexane (250 mL) and cooled to -10°C for 18 h. The mixture was filtered, and the precipitate was dried under high vacuum to afford 3'-O-isobutyrylthymidine (62%).

**Thymidine-thymidine (2',5') thioformacetal**. Into a solution of 3'-O-isobutyrylthymidine (0.312 g, 1.0 mmol), paraformaldehyde (45 mg, 1.5 mmol) and methylene chloride (10.0 mL) at 0°C was bubbled anhydrous hydrogen chloride for 10 min, and the solution was held at 0°C for 2 h. The solution was thoroughly dried (Na₂SO₄), and the solvent was evaporated to afford the chloromethyl ether. This chloromethyl ether was dissolved in methylene chloride (5.0 mL) and added dropwise to a 0°C solution of 2'-mercapto-5'-O-DMT-2',3'-dideoxythymidine (prepared from 0.6, 1.0 mmol of correspondent thioacetate) and diisopropylethylamine (DIPEA, 0.5 mL) in methylene chloride (5.0 mL). After stirring for 1 h, the reaction was quenched with saturated aqueous sodium bicarbonate (10.0 mL). The organic layer was separated, dried (Na₂SO₄), concentrated, and purified by flash chromatography in methanol/methylene chloride (0:100-5:95) to yield 0.27 (65%) T*T dimer. The resulting product was treated with sodium methoxide (0.20 g, 3.64 mmol) in methanol (20 mL) for 1 h. The reaction was quenched with acetic acid (lM solution) and concentrated. The crude product was extracted with methylene chloride, dried (Na₂SO₄) and purified by flash chromatography in methanol/methylene chloride (1:99-7:93) to deliver the product (0.17 g, 70%).

**H-Phosphonate of T*T dimer**. To a solution of PA (1.0 M in methylene chloride, 0.4 mmole), methylene chloride (5 mL), and pyridine (0.76 g, 0.8 mmole) at 0°C was added the T*T dimer (0.16 g, 0.20 mmole) in methylene chloride (2 mL). The reaction mixture was stirred at RT for 15 min, diluted with methylene chloride (10 mL), and quenched with TEAB (1M aqueous solution, 10 mL). The organic phase was dried (Na₂SO₄) and evaporated. Subsequent purification by flash chromatography in TEA/methanol/methylene chloride (0.5:2:97.5-0.5:5:94.5) delivered 0.14 g (72%). Thus, modified oligomers for use in oligonucleotide-based therapies have been disclosed. Although preferred embodiments of the subject invention have been described in some detail, it is understood that obvious variations can be made without departing from the spirit and the scope of the invention.

The results demonstrated that all linkages within the scope of the invention that were examined are binding competent. In some instances, notably in Hoogsteen binding to duplex DNA, binding of some invention 2',5' linkages is equal to or greater than corresponding control 3',5' phosphodiester linkages.

### Example 17

**Reduction of thymidine α,β-unsaturated aldehyde (TUA) to thymidine allyl alcohol (TAA)**. 0.5 g (1.0 mmole) of TUA (Montgomery, J. A. et al, J Org Chem (1981) 46:594; U. S. Patent No. 4,822,316) was treated with NaBH₄ (38 mg, 1.0 mmole) and 0.16 mL (1.0 mmole) of triethylsilane in 5 mL of THF at 20°C for 1 h. After removal of THF the residue was extracted with EtOAc and washed with 1 M citric acid. The organic layer was washed then with water dried over Na₂SO₄, evaporated and purified by flash chromatography in methanol/methylene chloride (0:100 - 1:99) to obtain 0.46 g (91%) of TAA.

**Bromnation of allyl alcohol TAA**. 0.5 g (1.0 mmole) of TAA was dissolved in 5 mL of dry DMF. At 20°C 0.53 g (2.0 mmole) of triphenylphosphine and 0.66 g (1.6 mmole) of carbon tetrabromide were added into the reaction mixture. Two hours later the reaction was shown to be complete by TLC. The mixture was evaporated to dryness and extracted with EtOAc/water. The organic layer was dried over Na₂SO₄ and purified after concentration by flash chromatography in hexane/EtOAc (1:1 - 1:4) to yield 0.45 g (81%) of thymidine allyl bromide (TAB).

**Thymidine-thymidine 2'S allylsulfide (TTAS-Si)**. A solution of 0.57 g (1.0 mmole) of TAB was added via syringe to a 0°C solution of 2'-mercapto-5'-O-DMT-2',3'-dideoxythymidine (**42**) and 0.35 mL (2 eq.) of diisopropyl ethyl amine (DIPEA) in methylene chloride (5.0 mL) under nitrogen. After sitrring overnight, the reaction was added to 10 mL of methylene chloride and quenched with water. The organic layer was separated, dried (Na₂SO₄), concentrated, and purified by flash chromatography in methanol/methylene chloride (0:100 - 3:97) to yield 0.75 g (72%) of T*T dimer (TTAS-Si).

**Desilylation on the 3'-position of T*T dimer (TTAS-Si)**. TTAS-Si (0.63 g, 0.6 mmole) was dissolved in 5 mL of THF and treated with 1 mL of 1 M TBAF/THF for 1 h. The reaction was concentrated. The crude product was extracted with methylene chloride, dried (Na₂SO₄) and purified by flash chromatography in methanol/methylene chloride (1:99 - 7:93) to deliver the product (TTAS) (0.31 g, 63%).

**H-phosphonate of T*T dimer**. To a solution of 2-chloro-4H-1,3,2-benzodioxaphosphorin-4-one (PA) (1.0 M in methylene chloride, 0.4 mmole), methylene chloride (5 mL), and pyridine (0.76 g, 0.8 mmole) at 0°C was added to T*T dimer (TTAS) (0.16 g, 0.20 mmole) in methylene chloride (2 mL). The reaction mixture was stirred at rt for 15 min., diluted with methylene chloride (10 mL), and quenched with TEAB (1 M aqueous solution, 10 mL). The organic phase was dried (Na₂SO₄) and evaporated. Subsequent purification by flash chromatography in TEA/methanol/methylene chloride (0.5:2:97.5 - 0.5:5:94.5) and then in TEA/H₂O/acetonitrile (0.5:2:97.5-0.5:5:94.5) delivered 0.14 g (69%).

## Claims

1. An oligomer comprising at least one substitute linkage between the 2' and 5' position of adjacent nucleomonomers, said substitute linkage having two to four atoms wherein at least one of the atoms making up the substitute linkage is selected from nitrogen, oxygen, or sulfur, with the remainder king carbon provided that no adjacent two or three atoms are oxygen nor adjacent three atom are all oxygen, nitrogen, or sulfur.

2. The oligomer of claim 1 wherein the substitute linkage contains an oxygen atom directly attached to the 2' position of the adjacent nucleomonomer.

3. An oligomer as claimed in Claim 1, wherein the substitute linkages are independently selected from the group consisting of:
-N(R⁶)-CH₂-
-CH₂-N(R⁶)-
-N(R⁶)-N(R⁶)-
-N(R⁶)-CH₂-CH₂-
-CH₂-N(R⁶)-CH₂-
-CH₂-CH₂-N(R⁶)-
-N(R⁶)-N(R⁶)-CH₂-
-N(R⁶)-CH₂-N-
-CH₂-N(R⁶)-N(R⁶)-
-N=C(NH₂)-N(R⁶)-
-O-CH₂-
-CH₂-O-
-O-CH₂-CH₂-
-CH₂-O-CH₂-
-CH₂-CH₂-O-
-O-CH₂-O-
-S-CH₂-O-
-O-CH₂-S-
-S-CH₂-S-
-S-CH₂-S(O)(O)-
-S-CH₂-
-CH₂-S-
-S(O)-CH₂-
-S(O)(O)-CH₂-
-CH₂-S(O)-
-CH₂-S(O)(O)-
-S-CH₂-CH₂-
-S(O)-CH₂-CH₂-
-S(O)(O)-CH₂-CH₂-
-CH₂-CH₂-S(O)(O)-
-CH₂-S(O)(O)-CH₂-
-CH₂-S-CH₂-
-CH₂-CH₂-S-
-S(O)-CH₂-S-
-S(O)(O)-CH₂-S(O)(O)-
-N(R⁶)-C(O)-S-
-N(R⁶)-C(S)-S-
-N(R⁶)-C(S)-N(R⁶)-
-N(R⁶)-C(O)-N(R⁶)-
-S-C(O)-N(R⁶)-
-S-C(S)-N(R⁶)-
-N(R⁶)-O-
-O-N(R⁶)-
-N(R⁶)-O-CH₂-
-N(R⁶)-CH₂-O-
-O-C(O)-N(R⁶)-
-O-C(S)-N(R⁶)-
-N(R⁶)-C(O)-O-
-N(R⁶)-C(S)-O-
-CH₂-N(R⁶)-O-
-O-N(R⁶)-CH₂-
-O-CH₂-N(R⁶)-
-CH₂-O-N(R⁶)-
-N(R⁶)-S(O)-
-N(R⁶)-S(O)(O)-
-S(O)-N(R⁶)-
-S(O)(O)-N(R⁶)-
-N(R⁶)-S(O)-CH₂-
-N(R⁶)-S(O)(O)-CH₂-
-N(R⁶)-CH₂-S-
-N(R⁶)-CH₂-S(O)-
-N(R⁶)-CH₂-S(O)(O)-
-N(R⁶)-S(O)(O)-
-S(O)(O)-N(R⁶)-
-S-N (R⁶)-CH₂-
-S(O)-N(R⁶)-CH₂-
-S(O)(O)-N(R⁶)-CH₂-
-CH₂-N(R⁶)-S-
-CH₂-N(R⁶)-S(O)-
-CH₂-N(R⁶)-S(O)(O)-
-S-CH₂-N(R⁶)-
-S(O)-CH₂-N(R⁶)-
-S(O)(O)-CH₂-N(R⁶)-
-CH₂-S-N(R⁶)-
-CH₂-S(O)-N(R⁶)-
-CH₂-S(O)(O)-N(R⁶)-
-N(R⁶)-S(O)-N(R⁶)-
-N(R⁶)-S(O)(O)-O-
-O-S(O)(O)-N(R⁶)-
-O-S(O)(O)-O-
-N(R⁶)-S(O)(O)-N(R⁶)-
-CH₂-S(O)(O)-O-
-O-CH₂-S(O)(O)-
-S(O)-CH₂-O-
-O-CH₂-S(O)-
-O-C(R⁸)₂-O-
-S-C(R⁸)₂-O-
-O-C(R⁸)₂-S-
-O-CH₂-CH=CH- (cis and trans isomers)
-S-CH₂-CH=CH- (cis and trans isomers)
-O-CH₂-C≡C-
and
-S-CH₂-C≡C-
wherein R⁶ is H- lower allyl, (1-6c), including methyl, ethyl, propyl, isopropyl and butyl, OMe, OH, heteroalkyl (1-6C, 1-2 halo, S, N or O heteroatoms), or aryl (6-7C); and
wherein R⁸ is CH₂F, or when both R⁸ are taken together with the atom to which they are attached, form a 4-membered or 6-membered ring where (R⁸)₂, is
-CH₂-X¹-CH₂-,
or
-(CH₂)₂-X¹-(CH₂)₂-;
wherein X¹ is selected from the group consisting of NH, NMe, NEt, NPr, S, SO, SO₂, O, CF₂ and CHF.

4. The oligomer of claim 3 wherein the substitute linkages are independently selected from the group consisting of:
-S-CH₂-CH₂
-S-CH₂-
-O-C(S)-NR⁶-
-O-CH₂-S-
-O-CH₂-O-
-O-C(O)-NR⁶-
-CH₂-CH₂-S
-CH₂-S-
NR⁶-C(S)-O-
-S-CH₂-O-
and
-NR⁶-C(O)-O-.

5. The oligomer of claim 3 where at least one substitute linkage is -S-CH₂-CH₂-, -CH₂CH₂-S-, -CH₂-S- or -S-CH₂-.

6. The oligomer of claim 3 wherein at least one substitute linkage is
2' -NH-C(O)-O- 5'
2' -O-C(O)-NH- 5'
2' -O-C(O)-NCH₃- 5'
or
2' -O-CH₂-S- 5'.

7. An oligomer as claimed in claim 1, having the formula (W, Y)-Q-(Z-Q)ₙ-(W,Y), where each 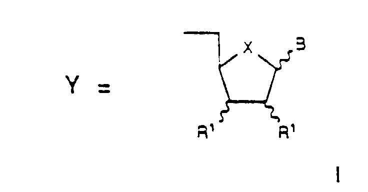 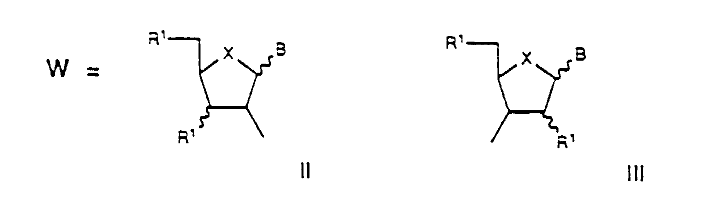 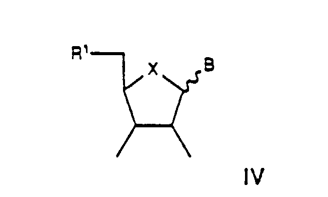 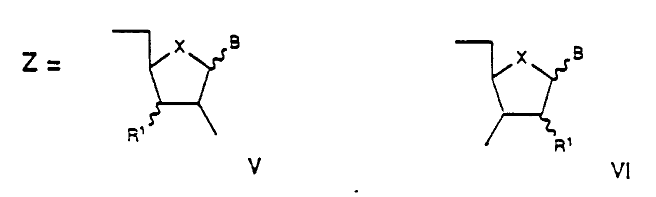 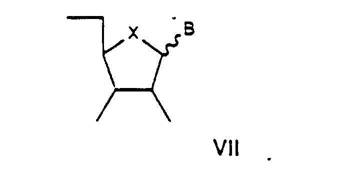 where X is S, O, CH₂, CHF or CF₂,
R¹ independently is -O-alkyl (C₁-C₁₂), -S-alkyl (C₁-C₁₂), H, OH, OCH₃, SCH₃, OC₃H₅ (O-allyl), OC₃H₇(O-propyl), SC₃H₅, or F and
where R¹ is on a terminal group of the oligomer, R¹ may additionally be PO₃⁻² or a blocking group selected from a dimethoxytrityl (DMT) moiety, a monomethoxytrityl (MMT) moiety, H-phosphonate (OPO₂H), methylphosphonate (OPO₂CH₃), methyl phosphonamidite or phosphoramidite;
B is a base, and
Q is independently
a phosphodiester analog or
a two to four atom long substitute linkage wherein at least one of the atoms making up the substitute linkage is selected from nitrogen, oxygen or sulfur, with the remainder being carbon but no two adjacent atoms are oxygen nor any adjacent three atoms are all oxygen, nitrogen or sulfur; and
n is 1-100, and
subject to the proviso that at least one Q is not a phosphodiester analog.

8. An oligomer as claimed in Claim 1, having the formula
(W,Y)-Q-(Z-Q)ₙ-(W,Y),
where each 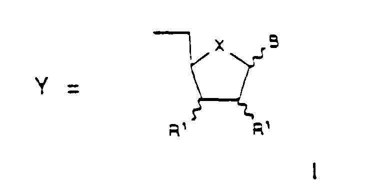 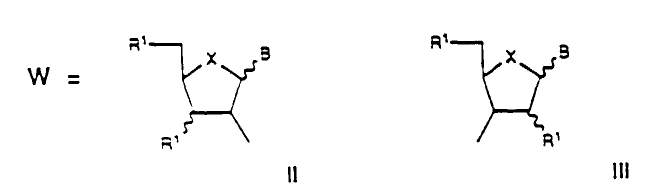 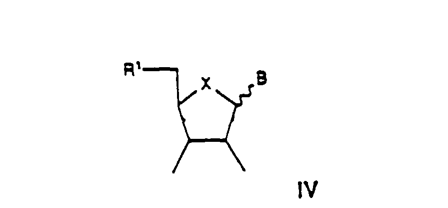 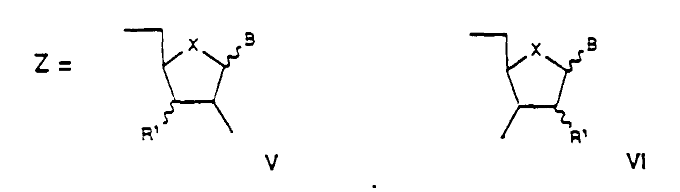 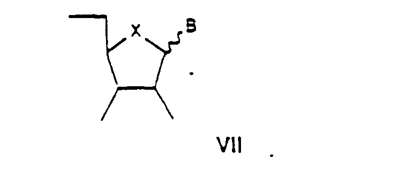 wherein X is S, O, CH₂, CHF or CF₂,
R¹ independently is -O-alkyl (C₁-C₁₂), -S-alkyl (C₁-C₁₂), H, OH, OCH₃, SCH₃, OC₃H₅ (O-allyl), OC₃H₇(O-propyl), SC₃H₅, or F and
when R¹ is on a terminal group of the oligomer, R¹ may additionally be PO₃⁻² or a blocking group selected from a dimethoxytrityl (DMT) moiety, a monomethoxytrityl (MMT) moiety, H-phosphonate (OPO₂H), methylphosphonate (OPO₂CH₃), methyl phosphonamidite or phosphoramidite; and
B is independently a purine or pyrimidine residue or an analogous residue, and
Q is independently
a phosphodiester analog or
a two to three atom long substitute linkage
wherein at least one of the atoms making up the substitute linkage is selected from nitrogen, oxygen or sulfur, but no two adjacent atoms are oxygen, with the remainder being carbon, and wherein the substitute linkages are independently selected from the group consisting of:
-N(R⁶)-CH₂-
-CH₂-N(R⁶)-
-N(R⁶)-N(R⁶)-
-N(R⁶)-CH₂-CH₂-
-CH₂-N(R⁶)-CH₂-
-CH₂-CH₂-N(R⁶)-
-N(R⁶)-N(R⁶)-CH₂-
-N(R⁶)-CH₂-N-
-CH₂-N(R⁶)-N(R⁶)-
-N=C(NH₂)-N(R⁶)-
-O-CH₂-
-CH₂-O-
-O-CH₂-CH₂-
-CH₂-O-CH₂-
-CH₂-CH₂-O-
-O-CH₂-O-
-S-CH₂-O-
-O-CH₂-S-
-S-CH₂-S-
-S-CH₂-S(O)(O)-
-S-CH₂-
-CH₂-S-
-S(O)-CH₂-
-S(O)(O)-CH₂-
-CH₂-S(O)-
-CH₂-S(O)(O)-
-S-CH₂-CH₂-
-S(O)-CH₂-CH₂-
-S(O)(O)-CH₂-CH₂-
-CH₂-CH₂-S(O)(O)-
-CH₂-S(O)(O)-CH₂-
-CH₂-S-CH₂-
-CH₂-CH₂-S-
-S(O)-CH₂-S-
-S(O)(O)-CH₂-S(O)(O)-
-N(R⁶)-C(O)-S-
-N(R⁶)-C(S)-S-
-N(R⁶)-C(S)-N(R⁶)-
-N(R⁶)-C(O)-N(R⁶)-
-S-C(O)-N(R⁶)-
-S-C(S)-N(R⁶)-
-N(R⁶)-O-
-O-N(R⁶)-
-N(R⁶)-O-CH₂-
-N(R⁶)-CH₂-O-
-O-C(O)-N(R⁶)-
-O-C(S)-N(R⁶)-
-N(R⁶)-C(O)-O-
-N(R⁶)-C(S)-O-
-CH₂-N(R⁶)-O-
-O-N(R⁶)-CH₂-
-O-CH₂-N(R⁶)-
-CH₂-O-N(R⁶)-
-N(R⁶)-S(O)-
-N(R⁶)-S(O)(O)-
-S(O)-N(R⁶)-
-S(O)(O)-N(R⁶)-
-N(R⁶)-S(O)-CH₂-
-N(R⁶)-S(O)(O)-CH₂-
-N(R⁶)-CH₂-S-
-N(R⁶)-CH₂-S(O)-
-N(R⁶)-CH₂-S(O)(O)-
-N(R⁶)-S(O)(O)-
-S(O)(O)-N(R⁶)-
-S-N(R⁶)-CH₂-
-S(O)-N(R⁶)-CH₂-
-S(O)(O)-N(R⁶)-CH₂-
-CH₂-N(R⁶)-S-
-CH₂-N(R⁶)-S(O)-
-CH₂-N(R⁶)-S(O)(O)-
-S-CH₂-N(R⁶)-
-S(O)-CH₂-N(R⁶)-
-S(O)(O)-CH₂-N(R⁶)-
-CH₂-S-N(R⁶)-
-CH₂-S(O)-N(R⁶)-
-CH₂-S(O)(O)-N(R⁶)-
-N(R⁶)-S(O)-N(R⁶)-
-N(R⁶)-S(O)(O)-O-
-O-S(O)(O)-N(R⁶)-
-O-S(O)(O)-O-
-N(R⁶)-S(O)(O)-N(R⁶)-
-CH₂-S(O)(O)-O-
-O-CH₂-S(O)(O)-
-S(O)-CH₂-O-
-O-CH₂-S(O)-
-O-C(R⁸)₂-O-
-S-C(R⁸)₂-O-
-O-C(R⁸)₂-S-
-O-CH₂-CH=CH- (cis and trans isomers)
-S-CH₂-CH=CH- (cis and trans isomers)
-O-CH₂-C≡C-
and
-S-CH₂-C≡C-.
wherein R⁶ is H, lower alkyl, OMe, OH, heteroalkyl, or aryl; and
wherein R⁸ is CH₂F, or when both R⁸ are taken together with the atom to which they are attached, form a 4-membered or 6-membered ring where (R⁸)₂ is
-CH₂-X¹-CH₂-,
-(CH₂)₂-X¹-(CH₂)₂-;
wherein X¹ is selected from the group consisting of NH, NMe, NEt, NPr, S, SO, SO₂, O, CF₂ and CHF;
n is 1-100, and
subject to the proviso that at least one Q is not a phosphodiester analog.

9. The oligomer of claim 8 where at least one substitute linkage is -S-CH₂-CH₂-, -CH₂CH₂-S-, -CH₂-S, or -S-CH₂-.

10. The oligomer of claim 8 where each substitute linkage is selected from the group consisting of -S-CH₂-O-, -O-CH₂-O- and -O-CH₂-S-.

11. The oligomer of claim 8 where each substitute linkage is -O-CH₂-CH=CH- (trans isomer) or -S-CH₂-CH=CH- (trans isomer).

12. The oligomer of claim 8 wherein the derivative comprises a conjugate with a drug, an intercalator or a label.

13. The oligomer of claim 1 having a covalent link between the 5' terminal nucleomonomer and the 3' terminal nucleomonomer forming a circular oligomer.

14. The oligomer of claim 1 which is a dimer, trimer or tetramer.

15. An ex vivo method of increasing or decreasing the expression of at least one selected protein in a cell wherein the protein is encoded by DNA sequences and the protein is translated from RNA sequences, comprising the steps of:
introducing an oligomer of claim 1 into the cell; and
permitting the oligomer to form a triplex with the DNA or RNA or a duplex with the DNA or RNA whereby expression of the protein is increased or decreased.

16. An ex vivo method of introducing an oligomer of claim 1 into cells, comprising:
mixing the oligomer with a permeation enhancing agent to form a complex; and contacting the complex with the cells.

17. A pharmaceutical composition comprising an oligomer as claimed in Claim 1 and a pharmaceutically acceptable carrier.

18. A pharmaceutical composition comprising an oligomer as claimed in Claim 1 and a permeating enhancing agent.

19. An oligomer as claimed in Claim 1, for use in the preparation of a medicament for oligomer based therapy.

20. An oligomer, as claimed in Claim 1, for use in the preparation of a medicament for the treatment of a disease associated with gene expression.

## Patentansprüche

1. Oligomer, aufweisend mindestens eine Austauschverknüpfung zwischen der 2'-und 5'-Stellung benachbarter Nucleomonomere, wobei die Austauschverknüpfung zwei bis vier Atome aufweist, worin mindestens eines der Atome, die die Austauschverknüpfung aufbauen, ausgewählt wird aus Stickstoff, Sauerstoff oder Schwefel und die übrigen Kohlenstoff sind unter der Voraussetzung, daß keine benachbarten zwei oder drei Atome Sauerstoff sind und auch keine benachbarten drei Atome alle Sauerstoff, Stickstoff oder Schwefel sind.

2. Oligomer nach Anspruch 1, bei welchem die Austauschverknüpfung ein Sauerstoffatom enthält, das direkt in der 2'-Stellung des benachbarten Nucleomonomers gebunden ist.

3. Oligomer nach Anspruch 1, bei welchem die Austauschverknüpfung unabhängig ausgewählt wird aus der Gruppe, bestehend aus:
-N(R⁶)-CH₂-
-CH₂-N(R⁶)-
-N(R⁶)-N(R⁶)-
-N(R⁶)-CH₂-CH₂-
-CH₂-N(R⁶)-CH₂-
-CH₂-CH₂-N(R⁶)-
-N(R⁶)-N(R⁶)-CH₂-
-N(R⁶)-CH₂-N-
-CH₂-N(R⁶)-N(R⁶)-
-N=C(NH₂)-N(R⁶)-
-O-CH₂-
-CH₂-O-
-O-CH₂-CH₂-
-CH₂-O-CH₂-
-CH₂-CH₂-O-
-O-CH₂-O-
-S-CH₂-O-
-O-CH₂-S-
-S-CH₂-S-
-S-CH₂-S(O)(O)-
-S-CH₂-
-CH₂-S-
-S(O)-CH₂-
-S(O)(O)-CH₂-
-CH₂-S(O)-
-CH₂-S(O)(O)-
-S-CH₂-CH₂-
-S(O)-CH₂-CH₂-
-S(O)(O)-CH₂-CH₂-
-CH₂-CH₂-S(O)(O)-
-CH₂-S(O)(O)-CH₂-
-CH₂-S-CH₂-
-CH₂-CH₂-S-
-S(O)-CH₂-S-
-S(O)(O)-CH₂-S(O)(O)-
-N(R⁶)-C(O)-S-
-N(R⁶)-C(S)-S-
-N(R⁶)-C(S)-N(R⁶)-
-N(R⁶)-C(O)-N(R⁶)-
-S-C(O)-N(R⁶)-
-S-C(S)-N(R⁶)-
-N(R⁶)-O-
-O-N(R⁶)-
-N(R⁶)-O-CH₂-
-N(R⁶)-CH₂-O-
-O-C(O)-N(R⁶)-
-O-C(S)-N(R⁶)-
-N(R⁶)-C(O)-O-
-N(R⁶)-C(S)-O-
-CH₂-N(R⁶)-O-
-O-N(R⁶)-CH₂-
-O-CH₂-N(R⁶)-
-CH₂-O-N(R⁶)-
-N(R⁶)-S(O)-
-N(R⁶)-S(O)(O)-
-S(O)-N(R⁶)-
-S(O)(O)-N(R⁶)-
-N(R⁶)-S(O)-CH₂-
-N(R⁶)-S(O)(O)-CH₂-
-N(R⁶)-CH₂-S-
-N(R⁶)-CH₂-S(O)-
-N(R⁶)-CH₂-S(O)(O)-
-N(R⁶)-S(O)(O)-
-S(O)(O)-N(R⁶)-
-S-N(R⁶)-CH₂-
-S(O)-N(R⁶)-CH₂-
-S(O)(O)-N(R⁶)-CH₂-
-CH₂-N(R⁶)-S-
-CH₂-N(R⁶)-S(O)-
-CH₂-N(R⁶)-S(O)(O)-
-S-CH₂-N(R⁶)-
-S(O)-CH₂-N(R⁶)-
-S(O)(O)-CH₂-N(R⁶)-
-CH₂-S-N(R⁶)-
-CH₂-S(O)-N(R⁶)-
-CH₂-S(O)(O)-N(R⁶)-
-N(R⁶)-S(O)-N(R⁶)-
-N(R⁶)-S(O)(O)-O-
-O-S(O)(O)-N(R⁶)-
-O-S(O)(O)-O-
-N(R⁶)-S(O)(O)-N(R⁶)-
-CH₂-S(O)(O)-O-
-O-CH₂-S(O)(O)-
-S(O)-CH₂-O-
-O-CH₂-S(O)-
-O-C(R⁸)₂-O-
-S-C(R⁸)₂-O-
-O-C(R⁸)₂-S-
-O-CH₂-CH=CH- (Cis- und Transisomer)
-S-CH₂-CH=CH- (Cis- und Transisomer)
-O-CH₂-C≡C-,
und
-S-CH₂-C≡C-
worin sind:
R⁶ Wasserstoff, niederes Alkyl , (1-6 C), einschließend Methyl, Ethyl, Propyl, Isopropyl und Butyl, OMe, OH, Heteroalkyl (1-6 C, 1-2 Halogen-, S-, N- oder O-Heteroatome) oder Aryl (6-7 C); und
R⁸ CH₂F oder, wenn beide R⁸ gemeinsam mit dem Atom, an dem sie gebunden sind, einen 4-gliedrigen oder 6-gliedrigen Ring bilden, dann ist (R⁸)₂
-CH₂-X¹-CH₂-,
oder
-(CH₂)₂-X¹-(CH₂)₂-;
worin X¹ ausgewählt wird aus der Gruppe, bestehend aus NH, NMe, NEt, NPr, S, SO, SO₂, O, CF₂ und CHF.

4. Oligomer nach Anspruch 3, bei welchem die Austauschverknüpfung unabhängig ausgewählt wird aus der Gruppe, bestehend aus:
-S-CH₂-CH₂-
-S-CH₂-
-O-C(S)-NR⁶-
-O-CH₂-S-
-O-CH₂-O-
-O-C(O)-NR⁶-
-CH₂-CH₂-S-
-CH₂-S-
-NR⁶-C(S)-O-
-S-CH₂-O-,
und
-NR⁶-C(O)-O-

5. Oligomer nach Anspruch 3, bei welchem mindestens eine Austauschverknüpfung -S-CH₂-CH₂-, -CH₂-CH₂-S-, -CH₂-S- oder -S-CH₂- ist.

6. Oligomer nach Anspruch 3, bei welchem mindestens eine Austauschverknüpfung
2' -NH-C(O)-O- 5'
2' -O-C(O)-NH- 5'
2' -O-C(O)-NCH₃- 5'
2' -O-CH₂-S- 5'
ist.

7. Oligomer nach Anspruch 1 mit der Formel (W,Y)-Q-(Z-Q)ₙ-(W,Y), worin sind, jedes: 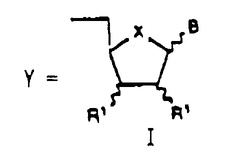 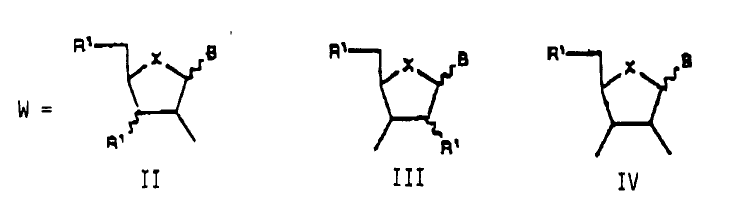 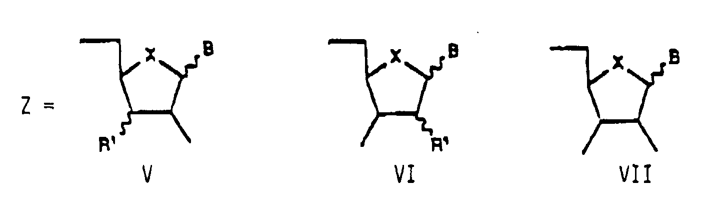
x ist S, O, CH₂, CHF oder CF₂;
R¹ unabhangig -O-Alkyl (C₁-C₁₂), -S-Alkyl (C₁-C₁₂), H, OH, OCH₃, SCH₃, OC₃H₅ (O-Allyl), -O-C₃H₇ (O-Propyl), SC₃H₅ oder F; und
worin sich R¹ an einer terminalen Gruppe des Oligomers befindet und R¹ zusätzlich PO₃⁻² sein kann oder eine blockierende Gruppe sein kann, ausgewählt aus einem Dimethoxytrityl (DMT)-Teil, einem Monomethoxytrityl (MMT)-Teil, H-Phosphonat (OPO₂H), Methylphosphonat (OPO₂CH₃), Methylphosphonamidit oder Phosphoramidit;
B ist eine Base, und
Q ist unabhängig
ein Phosphodiester-Analog oder
eine zwei bis vier Atome lange Austauschverknüpfung, worin mindestens eines der Atome, die die Austauschverknüpfung aufbauen, ausgewählt wird aus Stickstoff, Sauerstoff oder Schwefel und die Übrigen Kohlenstoff sind, jedoch nicht zwei benachbarte Atome Sauerstoff sind und auch keine benachbarten drei Atome alle Sauerstoff, Stickstoff oder Schwefel sind; und
n ist 1 bis 100, und
unter der Voraussetzung, daß mindestens ein Q kein Phosphodiester-Analog ist.

8. Oligomer nach Anspruch 1 mit der Formel (W,Y)-Q-(Z-Q)ₙ-(W,Y) worin sind, jedes: 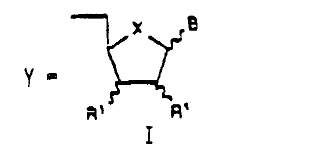 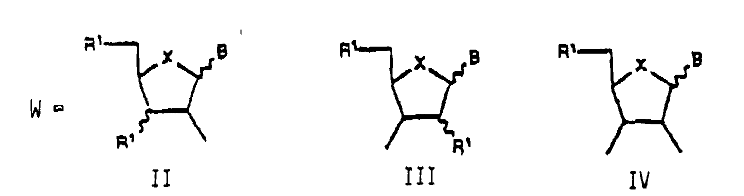 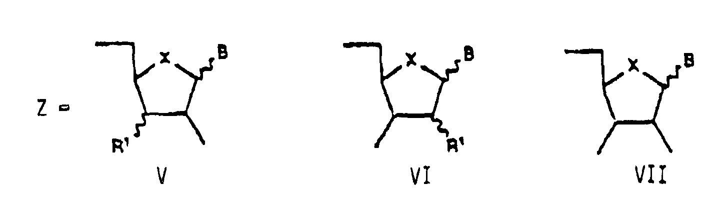
worin X ist S, O, CH₂, CHF oder CF₂;
R¹ unabhängig -O-Alkyl (C₁-C₁₂), -S-Alkyl (C₁-C₁₂), H, OH, OCH₃, SCH₃, OC₃H₅ (O-Allyl), -O-C₃H₇ (O-Propyl), SC₃H₅ oder F; und
worin sich R¹ an einer terminalen Gruppe des Oligomers befindet und R¹ zusätzlich PO₃⁻² sein kann oder eine blockierende Gruppe sein kann, ausgewählt aus einem Dimethoxytrityl (DMT)-Teil, einem Monomethoxytrityl (MMT)-Teil, H-Phosphonat (OPO₂H) , Methylphosphonat (OPO₂CH₃), Methylphosphonamidit oder Phosphoramidit; und
B ist unabhängig ein Purin-Rest oder ein Pyrimidin-Rest oder ein analoger Rest, und
Q ist unabhängig
ein Phosphodiester-Analog oder
eine zwei bis vier Atome lange Austauschverknüpfung, worin mindestens eines der Atome, die die Austauschverknüpfung aufbauen, ausgewählt wird aus Stickstoff, Sauerstoff oder Schwefel, jedoch nicht zwei benachbarte Atome Sauerstoff sind, und die Übrigen Kohlenstoff sind, und worin die Austauschverknüpfungen unabhängig ausgewählt werden aus der Gruppe, bestehend aus:
-N(R⁶)-CH₂-
-CH₂-N(R⁶)-
-N(R⁶)-N(R⁶)-
-N(R⁶)-CH₂-CH₂-
-CH₂-N(R⁶)-CH₂-
-CH₂-CH₂-N(R⁶)-
-N(R⁶)-N(R⁶)-CH₂-
-N(R⁶)-CH₂-N-
-CH₂-N(R⁶)-N(R⁶)-
-N=C(NH₂)-N(R⁶)-
-O-CH₂-
-CH₂-O-
-O-CH₂-CH₂-
-CH₂-O-CH₂-
-CH₂-CH₂-O-
-O-CH₂-O-
-S-CH₂-O-
-O-CH₂-S-
-S-CH₂-S-
-S-CH₂-S(O)(O)-
-S-CH₂-
-CH₂-S-
-S(O)-CH₂-
-S(O)(O)-CH₂-
-CH₂-S(O)-
-CH₂-S(O)(O)-
-S-CH₂-CH₂-
-S(O)-CH₂-CH₂-
-S(O)(O)-CH₂-CH₂-
-CH₂-CH₂-S(O)(O)-
-CH₂-S(O)(O)-CH₂-
-CH₂-S-CH₂-
-CH₂-CH₂-S-
-S(O)-CH₂-S-
-S(O)(O)-CH₂-S(O)(O)-
-N(R⁶)-C(O)-S-
-N(R⁶)-C(S)-S-
-N(R⁶)-C(S)-N(R⁶)-
-N(R⁶)-C(O)-N(R⁶)-
-S-C(O)-N(R⁶)-
-S-C(S)-N(R⁶)-
-N(R⁶)-O-
-O-N(R⁶)-
-N(R⁶)-O-CH₂-
-N(R⁶)-CH₂-O-
-O-C(O)-N(R⁶)-
-O-C(S)-N(R⁶)-
-N(R⁶)-C(O)-O-
-N(R⁶)-C(S)-O-
-CH₂-N(R⁶)-O-
-O-N(R⁶)-CH₂-
-O-CH₂-N(R⁶)-
-CH₂-O-N(R⁶)-
-N(R⁶)-S(O)-
-N(R⁶)-S(O)(O)-
-S(O)-N(R⁶)-
-S(O)(O)-N(R⁶)-
-N(R⁶)-S(O)-CH₂-
-N(R⁶)-S(O)(O)-CH₂-
-N(R⁶)-CH₂-S-
-N(R⁶)-CH₂-S(O)-
-N(R⁶)-CH₂-S(O)(O)-
-N(R⁶)-S(O)(O)-
-S(O)(O)-N(R⁶)-
-S-N(R⁶)-CH₂-
-S(O)-N(R⁶)-CH₂-
-S(O)(O)-N(R⁶)-CH₂-
-CH₂-N(R⁶)-S-
-CH₂-N(R⁶)-S(O)-
-CH₂-N(R⁶)-S(O)(O)-
-S-CH₂-N(R⁶)-
-S(O)-CH₂-N(R⁶)-
-S(O)(O)-CH₂-N(R⁶)-
-CH₂-S-N(R⁶)-
-CH₂-S(O)-N(R⁶)-
-CH₂-S(O)(O)-N(R⁶)-
-N(R⁶)-S(O)-N(R⁶)-
-N(R⁶)-S(O)(O)-O-
-O-S(O)(O)-N(R⁶)-
-O-S(O)(O)-O-
-N(R⁶)-S(O)(O)-N(R⁶)-
-CH₂-S(O)(O)-O-
-O-CH₂-S(O)(O)-
-S(O)-CH₂-O-
-O-CH₂-S(O)-
-O-C(R⁸)₂-O-
-S-C(R⁸)₂-O-
-O-C(R⁸)₂-S-
-O-CH₂-CH=CH- (Cis- und Transisomer)
-S-CH₂-CH=CH- (Cis- und Transisomer)
-O-CH₂-C≡C-,
und
-S-CH₂-C≡C-
worin R⁶ Wasserstoff, niederes Alkyl, OMe, OH, Heteroalkyl oder Aryl ist; und
worin R⁸ CH₂F ist, oder, wenn beide R⁸ gemeinsam mit dem Atom, an dem sie gebunden sind, einen 4-gliedrigen oder 6-gliedrigen Ring bilden, wobei (R⁸)₂ ist:
-CH₂-X¹-CH₂-,
-(CH₂)₂-X¹-(CH₂)₂-;
worin X¹ ausgewählt wird aus der Gruppe, bestehend aus NH, NMe, NEt, NPr, S, SO, SO₂, O, CF₂ und CHF;
n ist 1 bis 100, und
unter der Voraussetzung, daß mindestens ein Q kein Phosphodiester-Analog ist.

9. Oligomer nach Anspruch 8, bei welchem mindestens eine Austauschverknüpfung -S-CH₂-CH₂-, -CH₂-CH₂-S-, -CH₂-S- oder -S-CH₂- ist.

10. Oligomer nach Anspruch 8, bei welchem mindestens eine Austauschverknüpfung ausgewählt wird aus der Gruppe, bestehend aus -S-CH₂-O-, -O-CH₂-O- und -O-CH₂-S-.

11. Oligomer nach Anspruch 8, bei welchem jede Austauschverknüpfung -O-CH₂-CH=CH- (Transisomer) oder -S-CH₂-CH=CH- (Transisomer) ist.

12. Oligomer nach Anspruch 8, bei welchem das Derivat ein Konjugat mit einem Arzneimittel, einem Interkalator oder einem Label aufweist.

13. Oligomer nach Anspruch 1 mit einer kovalenten Verknüpfung zwischen dem 5'-terminalen Nucleomonomer und dem 3'-terminalen Nucleomonomer, die ein ringförmiges Oligomer erzeugt.

14. Oligomer nach Anspruch 1, das ein Dimer, Trimer oder Tetramer ist.

15. Verfahren, ex vivo, zur Verstärkung oder Verringerung der Expression von mindestens einem ausgewählten Protein in einer Zelle, in welchem Verfahren das Protein durch DNA-Sequenzen kodiert wird und das Protein von RNA-Sequenzen translatiert wird, aufweisend die Schritte:
Einführen eines Oligomers nach Anspruch 1 in die Zelle; und
das Oligomer einen Triplex mit der DNA oder RNA oder einen Duplex mit der DNA oder RNA bilden lassen, wodurch die Expression des Proteins verstärkt oder verringert wird.

16. Verfahren, ex vivo, zum Einführen in Zellen eines Oligomers nach Anspruch 1, aufweisend:
Mischen des Oligomers mit einem die Permeation verstärkendes Mittel, um einen Komplex zu bilden; sowie Kontaktieren des Komplexes mit den Zellen.

17. Pharmazeutische Zusammensetzung, aufweisend ein Oligomer nach Anspruch 1 und einen pharmazeutisch verträglichen Träger.

18. Pharmazeutische Zusammensetzung, aufweisend ein Oligomer nach Anspruch 1 und ein die Permeation verstärkendes Mittel.

19. Oligomer nach Anspruch 1 zur Verwendung in der Herstellung eines Arzneimittels für eine Oligomer-basierte Therapie.

20. Oligomer nach Anspruch 1 zur Verwendung in der Herstellung eines Arzneimittels für die Behandlung einer mit Genexpression assoziierten Erkrankung.

## Revendications

1. Oligomère comprenant au moins une liaison de substitution entre la position 2' et 5' de nucléomonomères adjacents, ladite liaison de substitution possédant deux à quatre atomes dans laquelle au moins un des atomes constituant la liaison de substitution est choisi parmi l'azote, l'oxygène ou le soufre, le reste des atomes étant du carbone à condition que deux ou trois atomes adjacents ne soient pas de l'oxygène ou que trois atomes adjacents ne soient pas tous de l'oxygène, de l'azote ou du soufre.

2. Oligomère de la revendication 1, dans lequel la liaison de substitution contient un atome d'oxygène directement fixé à la position 2' du nucléomonomère adjacent.

3. Oligomère comme revendiqué dans la revendication 1, dans lequel les liaisons de substitution sont indépendamment choisies parmi le groupe composé de:
-N(R⁶)-CH₂-
-CH₂-N(R⁶)-
-N(R⁶)-N(R⁶)-
-N(R⁶)-CH₂-CH₂-
-CH₂-N(R⁶)-CH₂-
-CH₂-CH₂-N(R⁶)-
-N(R⁶)-N(R⁶)-CH₂-
-N(R⁶)-CH₂-N-
-CH₂-N(R⁶)-N(R⁶)-
-N=C(NH₂)-N(R⁶)-
-O-CH₂-
-CH₂-O-
-O-CH₂-CH₂-
-CH₂-O-CH₂-
-CH₂-CH₂-O-
-O-CH₂-O-
-S-CH₂-O-
-O-CH₂-S-
-S-CH₂-S-
-S-CH₂-S(O)(O)-
-S-CH₂-
-CH₂-S-
-S(O)-CH₂-
-S(O)(O)-CH₂-
-CH₂-S(O)-
-CH₂-S(O)(O)-
-S-CH₂-CH₂-
-S(O)-CH₂-CH₂-
-S(O)(O)-CH₂-CH₂-
-CH₂-CH₂-S(O)(O)-
-CH₂-S(O)(O)-CH₂-
-CH₂-S-CH₂-
-CH₂-CH₂-S-
-S(O)-CH₂-S-
-S(O)(O)-CH₂-S(O)(O)-
-N(R⁶)-C(O)-S-
-N(R⁶)-C(S)-S-
-N(R⁶)-C(S)-N(R⁶)-
-N(R⁶)-C(O)-N(R⁶)-
-S-C(O)-N(R⁶)-
-S-C(S)-N(R⁶)-
-N(R⁶)-O-
-O-N(R⁶)-
-N(R⁶)-O-CH₂-
-N(R⁶)-CH₂-O-
-O-C(O)-N(R⁶)-
-O-C(S)-N(R⁶)-
-N(R⁶)-C(O)-O-
-N(R⁶)-C(S)-O-
-CH₂-N(R⁶)-O-
-O-N(R⁶)-CH₂-
-O-CH₂-N(R⁶)-
-CH₂-O-N(R⁶)-
-N(R⁶)-S(O)-
-N(R⁶)-S(O)(O)-
-S(O)-N(R⁶)-
-S(O)(O)-N(R⁶)-
-N(R⁶)-S(O)-CH₂-
-N(R⁶)-S(O)(O)-CH₂-
-N(R⁶)-CH₂-S-
-N(R⁶)-CH₂-S(O)-
-N(R⁶)-CH₂-S(O)(O)-
-N(R⁶)-S(O)(O)-
-S(O)(O)-N(R⁶)-
-S-N(R⁶)-CH₂-
-S(O)-N(R⁶)-CH₂-
-S(O)(O)-N(R⁶)-CH₂-
-CH₂-N(R⁶)-S-
-CH₂-N(R⁶)-S(O)-
-CH₂-N(R⁶)-S(O)(O)-
-S-CH₂-N(R⁶)-
-S(O)-CH₂-N(R⁶)-
-S(O)(O)-CH₂-N(R⁶)-
-CH₂-S-N(R⁶)-
-CH₂-S(O)-N(R⁶)-
-CH₂-S(O)(O)-N(R⁶)-
-N(R⁶)-S(O)-N(R⁶)-
-N(R⁶)-S(O)(O)-O-
-O-S(O)(O)-N(R⁶)-
-O-S(O)(O)-O-
-N(R⁶)-S(O)(O)-N(R⁶)-
-CH₂-S(O)(O)-O-
-O-CH₂-S(O)(O)-
-S(O)-CH₂-O-
-O-CH₂-S(O)-
-O-C(R⁸)₂-O-
-S-C(R⁸)₂-O-
-O-C(R⁸)₂-S-
-O-CH₂-CH=CH- (isomères cis et trans)
-S-CH₂-CH=CH- (isomères cis et trans)
-O-CH₂-C≡C-,
et
-S-CH₂-C≡C-
dans lesquels R⁶ est H, un groupe alkyle inférieur (1-6C), y compris un groupe méthyle, éthyle, propyle, isopropyle et butyle, OMe, OH, hétéroalkyle (1-6C, 1-2 hétéroatomes d'halogène, de S, de N ou de O) ou un groupe aryle (6-7C); et
dans lesquels R⁸ est CH₂F, ou lorsque les deux radicaux R⁸ sont considérés ensemble avec l'atome auquel ils sont fixés alors que R⁸ forme un cycle à 4 membres ou à 6 membres où (R⁸)₂ est
-CH₂-X¹-CH₂-,
ou
-(CH₂)₂-X¹-(CH₂)₂-;
dans lesquels X¹ est choisi parmi le groupe composé de NH, NMe, NEt, NPr, S, SO, SO₂, O, CF₂ et CHF.

4. Oligomère de la revendication 3, dans lequel les liaisons de substitution sont indépendamment choisies parmi le groupe composé de:
-S-CH₂-CH₂-
-S-CH₂-
-O-C(S)-NR⁶-
-O-CH₂-S-
-O-CH₂-O-
-O-C(O)-NR⁶-
-CH₂-CH₂-S-
-CH₂-S-
-NR⁶-C(S)-O-
-S-CH₂-O-,
et
-NR⁶-C(O)-O-

5. Oligomère de la revendication 3, où au moins une liaison de substitution est -S-CH₂-CH₂-, -CH₂-CH₂-S-, -CH₂-S- ou -S-CH₂-.

6. Oligomère de la revendication 3, dans lequel au moins une liaison de substitution est
2' -NH-C(O)-O- 5'
2' -O-C(O)-NH- 5'
2' -O-C(O)-NCH₃- 5'
2' -O-CH₂-S- 5'

7. Oligomère comme revendiqué dans la revendication 1, possédant la formule (W,Y)-Q-(Z-Q)ₙ-(W,Y), où chaque 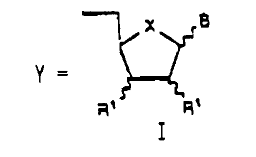 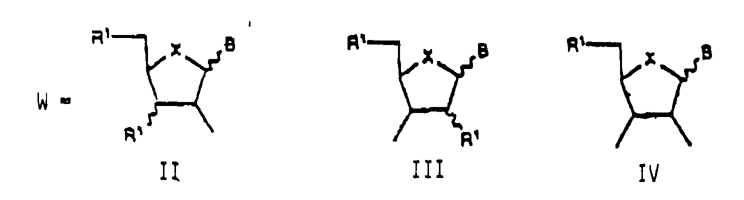 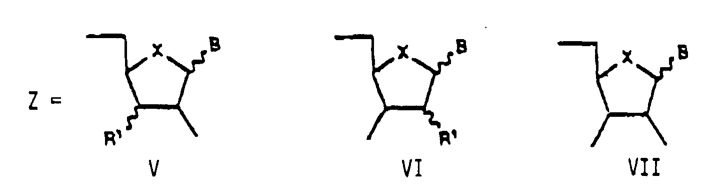
où X est S, O, CH₂, CHF ou CF₂,
R¹ est indépendamment un groupe -O-alkyle (C₁-C₁₂), -S-alkyle (C₁-C₁₂), H, OH, OCH₃, SCH₃, OC₃H₅ (O-allyle), OC₃H₇ (O-propyle), SC₃H₅ ou F, et
où R¹ est situé sur un groupe terminal de l'oligomère, R¹ peut en outre être PO₃⁻² ou un groupe bloquant choisi parmi une fraction de diméthoxytrityle (DMT), une fraction de monométhoxytrityle (MMT), le H-phosphonate (OPO₂H), le méthylphosphonate (OPO₂CH₃), la méthylphosphonamidite ou la phosphoramidite;
B est une base, et
Q est indépendamment
un analogue de phosphodiester ou
une liaison de substitution de deux à quatre atomes de longueur dans laquelle au moins un des atomes constituant la liaison de substitution est choisi parmi l'azote, l'oxygène ou le soufre, le reste des atomes étant du carbone, mais deux atomes adjacents ne peuvent pas être de l'oxygène ou trois atomes adjacents quelconques ne peuvent pas tous être de l'oxygène, de l'azote, ou du soufre; et
n est compris entre 1 et 100, et
soumis à la condition qu'au moins un radical Q n'est pas un analogue de phosphodiester.

8. Oligomère comme revendiqué dans la revendication 1, possédant la formule (W,Y)-Q-(Z-Q)ₙ-(W,Y), où chaque 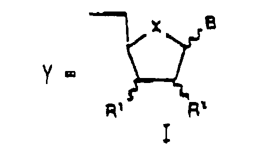 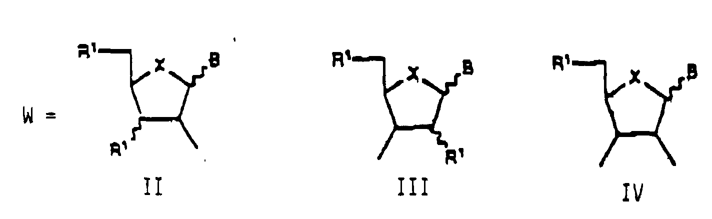 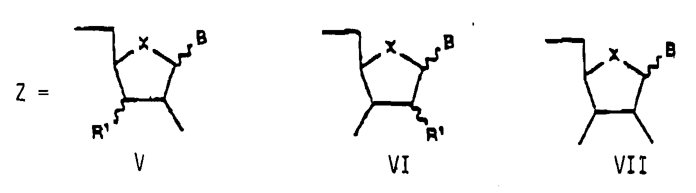
dans lequel X est S, O, CH₂, CHF ou CF₂,
R¹ est indépendamment un groupe -O-alkyle (C₁-C₁₂), -S-alkyle (C₁-C₁₂), H, OH, OCH₃, SCH₃, OC₃H₅ (O-allyle), OC₃H₇ (O-propyle), SC₃H₅ ou F, et
lorsque R¹ se situe sur un groupe terminal de l'oligomère, R¹ peut être en outre PO₃⁻² ou un groupe bloquant choisi parmi une fraction de diméthoxytrityle (DMT), une fraction de monométhoxytrityle (MMT), le H-phosphonate (OPO₂H), le méthylphosphonate (OPO₂CH₃), la méthylphosphonamidite ou la phosphoramidite, et
B est indépendamment un résidu de purine ou de pyrimidine ou un résidu analogue, et
Q est indépendamment
un analogue de phosphodiester ou
une liaison de substitution de deux à trois atomes de longueur dans laquelle au moins un des atomes constituant la liaison de substitution est choisi parmi l'azote, l'oxygène ou le soufre, mais deux atomes adjacents ne peuvent pas être de l'oxygène, le reste des atomes étant du carbone. et dans laquelle les liaisons de substitution sont indépendamment choisies parmi le groupe composé de:
-N(R⁶)-CH₂-
-CH₂-N(R⁶)-
-N(R⁶)-N(R⁶)-
-N(R⁶)-CH₂-CH₂-
-CH₂-N(R⁶)-CH₂-
-CH₂-CH₂-N(R⁶)-
-N(R⁶)-N(R⁶)-CH₂-
-N(R⁶)-CH₂-N-
-CH₂-N(R⁶)-N(R⁶)-
-N=C(NH₂)-N(R⁶)-
-O-CH₂-
-CH₂-O-
-O-CH₂-CH₂-
-CH₂-O-CH₂-
-CH₂-CH₂-O-
-O-CH₂-O-
-S-CH₂-O-
-O-CH₂-S-
-S-CH₂-S-
-S-CH₂-S(O)(O)-
-S-CH₂-
-CH₂-S-
-S(O)-CH₂-
-S(O)(O)-CH₂-
-CH₂-S(O)-
-CH₂-S(O)(O)-
-S-CH₂-CH₂-
-S(O)-CH₂-CH₂-
-S(O)(O)-CH₂-CH₂-
-CH₂-CH₂-S(O)(O)-
-CH₂-S(O)(O)-CH₂-
-CH₂-S-CH₂-
-CH₂-CH₂-S-
-S(O)-CH₂-S-
-S(O)(O)-CH₂-S(O)(O)-
-N(R⁶)-C(O)-S-
-N(R⁶)-C(S)-S-
-N(R⁶)-C(S)-N(R⁶)-
-N(R⁶)-C(O)-N(R⁶)-
-S-C(O)-N(R⁶)-
-S-C(S)-N(R⁶)-
-N(R⁶)-O-
-O-N(R⁶)-
-N(R⁶)-O-CH₂-
-N(R⁶)-CH₂-O-
-O-C(O)-N(R⁶)-
-O-C(S)-N(R⁶)-
-N(R⁶)-C(O)-O-
-N(R⁶)-C(S)-O-
-CH₂-N(R⁶)-O-
-O-N(R⁶)-CH₂-
-O-CH₂-N(R⁶)-
-CH₂-O-N(R⁶)-
-N(R⁶)-S(O)-
-N(R⁶)-S(O)(O)-
-S(O)-N(R⁶)-
-S(O)(O)-N(R⁶)-
-N(R⁶)-S(O)-CH₂-
-N(R⁶)-S(O)(O)-CH₂-
-N(R⁶)-CH₂-S-
-N(R⁶)-CH₂-S(O)-
-N(R⁶)-CH₂-S(O)(O)-
-N(R⁶)-S(O)(O)-
-S(O)(O)-N(R⁶)-
-S-N(R⁶)-CH₂-
-S(O)-N(R⁶)-CH₂-
-S(O)(O)-N(R⁶)-CH₂-
-CH₂-N(R⁶)-S-
-CH₂-N(R⁶)-S(O)-
-CH₂-N(R⁶)-S(O)(O)-
-S-CH₂-N(R⁶)-
-S(O)-CH₂-N(R⁶)-
-S(O)(O)-CH₂-N(R⁶)-
-CH₂-S-N(R⁶)-
-CH₂-S(O)-N(R⁶)-
-CH₂-S(O)(O)-N(R⁶)-
-N(R⁶)-S(O)-N(R⁶)-
-N(R⁶)-S(O)(O)-O-
-O-S(O)(O)-N(R⁶)-
-O-S(O)(O)-O-
-N(R⁶)-S(O)(O)-N(R⁶)-
-CH₂-S(O)(O)-O-
-O-CH₂-S(O)(O)-
-S(O)-CH₂-O-
-O-CH₂-S(O)-
-O-C(R⁸)₂-O-
-S-C(R⁸)₂-O-
-O-C(R⁸)₂-S-
-O-CH₂-CH=CH- (isomères cis et trans)
-S-CH₂-CH=CH- (isomères cis et trans)
-O-CH₂-C≡C-,
et
-S-CH₂-C≡C-
dans lesquels R⁶ est H, un groupe alkyle inférieur, OMe, OH, hétéroalkyle ou aryle; et
dans lesquels R⁸ est CH₂F, ou lorsque les deux radicaux R⁸ sont considérés ensemble avec l'atome auquel ils sont fixés, R⁸ forme un cycle à 4 membres ou à 6 membres où (R⁸)₂ est
-CH₂-X¹-CH₂-,
-(CH₂)₂-X¹-(CH₂)₂-;
dans lesquels X¹ est choisi parmi le groupe composé de NH, NMe, NEt, NPr, S, SO, SO₂, O, CF₂ et CHF;
n est compris entre 1 et 100, et
soumis a la condition qu'au moins un radical Q n'est pas un analogue de phosphodiester.

9. Oligomère de la revendication 8, où au moins une liaison de substitution est -S-CH₂-CH₂-, -CH₂-CH₂-S-, -CH₂-S- ou -S-CH₂-.

10. Oligomère de la revendication 8, où chaque liaison de substitution est choisie parmi le groupe composé de -S-CH₂-O-, -O-CH₂-O- et -O-CH₂-S-.

11. Oligomère de la revendication 8, où chaque liaison de substitution est -O-CH₂-CH=CH- (isomère trans) ou -S-CH₂-CH=CH- (isomère trans).

12. Oligomère de la revendication 8, dans lequel le dérivé comprend un conjugué avec un médicament, un intercalaire ou un marquage.

13. Oligomère de la revendication 1, possédant une liaison covalente entre le nucléomonomère terminal 5' et le nucléomonomère terminal 3' formant un oligomère circulaire.

14. Oligomère de la revendication 1, qui consiste en un dimère, un trimère ou un tétramère.

15. Procédé ex vivo d'accroissement ou de réduction de l'expression d'au moins une protéine choisie dans une cellule dans laquelle la protéine est codée par des séquences d'ADN et la protéine est transcodée à partir des séquences d'ARN, comprenant les étapes consistant:
à introduire un oligomère de la revendication 1 dans la cellule; et
à permettre à l'oligomère de former un triplex avec l'ADN ou l'ARN ou un duplex avec l'ADN ou l'ARN moyen par quel l'expression de la protéine est augmentée ou réduite.

16. Procédé ex vivo d'introduction de l'oligomère de la revendication 1 dans des cellules, comprenant:
le mélange de l'oligomère avec un agent activateur de perméation pour former un complexe; et
la mise en contact du complexe avec les cellules,

17. Composition pharmaceutique comprenant un oligomère comme revendiqué dans la revendication 1 et un support pharmaceutiquement acceptable.

18. Composition pharmaceutique comprenant un oligomère comme revendiqué dans la revendication 1 et un agent activateur de perméation.

19. Oligomère comme revendiqué dans la revendication 1, destiné à être utilisé dans la préparation d'un médicament pour une thérapie basée sur un oligomère.

20. Oligomère comme revendiqué dans la revendication 1. destiné à être utilisé dans la préparation d'un médicament pour le traitement d'une maladie associée à l'expression de gènes.
